(19) Europäisches Patentamt
European Patent Office
Office européen des brevets

(11) Publication number: **0 234 708 B1**

# (12) EUROPEAN PATENT SPECIFICATION

(45) Date of publication of patent specification: **27.02.91 Bulletin 91/09**

(51) Int. Cl.$^5$: **C07D 209/86**, C07D 209/88, A61K 31/40

(21) Application number: **87300449.3**

(22) Date of filing: **20.01.87**

## (54) Tetrahydrocarbazole 1-alkanoic acids.

(30) Priority: **23.01.86 US 821726**

(43) Date of publication of application: **02.09.87 Bulletin 87/36**

(45) Publication of the grant of the patent: **27.02.91 Bulletin 91/09**

(84) Designated Contracting States: **AT BE CH DE ES FR GB GR IT LI LU NL SE**

(56) References cited:
**EP-A- 0 200 391**
**FR-A- 2 103 403**
**GB-A- 1 511 169**
**US-A- 4 057 559**

(73) Proprietor: **MERCK FROSST CANADA INC.
16711 Trans-Canada Highway
Kirkland Quebec (CA)**

(72) Inventor: **Guindon, Yvan
7750 Jean Bourdon
Montreal, QUE H4K 1H3 (CA)**
Inventor: **Yoakim, Christiane
5770 Cote St. Luc Road Apt. 12
Montreal, QUE H3X 2G1 (CA)**
Inventor: **Gillard, John W.
710 Westchester Baie d'Urfe
QUE H9X 2S1 (CA)**

(74) Representative: **Hesketh, Alan, Dr.
European Patent Department Merck & Co., Inc. Terlings Park Eastwick Road
Harlow Essex, CM20 2QR (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 234 708 B1

## Description

### BACKGROUND OF THE INVENTION

This invention relates to prostaglandin antagonists useful in treating a variety of conditions, such as allergic asthma where excessive contractile activity of prostaglandins and prostaglandin biosynthetic intermediates occur.

These compounds antagonize the actions of contractile prostaglandins, such as $PGF_{2\alpha}$, $PGG_2$, $PGH_2$, $PGD_2$ and $TXA_2$. The use of agents which act as prostaglandin antagonists offers new approaches to therapy in a number of disease states. For example, certain prostaglandins, such as $PGF_{2\alpha}$, $PGD_2$, $PGG_2$, and $PGH_2$, are potent bronchospastic agents. Indeed human asthmatics have been shown to be especially sensitive to the bronchial constricting action of $PGF_{2\alpha}$.

The compounds of the present invention are also antithrombotic agents. Thus, they are useful in the treatment and/or prevention of thromboembolic diseases such as arterial thrombosis and those involving platelet deposition, e.g. prothesis.

In addition to the involvement of contractile prostaglandins in asthma, prostaglandins are known to play a role in other allergic conditions, as well as, diarrhea, hypertension, angina, platelet aggregation, cerebral spasm, cerebral ischemia, arrythmia, circulatory shock, sudden death, atherosclerosis, myocardial ischemia, premature labor, spontaneous abortion, dysmenorrhea, glomerular nephritis, and systemic lupus erythematosis. Consequently, the compounds of this invention will alleviate the above mentioned diseases.

In addition to the prostaglandin antagonist actions, the compounds of this invention are inhibitors of the biosynthesis of 5-lipoxygenase metabolites of arachidonic acid, such as 5-HPETE, 5-HETE and the leukotrienes. Leukotrienes $B_4$, $C_4$, $D_4$ and $E_4$ are known to contribute to various disease conditions such as asthma, psoriasis, pain, ulcers and systemic anaphylaxis. Thus inhibition of the synthesis of such compounds will alleviate these and other leukotriene-related disease states.

The compounds of the present invention may be used to treat or prevent mammalian (especially, human) disease states such as erosive gastritis ; erosive esophagitis ; ethanol-induced hemorrhagic erosions ; hepatic ischemia ; noxious agent induced damage or necrosis of hepatic, pancreatic, renal, or myocardial tissue ; liver parenchymal damage caused by hepatotoxic agents such as $CCl_4$ and D-galactosamine ; ischemic renal failure ; disease-induced hepatic damage ; bile salt induced pancreatic or gastric damage ; trauma- or stress-induced cell damage ; and glycerol-induced renal failure.

Certain 9-benzyl-1,2,3,4-tetrahydrocarbazole acetic acids or esters thereof are shown as chemical intermediates in the preparation of carbazoles that are known in the art as antiinflammatory, analagesic and anti-rheumatic agents (see U.S. Patent 3,896,145 and British Patent 1,385,620). Certain 9-benzyl-1,2,3,4-tetrahydrocarbazole carboxylic acids are known in the art as anti-inflammatory, analgesic and anti-rheumatic agents (see U.S. patents 3,868,387 ; 4,009,181 ; 3,905,998 and 3,758,496), and 9-benzyl-carbazole carboxylic acids (U.S. Patents 3,956,295 and 4,057,640) and 9-benzylcarbazole acetic acids and esters thereof (U.S. Patent 3,896,145 and British Patent 1,385,620) are known as anti-inflammatory, analgesic and anti-rheumatic agents. None of these compounds, however, are shown to be prostaglandin, or thromboxane antagonists or inhibitors of leukotrine biosynthesis.

### DESCRIPTION OF THE INVENTION

The present invention relates to novel compounds of Formula I :

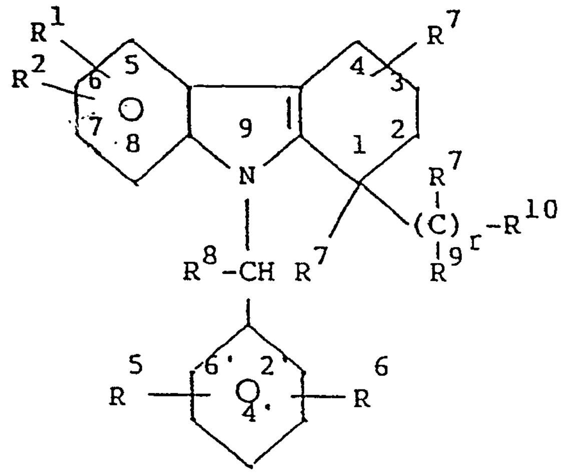

I

wherein :

$R^1$, $R^2$, $R^5$ and $R^6$ are each independently selected from :

(1) hydrogen ;

(2) linear alkyl having 1 to 6 carbon atoms ;

(3) M

wherein M is

a) $OR^{13}$ ;

b) halogen ;

c) $CF_3$ ;

d) $SR^{13}$ ;

e) phenyl ;

f) $COOR^{14}$ ;

g) 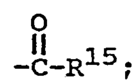 ;

h) tetrazole ;

i) 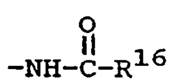 wherein $R^{16}$ is linear $C_1$ to $C_6$ alkyl;

j) $-NR^{14}R^{14}$.

k) $-NHSO_2R^{17}$ wherein $R^{17}$ is linear $C_1$ to $C_6$ alkyl, or $CF_3$ ;

l) $-SOR^{13}$ ;

m) $-CONR^{14}R^{14}$ ;

n) $-SO_2NR^{14}R^{14}$ ;

o) $-SO_2R^{13}$ ;

p) $NO_2$ ;

q) CN ;

r $N_3$ ;

$R^7$ is H or linear alkyl of 1 to 6 carbons ;

$R^8$ is H or linear alkyl of 1 to 6 carbon atoms ;

each $R^9$ is independently H, OH, linear $C_1$ to $C_4$-O-alkyl or linear alkyl of 1 to 4 carbons ;

$R^{10}$ is COOH ; $CH_2OH$ ; CHO ; tetrazole ; $CONHSO_2R^{11}$ ; CN ; $CON(R^9)_2$ ; or $NHSO_2R^{11}$ wherein $R^{11}$

is OH, linear alkyl or linear alkoxy of 1 to 6 carbons, linear perhaloalkyl of 1 to 6 carbons, phenyl or phenyl substituted by linear alkyl or linear alkoxy groups of 1 to 3 carbons, halogen, hydroxy, COOH, CN, formyl or acyl to 1 to 6 carbons ;

r is 1 to 6 ;

each $R^{13}$ independently is H ; or linear $C_1$ to $C_6$ alkyl ;

each $R^{14}$ is independently H, or linear $C_1$ to $C_6$ alkyl ; and,

each $R^{15}$ independently is H, linear $C_1$ to $C_6$ alkyl, or $CF_3$ ; or a pharmaceutically acceptable saltthereof.

As used herein, the terms "each independently" or the equivalents thereof are employed to describe a number of possible position isomers and/or structural variations. For example, as described above, the following unit is attached to position 1 of the tetrahydrocarbazole ring :

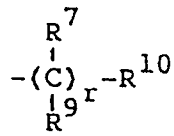

The letter r represents possible alkane chains of from 1 to 6 carbon atoms, each having the $R^7$ and $R^9$ substituent groups. On each carbon atom of the alkane chain, the $R^7$ and/or $R^9$ substituent may be different. The above description therefore contemplates structures such as the following for the segment $-(CR^7R^9)r-$:

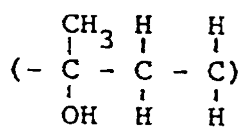 , $(-\ CH_2\ -)$

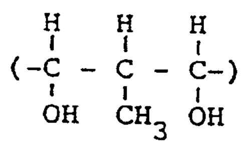 , 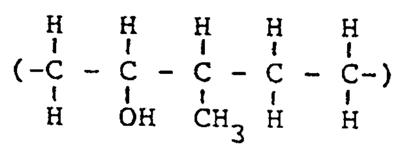 ,

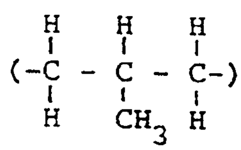 , and the like.

If an $R^9$ is OH and $R^{10}$ is $CO_2H$, such compounds may form a lactone, and such lactones are to be regarded as part of the present invention.

The alkyl groups referred to above are linear,

As used herein, the term ; "lower" as applied to alkyl, acyl, alkoxy and the like, unless stated otherwise refers to groups having 1 to 6 carbon atoms. Halogen or halo means fluoro, chloro, bromo and/or iodo.

Pharmaceutically acceptable salts of the compounds described herein are included within the scope of the present invention. Such salts may be prepared from pharmaceutically acceptable non-toxic bases including inorganic bases and organic bases. Salts derived from inorganic bases include sodium, potassium, lithium, ammonium, calcium, magnesium, ferrous, zinc, copper, manganous, aluminum, ferric, manganic salts and the like. Particularly preferred are the potassium, sodium calcium and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, tri-methylamine, diethanolamine, diethylamine, triethylamine, tripropylamine, ethanolamine, 2-dimethylaminoethanol, 2-diethylamino-ethanol, tomethamine, lysine, arginine, histidine, caffeine, procaine, hydrabamine, choline, imidazole, betaine,

ethylenediamine, glucosamine, methylglucamine, theobromine, purines piperazine, N,N-dibenzylethylenediamine, piperidine, N-ethyl-piperidine, morpholine, N-ethylmorpholine, polyamine resins and the like.

Preferred compounds of the present invention comprise the compounds of formula I wherein :

$R^1$, $R^2$, $R^5$ and $R^6$ are each independently selected from :

(1) hydrogen ;

(2) linear alkyl having 1 to 6 carbon atoms ;

(3) M

wherein M is as defined previously for Formula I ;

$R^{10}$ is COOH ; $CH_2OH$ ; CHO ; tetrazole ; $CONHSO_2R^{11}$ wherein $R^{11}$ is OH, linear alkyl or linear alkoxy of 1 to 6 carbons, linear perhaloalkyl of 1 to 6 carbons, phenyl or phenyl substituted by linear alkyl or linear alkoxy groups of 1 to 3 carbons, halogen, hydroxy, COOH, CN, formyl or acyl to 1 to 6 carbons ; CN ; or $CON(R^9)_2$ ;

r is 1 to 6 ; and the remaining substituents are as defined previously for Formula I.

More preferred compounds of the present invention comprise the compounds of Formula I wherein :

$R^1$, $R^2$, $R^5$ and $R^6$ are each independently selected from :

(1) hydrogen ;

(2) linear alkyl having 1 to 6 carbon atoms ;

(3) M wherein M is as defined initially for Formula I ;

$R^{10}$ is COOH ; $CH_2OH$ ; CHO ; or tetrazole ;

r is 1 or 2 ; and the remaining substituents are as defined initially for Formula I.

Most preferred compounds of the present invention comprise the compounds of Formula I wherein :

$R^1$, $R^2$, $R^5$ and $R^6$ are each independently selected from :

(1) hydrogen ;

(2) linear alkyl having 1 to 6 carbon atoms ;

(3) M wherein M is

a) $OR^{13}$ ;

b) hallogen ;

c) $CF_3$ ;

d) $SR^{13}$ ;

e) $COOR^{14}$ ;

$$\text{f)}\quad -\overset{\overset{\displaystyle O}{\|}}{C}-R^{15};$$

g) tetrazole ;

h) $-SOR^{13}$ ;

i) $-CONR^{14}R^{14}$ ;

j) $-SO_2NR^{14}R^{14}$ ;

k) $-SO_2R^{13}$ ;

l) CN ;

m) $N_3$ ;

each $R^9$ is independently H, or linear alkyl of 1 to 4 carbons ;

$R^{10}$ is COOH ; or tetrazole ;

r is 1 and the remaining substituents are as defined initially for Formula I.

In the above most preferred embodiment, those compounds are particularly preferred wherein at least one of $R^1$ and $R^2$ is not hydrogen ; one of $R^5$ or $R^6$ is not hydrogen ; $R^7$ is hydrogen ; $R^9$ is hydrogen ; and the remaining substituents are as defined in the most preferred embodiment.

A further embodiment of the present invention are the following novel compounds (Table 1) Among the resolved isomers in Table 1, the (–) isomers, compounds 3,37,39 and 41, are preferred.

Table 1

Novel Tetrahydrocarbazole

Alkanoic Acids

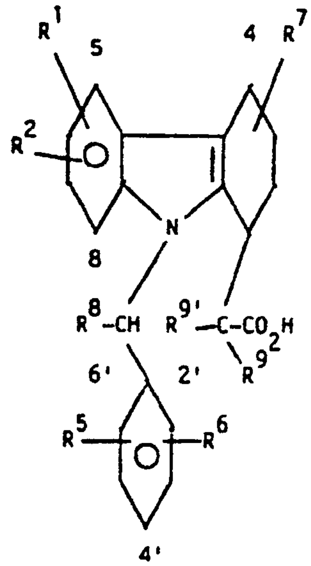

| Compound | $R^1$ | $R^2$ | $R^5$ | $R^6$ | $R^9$, $R^{9'}$ | $R^7$ | $R^8$ |
|---|---|---|---|---|---|---|---|
| 1 (Ex.1) | 6-F | H | 4'-Cl | H | H, H | H | H |
| 2 (Ex. 4) | 6-OMe | H | 4'-Cl | H | H, H | H | H |
| 3 (Ex. 7) | 6-F (-) isomer | H | 4'-Cl | H | H, H | H | H |
| 4 (Ex. 8) | 6-F (+) isomer | H | 4'-Cl | H | H, H | H | H |
| 5 (Ex. 9) | 6-F | H | H | H | H, H | H | H |
| 6 (Ex. 10) | 6-F | H | 4'-OMe | H | H, H | H | H |

| Compound | $R^1$ | $R^2$ | $R^5$ | $R^6$ | $R^9$, $R^{9'}$ | $R^7$ | $R^8$ |
|---|---|---|---|---|---|---|---|
| 7 (Ex. 11) | 6-F | H | 3'-Cl | 4'-Cl | H, H | H | H |
| 8 (Ex. 12) | 6-F | H | H | H | H, H | H | Me |
| 9 (Ex 13) | H | H | 4'-Cl | H | H, H | H | H |
| 10 (Ex. 14) | 6-Cl | H | 4'-Cl | H | H, H | H | H |
| 11 (Ex. 15) | 8-Me | H | 4'-Cl | H | H, H | H | H |
| 12 (Ex. 16) | 6-Br | H | 4'-Cl | H | H, H | H | H |
| 13 (Ex. 17) | 6-Me | H | 4'-Cl | H | H, H | H | H |
| 14 (Ex. 19) | 8-F | H | 4'-Cl | H | H, H | H | H |
| 15 (Ex. 20) | 5-F | H | 4'-Cl | H | H, H | H | H |
| 16 (Ex. 20) | 7-F | H | 4'-Cl | H | H, H | H | H |
| 17 (Ex. 21) | 5-Cl | 7-Cl | 4'-Cl | H | H, H | H | H |
| 18 (Ex. 22) | 6-Cl | 8-Cl | 4'-Cl | H | H, H | H | H |
| 19 (Ex. 18) | 6-F | H | 4'-Cl | H | Me, H | H | H |
| 20 | 6-F | H | 4'-Cl | H | Me, Me | H | H |
| 21 | 6-F | H | 4'-Cl | H | H, H | 1-Me | H |

| Compound | $R^1$ | $R^2$ | $R^5$ | $R^6$ | $R^9$,$R^{9'}$ | $R^7$ | $R^8$ |
|---|---|---|---|---|---|---|---|
| 22 | 8-Br | H | 4'-Cl | H | H,H | H | H |
| 23 (Ex. 23) | 6-$CF_3$ | H | 4'-Cl | H | H,H | H | H |
| 24 (Ex. 24) | 6-SMe | H | 4'-Cl | H | H,H | H | H |
| 25 (Ex. 25) | 6-S(O)Me | H | 4'-Cl | H | H,H | H | H |
| 26 (Ex. 26) | 6-$S(O)_2$Me | H | 4'-Cl | H | H,H | H | H |
| 27 (Ex. 27) | 8-SMe | H | 4'-Cl | H | H,H | H | H |
| 28 (Ex. 28) | 8-S(O)Me | H | 4'-Cl | H | H,H | H | H |
| 29 (Ex. 29) | 6-F | H | 4'-Cl | H | H,H | 3-Me | H |
| 30 (Ex. 30) | 6-F | 8-F | 4'-Cl | H | H,H | H | H |
| 31 (Ex. 31) | 6-Me | 8-Me | 4'-Cl | H | H,H | H | H |
| 32 (Ex. 32) | 6-OMe | 8-Me | 4'-Cl | H | H,H | H | H |
| 33 (Ex. 33) | 6-F(-)Isomer | 8-F | 4'-Cl | H | H,H | H | H |

| Compound | $R^1$ | $R^2$ | $R^5$ | $R^6$ | $R^9$,$R^{9'}$ | $R^7$ | $R^8$ |
|---|---|---|---|---|---|---|---|
| 34 (Ex. 34) | 6-F(+)Isomer | 8-F | 4'-Cl | H | H,H | H | H |
| 35 (Ex. 35) | 8-Me(-)Isomer | H | 4'-Cl | H | H,H | H | H |
| 36 (Ex. 36) | 8-Me(+)Isomer | H | 4'-Cl | H | H,H | H | H |
| 37 (Ex. 37) | 8-F(-)Isomer | H | 4'-Cl | H | H,H | H | H |
| 38 (Ex. 38) | 8-F(+)Isomer | H | 4'-Cl | H | H,H | H | H |
| 39 | 6-F | 8-F | 3'-Cl | 4'-Cl | H,H | H | H |
| 40 | 6-F | 8-F | 2'-Cl | 4'-Cl | H,H | H | H |
| 41 | 6-F | 8-F | 4'-OMe | H | H,H | H | H |
| 42 | 6-F | 8-F | 4'-OH | H | H,H | H | H |
| 43 | 6-F | 8-F | 4'-SMe | H | H,H | H | H |
| 44 | 6-F | H | 4'-S(O)Me | H | H,H | H | H |
| 45 | 6-F | 8-F | 4'-NHCOMe | H | H,H | H | H |
| 46 | 6-F | H | 4'-$S(O)_2$Me | H | H,H | H | H |
| 47 | 6-F | H | 4'-F | H | H,H | H | H |
| 48 | 6-F | H | 4'-Br | H | H,H | H | H |

| Compound | $R^1$ | $R^2$ | $R^5$ | $R^6$ | $R^9$,$R^{9'}$ | $R^7$ | $R^8$ |
|---|---|---|---|---|---|---|---|
| 49 | 6-F | 8-Me | 4'-Cl | H | H,H | H | H |
| 50 | 6-F | H | 4'-$CO_2H$ | H | H,H | H | H |
| 51 | 6-F | H | 4'-$CO_2Me$ | H | H,H | H | H |
| 52 | 6-F | 8-F | 4'-n-$C_3H_7$ | H | H,H | H | H |
| 53 | 6-F | 8-F | 3'-I | 4'-OH | H,H | H | H |
| 54 | 6-F | 8-F | 4'-I | H | H,H | H | H |
| 55 | 6-$N_3$ | H | 4'-Cl | H | H,H | H | H |
| 56 | 6-F | H | 4'-$N_3$ | H | H,H | H | H |

The following reaction schemes illustrate the preparation of the compounds of the present invention :

Scheme I    Preparation of Formula I Compounds

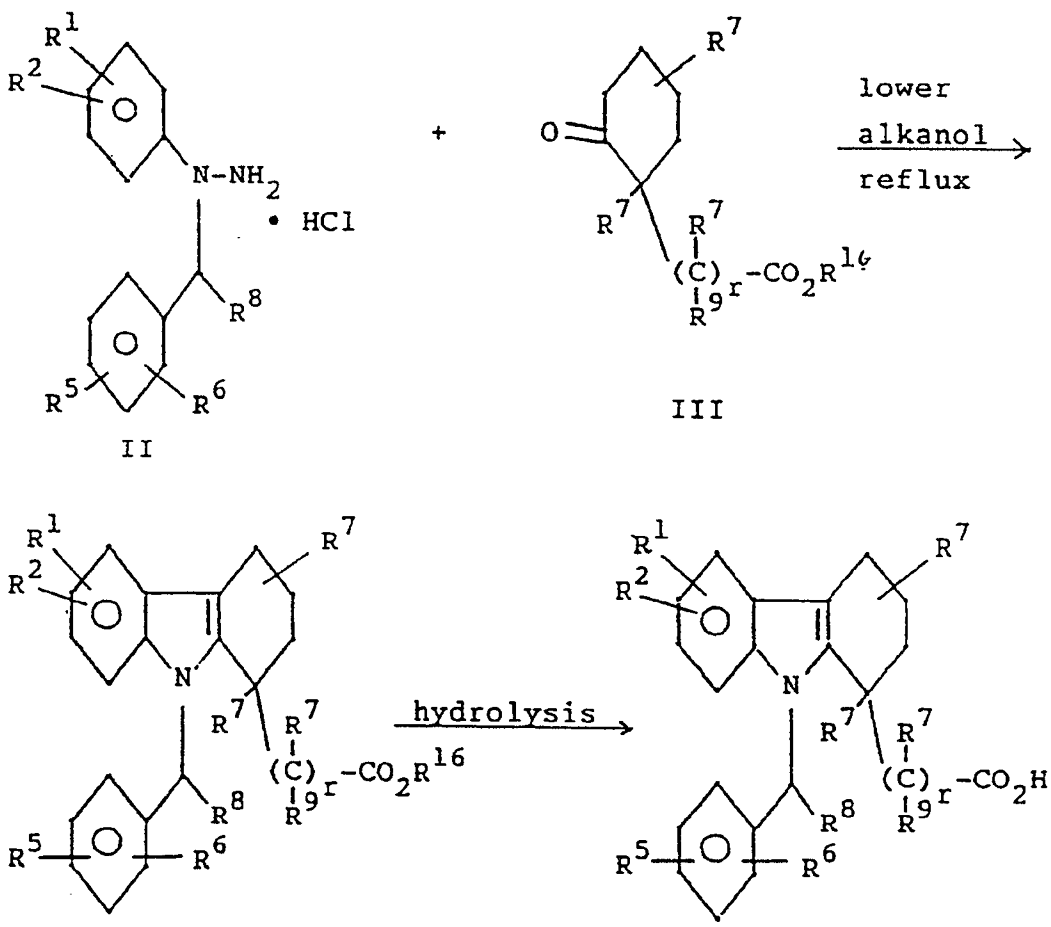


II    III

IIIa    I

The reaction can be conveniently carried out in an alcohol solvent such as t-butanol, i-butanol, and the like. The hydrolysis of the ester intermediates IIIa is conveniently carried out by using NaOH or KOH in aqueous ethanol or methanol followed by acidification to obtain compounds of Formula I.

The following ketones (1,2,4) of structure III are known in the art, and ketone 3 is readily prepared by procedures analogous to those for the known ketones.

TABLE 2

ketones of Formula III

| No. | Structure | Reference |
| --- | --- | --- |
| 1. | 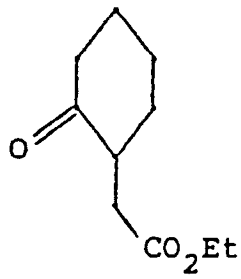 | Ethyl 2-cyclohexanone acetate; commercially available (Aldrich) |
| 2. | 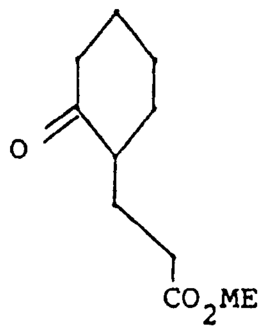 | Methyl 2-cyclohexanone propionate; J.A.C.S. 85 207 (1963) G. Stork, A. Brizzolara, H. Landesman, J. Scmuszkovicz and R. Terrell |
| 3. | 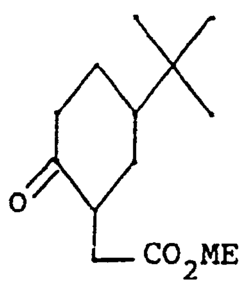 | Methyl 4-t-butyl-2-cyclohexanone acetate |

TABLE 2 (Cont'd)

4.

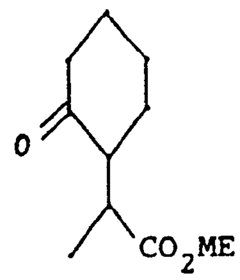

Methyl 2-(2-cyclohexanone) propionate
J.A.C.S. 85 207 (1963)
G. Stork, A. Brizzolara, H. Landesman, J. Scmuszkovicz and R. Terrell

5.

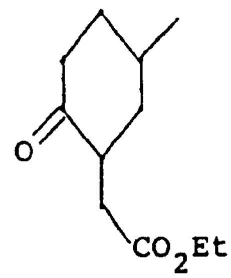

Ethyl 4-methyl-2-cyclohexanone acetate

The sequence described above is an application of the Fischer Indole Synthesis. Numerousindole syntheses are described in reviews, such as, for example "Heterocyclic Compounds" Volume 25, Parts 1, 11, 111, W. J. Houlihan (Ed.), Interscience, J. Wiley & Sons, N. Y., 1979. Appropriate manipulations of functional groups using sequences described in such reviews will lead to the compounds of the present invention.

Scheme II Preparation of Hydrazine Derivatives (II)

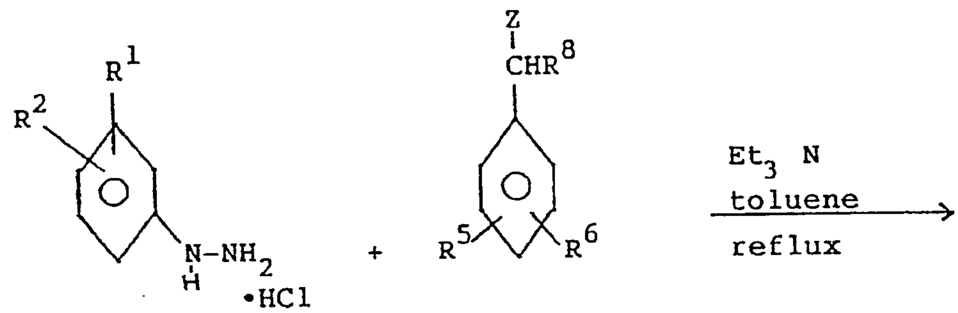


(IV) (V)

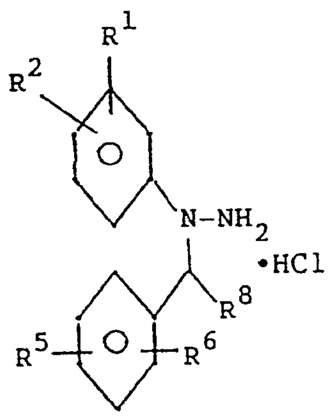

II

Z is a leaving group such as Cl, Br, I, mesylate or tosylate

With regard to Scheme II, the preparation of hydrazine starting materials is illustrated by the preparation of 1-(4-chlorobenzyl)-1-(4-methoxyphenyl)hydrazine. A mixture of 10 g of p-methoxyphenylhydrazine hydrochloride, 75 ml of toluene and 11.5 ml of triethylamine was heated at reflux for 60 minutes. Then, 7.1 g of p-chlorobenzyl chloride was added. After stirring 16 hours at reflux, triethylamine hydrochloride was filtered off and washed with ethyl ether. The filtrate and washing were concentrated in vacuo and chromatographed on a silica gel column (hexane-ethyl acetate, 9 : 1) to give 6.64 g of 1-(4chlorobenzyl)-1-(4-methoxyphenyl)hydrazine. Other hydrazines, similarly prepared, are also shown in Table 3, below.

TABLE 3

Hydrazines

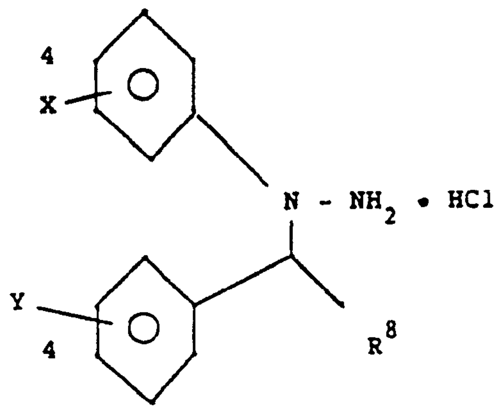

| Compound No. | X | Y | $R^8$ | Compound Name |
|---|---|---|---|---|
| 1. | 4-F | 4-Cl | H | 1-(4-chlorobenzyl)-1-(4-fluorophenyl) hydrazine hydrochloride |
| 2. | 3,5-$Cl_2$ | 4-Cl | H | 1-(4-chlorobenzyl)-1-(3,5-dichlorophenyl)hydrazine hydrochloride |

TABLE 3 (Cont'd)

| | | | | |
|---|---|---|---|---|
| 3. | 4-OMe | 4-Cl | H | 1-(4-chlorobenzyl)-1-(4-methoxyphenyl) hydrazine hydrochloride |
| 4. | 2-Me | 4-Cl | H | 1-(4-chlorobenzyl)-1-(2-methylphenyl) hydrazine hydrochloride |
| 5. | 4-Me | 4-Cl | H | 1-(4-chlorobenzyl)-1-(4-methylphenyl) hydrazine hydrochloride |
| 6. | 4-Cl | 4-Cl | H | 1-(4-chlorobenzyl)-1-(4-chlorophenyl) hydrazine hydrochloride |
| 7. | H | 4-Cl | H | 1-(4-chlorobenzyl)-1-(phenyl) hydrazine hydrochloride |
| 8. | 4-Br | 4-Cl | H | 1-(4-chlorobenzyl)-1-(4-bromophenyl) hydrazine hydrochloride |
| 9. | 3-F | 4-Cl | H | 1-(4-chlorobenzyl)-1-(3-fluorophenyl) hydrazine hydrochloride |
| 10. | 2,4-$Cl_2$ | 4-Cl | H | 1-(4-chlorobenzyl)-1-(2,4-dichlorophenyl)hydrazine hydrochloride |

TABLE 3 (Cont'd)

| | | | | |
|---|---|---|---|---|
| 11. | 4-F | H | H | 1-(benzyl)-1-(4-fluorophenyl) hydrazine hydrochloride |
| 12. | 4-F | 4-OMe | H | 1-(4-methoxybenzyl)-1-(4-fluorophenyl) hydrazine hydrochloride |
| 13. | 4-F | 3,4-$Cl_2$ | H | 1-(3,4-dichlorobenzyl)-1-(4-fluoro-phenyl) hydrazine hydrochloride. |
| 14. | 4-F | H | $CH_3$ | 1-[1-(phenyl)ethyl]-1-(4-fluorophenyl) hydrazine hydrochloride |
| 15. | 2-F | 4-Cl | H | 1-(4-chlorobenzyl)-1-(2-fluorophenyl) hydrazine hydrochloride. |

16. 1-[2-(4-chlorophenyl)ethyl]-1-(4-fluorophenyl) hydrazine hydrochloride.

17. 1-(2-propyl)-1-(4-fluorophenyl)hydrazine hydrochloride.

TABLE 3 (Cont'd)

| | | | | |
|---|---|---|---|---|
| 18. | 4-$CF_3$ | 4-Cl | H | 1-(4-chlorobenzyl)-1-(4-trifluoromethylphenyl)-hydrazine hydrochloride |
| 19. | 4-SMe | 4-Cl | H | 1-(4-chlorobenzyl)-1-(4-methylthiophenyl)hydrazine hydrochloride |

Scheme III Alternative Preparation of Formula I Compounds

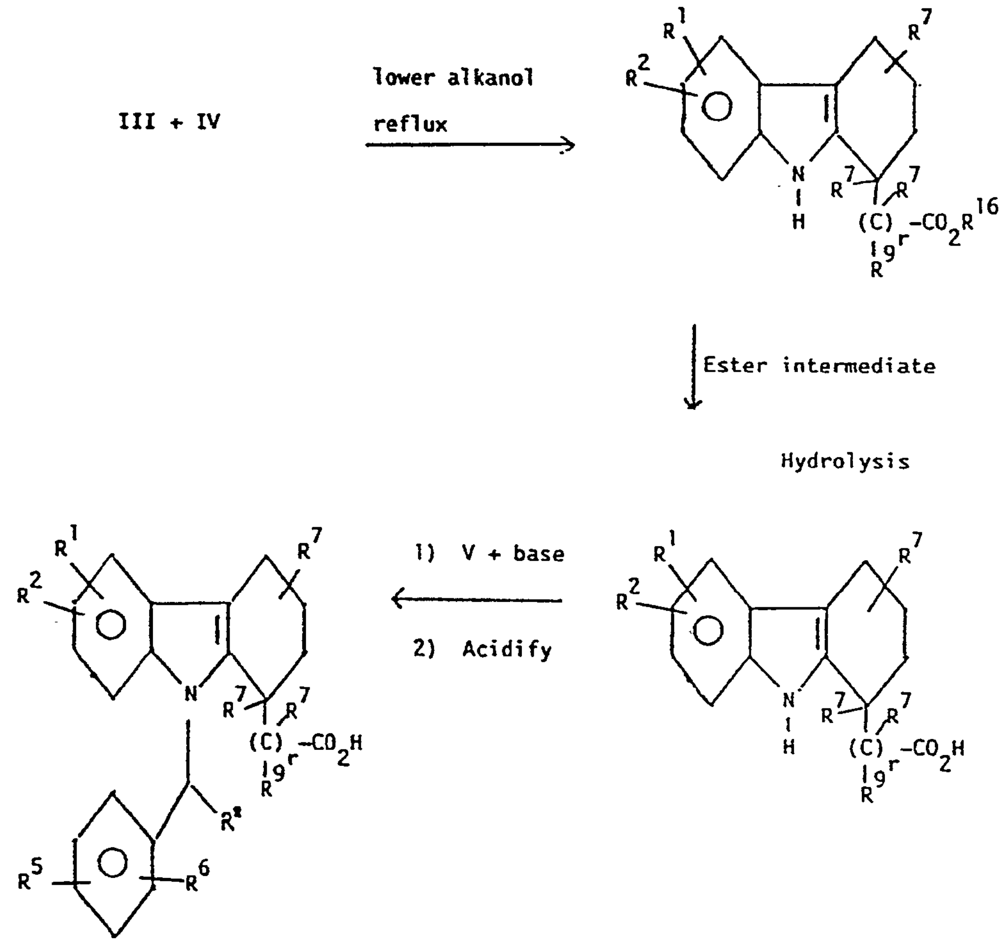


I

Acid intermediate

Scheme III illustrates an alternative synthesis of the compounds of Formula I. In this Scheme a Fischer indole synthesis is carried out using a phenylhydrazine IV and the ketone III, followed by hydrolysis. The acid intermediate is then N-benzylated with the reagent V, preferably using a strong base such as potassium t-butoxide to effect the reaction. Acidification of the reaction mixture then yields the free acid of I.

Scheme IV   Preparation of Sulfoxides and Sulfones of Formula I Compounds

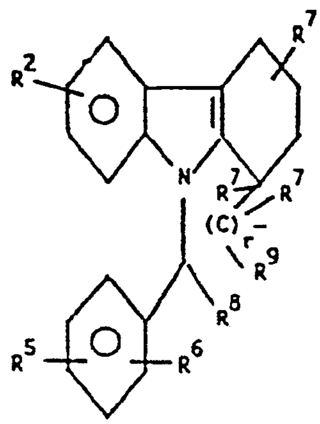

= TC below.

$R^1$ is replaced by $SR^{13}$, $S(O)R^{13}$ or $S(O)_2R^{13}$

$R^2$-$R^6$ ≠ $SR^{13}$ or $S(O)R^{13}$

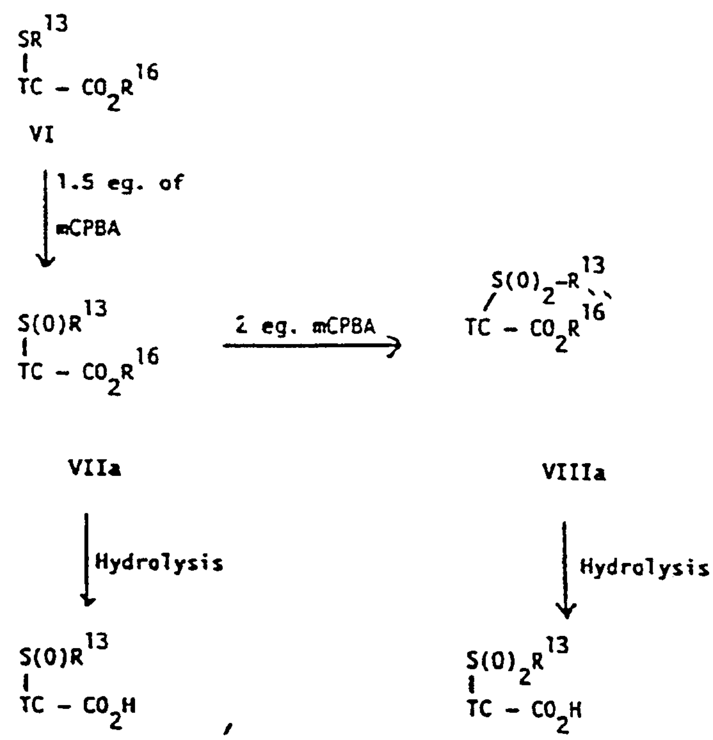


VI

VIIa

VIIIa

VII , VIII

In Scheme IV is illustrated a method of preparing derivatives of Formula I in which one of the substituents $R^1$ or $R^2$ is a sulfoxide or a sulfone. It will be obvious to one skilled in the art that a sulfoxide or sulfone derivative of $R^5$ or $R^6$ could be prepared in the same way.

Ester VI (a representative of III a, Scheme I) is prepared according to Scheme I or Scheme III followed by esterification of acid I. Treatment of VI with a limited amount of an oridizing agent such as m-chloro-perbenzoic acid yields the sulfoxide ester VIIa, which upon hydrolysis yields sulfoxide acid VII. Further treatment of VIIa with the oridizing agent, or treatment of VI with an excess (>2 eq.) of the oridizing agent yields the sulfone ester VIIIa, hydrolysis of which yields the sulfone acid VIII. Both VII and VIII are representatives of Formula I compounds.

Scheme V    Preparation of Further compounds of Formula I

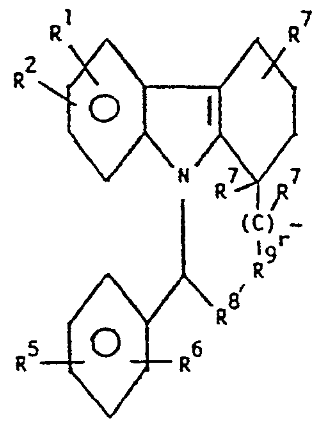

= THC below

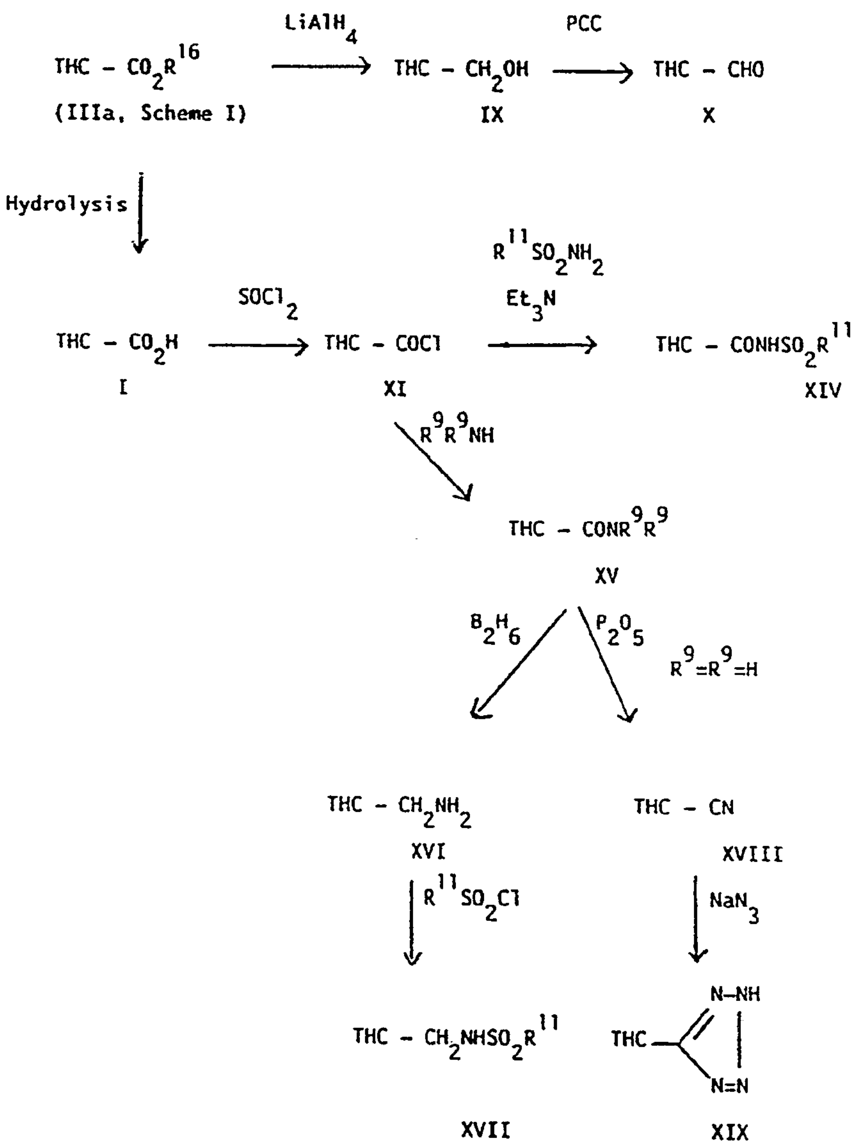

Other compounds of Formula I can be prepared as indicated in Scheme V. Thus the ester derivative IIIa can be reduced to the alcohol IX by lithium aluminum hydride or other suitable reducing agents. Alcohol IX can then be oxidized to aldehyde X by pyridinium chlorochromate or other suitable oxidizing agents. Carboxylic acids of Formula I can be converted to the acid chloride XI (the acid bromide or a mired carbonate anhydride could also be used).

Acid chloride XI, upon reaction with a sulfonamide, $R^{11}SO_2NH_2$, in the presence of a weak base yields the acyl-sulfonamide XIV. Reaction of XI with an amine, $R^9R^9NH$, yields amide XV. Amide XV can be sequentially reduced, to amine XVI, with diborane or lithium aluminum hydride, and sulfonylated with $R^{11}SO_2Cl$ to produce sulfonamide XVII. Amide XV (when both $R^9$ substituents are hydrogen) can be dehydrated by standard reagents to nitrile XVIII, which is converted to tetrazole XIX by reaction with sodium azide, tri-n-butyltin azide or other suitable methods. Compounds IX, X, XIV, XV, XVII, XVIII and XIX are representatives of Formula I compounds.

Scheme VI Preparation of Hydrazine Derivatives IV

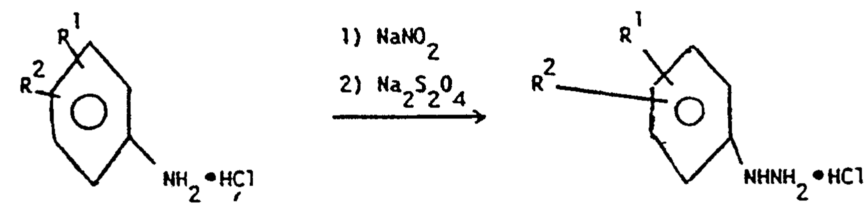


IV

With regard to Scheme VI, the preparation of hydrazine starting materials is illustrated by preparation of 4-methylthiophenyl hydrazine hydrochloride. 4-Methylthioaniline (13.9 g) was added dropwise to cold HC1 (6N) (50 mL) and stirred for 5 min in an ice bath. A solution of $NaNO_2$ in water (7.25 g, 15 mL) was then added dropwise and stirred for 15 min. The cold diazonium salt was then cannulated into a stirred cold solution of $Na_2S_2O_4$ in water (50 g, 250 mL). After 20 min, ether (200 mL) was added and the reaction mixture basified with NaOH(10N). The ether layer was decanted, washed with brine, dried over $Na_2SO_4$ and HCl gas was passed through the ether solution to form the hydrochloride salt which precipitated out. After filtration, there was obtained 7.0 g of pure final product. Other hydrazines, similarly prepared, are also shown in Table 3a, below.

TABLE 3a

HYDRAZINES

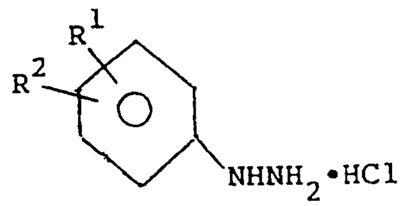

IV

| No. | $R^1$ | $R^2$ | Compound Name |
|---|---|---|---|
| 1 | 4-SMe | H | 4-methylthiophenyl hydrazine hydrochloride |

TABLE 4 (Cont'd)

| | | | |
|---|---|---|---|
| 2 | 2-SMe | H | 2-methylthiophenyl hydrazine hydrochloride |
| 3 | 2-Me | 4-Me | 2,4-dimethylphenyl hydrazine hydrochloride |
| 4 | 2-Me | 4-OMe | 4-methoxy-2-methylphenyl hydrazine hydrochloride |

In those instances when asymmetric centers are present, more than one stereoisomer is possible, and all possible isomeric forms are deemed to be included within the planar structural representations shown. Optically active (R) and (S) isomers may be resolved using conventional techniques known to the skilled artisan.

The prostaglandin antagonist properties of the compounds of the present invention can be demonstrated by a number of biological assays, two of which, inhibition of platelet aggregation and measurement of $pA_2$ valves are described below.

Inhibition of Induced Threshold Aggregation of Human platelets

Human platelet rich plasma (PRP) is prepared from venous blood of male volunteers who have taken no medication for ten days prior to test. Blood is transferred into plastic centrifuge tubes containing 3.8% Trisodium Citrate in 0.9% NaCl (in a ratio of blood to anticoagulant of 9 : 1), mixed by gentle inversion, and

centrifuged at room temperature for ten minutes at 116 g. The supernatant (PRP) is transferred into plastic tubes. Platelet poor plasma (PPP) is obtained by centrifuging the residual blood cells at 4000 g for ten minutes. PRP is left to stand at least one half hour prior to testing.

Platelet Aggregation is measured using a Payton Aggregometer and Recorder. Following calibration of the instrument, a cuvette containing PRP (225 microliters) is incubated for three minutes at 37°C. Drug vehicle (control), or a drug concentration is then added in a volume of 0.5 microliter. After one minute, the aggregating agent (U44069, 9,11-dideoxy-9α,11α-epoxymethano $PGF_{2\alpha}$) is added to the cuvette in a volume of 25 microliters. Recording is continued until the maximal response is obtained.

The threshold (approximately 20-30% of maximum) aggregation concentration of the agonist to be used is first determined in the presence of the drug vehicle (control). Test compounds are then assayed at 10 or 30 micrograms/ml initially, and if active, are further tested in order to determine the concentration range at which 20-80% of the threshold aggregatory response is inhibited. All drugs are dissolved in dimethylsulfoxide.

The height of the aggregation response (measured in divisions of the recorder paper, 1 division = 2.5 mm) in the presence of the drug is recorded, and calculated as percent inhibition of the mean height of the control threshold responses. The $IC_{50}$ (drug concentration which inhibits 50% of the aggregatory response) is obtained by regression analysis.

Estimation of $pA_2$ Values in Guinea Pig Tracheal Chain

Male albino Hartley strain guinea pigs (300-350 gm) were sacrificed by a blow to the head and exsanguinated. The trachea was removed, freed of extraneous tissue and sectioned into rings of 1-2 mm thickness. Five rings were tied together in series, ensuring that the tracheal muscle lay in the same vertical plane, and the cartilage of each ring then separated at a point directly opposite the muscle. The chains were suspended under 1 gm resting tension in modified Krebs solution (NaCl, 6.87 ; $NaHCO_3$, 2.1 ; dextrose, 2.1 ; KCl, 0.32 ; $CaCl_2$, 0.28 ; $MgSO_4$, $7H_2O$, 0.11 ; $KH_2PO_4$, 0.16 ; gm/L : equilibrated with 5% $CO_2$ in $O_2$ for I hour) containing indomethacin ($1.4 \times 10^{-5}$M) to suppress endogenous protaglandin synthesis, Organ bath temperature was maintained at 37°C and 5% $CO_2$ in $O_2$ diffused continously. Isometric tension changes were recorded from a Gould-Statham (UTC 2) force displacement transducer connected to a Beckman Type R Dynograph. For assay purposes initial maximal contractions were elicited with a high concentration of the contractile agonist [U-44069, 9.11-dideoxy-9α,11α-epoxymethano $PGF_{2\alpha}$] and the tissue subsequently washed at intervals until tension returned to baseline. Agonist dose response curves were obtained using a cumulative-dose schedule (4-8 doses) and the preparations then washed at regular intervals until baseline tension was recorded. After an appropriate interval (1-1.5 hrs) agonist dose response curves were repeated in the presence of antagonist drug concentrations. Drug doses were delivered in 10 µl volumes 5 minutes prior to the second agonist challenge, and cumulative agonist volumes did not exceed 100 µl per bath. $EC_{50}$ values were obtained by regression analysis and used to calculate 'apparent' and Schild Plot $pA_2$ values by the method of Tallarida and Murray 1981.

Compounds of Formula I can be tested using the following assay to determine their mammalian leukotriene biosynthesis inhibiting activity.

Rat Peritoneal PolymorPhonuclear (PMN) Leukocyte Assay

Rats under ether anesthesia are injected (i.p.) with 8 ml of a suspension of sodium caseinate (6 grams in ca. 50 ml water). After 15-24 hr. the rats are sacrificed ($CO_2$) and the cells from the peritoneal cavity are recovered by lavage with 20 ml of buffer (Eagles MEM containing 30 mM HEPES adjusted to pH 7.4 with NaOH). The cells are pelleted (350 × g, 5 min.), resuspended in buffer with vigorous shaking, filtered, through lens paper, recentrifuged and finally suspended in buffer at a concentration of 10 cells/ml. A 500 µl aliquot of PMN suspension and test compound are preincubate for 2 minutes at 37°C, followed by the addition of 10 µM A-23187. The suspension is stirred for an additional 4 minutes then bioassayed for $LTB_4$ content by adding an aliquot to a second 500 µl portion of the PMN at 37°C. The $LTB_4$ produced in the first incubation causes aggregation of the second PMN, which is measured as a change in light transmission. The size of the assay aliquot is chosen to give a submaximal transmission change (usually –70%) for the untreated control. The percentage inhibition of $LTB_4$ formation is calculated from the ratio of transmission change in the sample to the transmission change in the compound-free control.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-

inflammatory drugs on the gastrointestinal tract, animal studies show that cytoprotective compounds will prevent gastric lesions induced by oral administration of strong acids, strong bases, ethanol, hypertonic saline solutions and the like.

Two assays can be used to measure cytoprotective ability. These assays are ; (A) an ethanolinduced lesion assay and (B) an indomethacin-induced ulcer assay and are described in Ep 140,684.

In Table 4 below are presented data indicating the prostanoid antagonist activities of compounds of the present invention indicated in Table 1. It is to be noted that $pA_2$ values are on a logerithmic scale, so that, for instance, a difference between two $pA_2$ values of 1 represents a difference in potency by a factor of 10.

Compounds A, B, C and D in Table 4 are known in the art : U.S. Patent 3,896,145 describes compounds A and C, U.S. Patent 3,868,387 describes compound B, and compound D is described in U.S. Patent 3,905,998.

Compound A, which is a positional isomer of the novel compound 2, is nevertheless almost 12 times less potent as an inhibitor of platelet aggregation and its $pA_2$ is a factor of 14 (antilog of 1.6) less than that of compound 2. The homolog B, with one carbon less than A, shows no significant activity. Also of interest is the fact that the fully aromatic carbazole analog of the novel compound 2, compound C, does not possess any significant activity. Compounds D and E, isomeric with compound B further demonstrate that direct attachment of the carboxyl group to the tetrahydrocarbazole nucleus results in severe loss of activity.

Compound 8 indicates an interesting differentiation of prostaglandin antagonist activity, depending upon the tissue, with very weak action on the guinea pig trachea ($pA_2 < 6$), but with very good potency as an inhibitor of human platelet aggregation ($IC_{50} = 0.09$ µg/ml).

Compound F demonstrates that a hydrogen atom attached to the 9-position (the nitrogen atom) results in severe loss of activity. Likewise, compound G, with a 9-alkyl substituted, has almost completely lost activity. Compound H, with a 2-phenylethyl substituent at the 9-position has also suffered a serious loss of activity by the addition of a $CH_2$ group compared to Compound 1. The last three compounds (F, G and H) demonstrate the requirement for a benzyl or substituted benzyl group at position-9 in the compounds of the present invention.

## Table 4

## Prostanoid Antagonist Activities

| Compound | Inhibition of platelet aggregation ($IC_{50}$ in µg/ml) | $pA_2$ |
|---|---|---|
| A 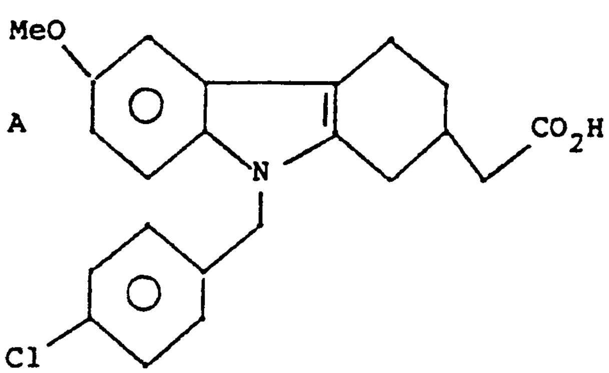 | 3.5 | 6.8 |

## Table 4 (Cont'd)

| | | | |
|---|---|---|---|
| B | 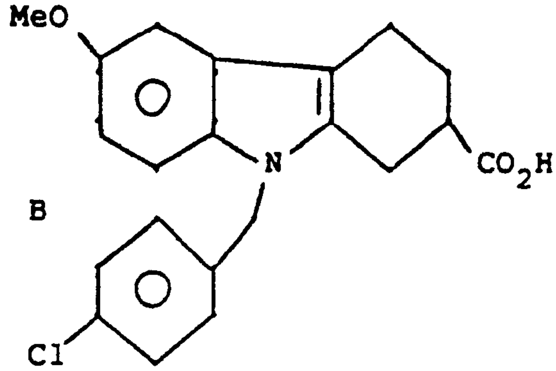 | >30 | <6 |
| C | 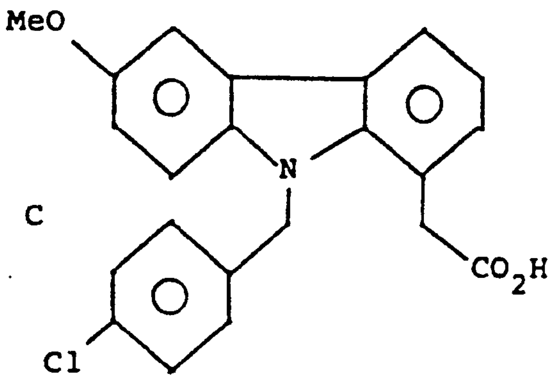 | >30 | 6.58 |
| D | 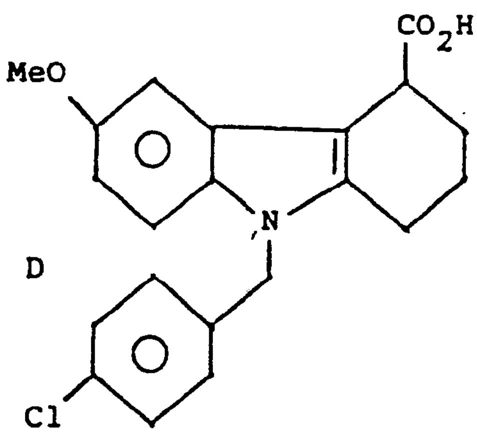 | | <6 |

## Table 4 (Cont'd)

| | | | |
|---|---|---|---|
| E | 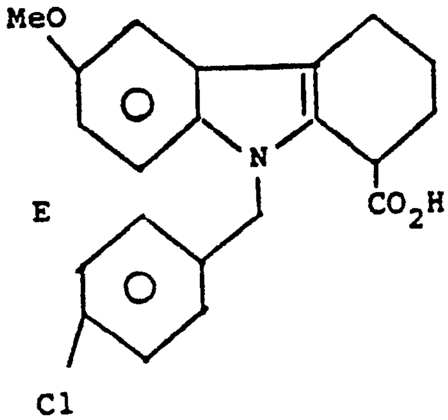 | 4.23 | 6.66 |
| F | 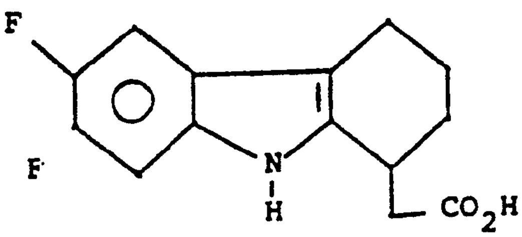 | 3.41 | < 6 |
| G | 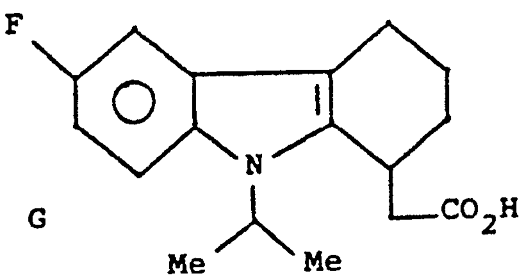 | 13.6 | < 6 |

Table 4 (Cont'd)

| | | | |
|---|---|---|---|
| H | 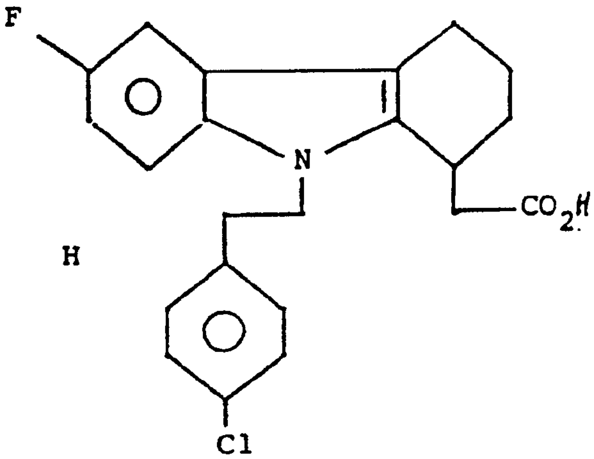 | 13.6 | 6.8 |
| 1 | 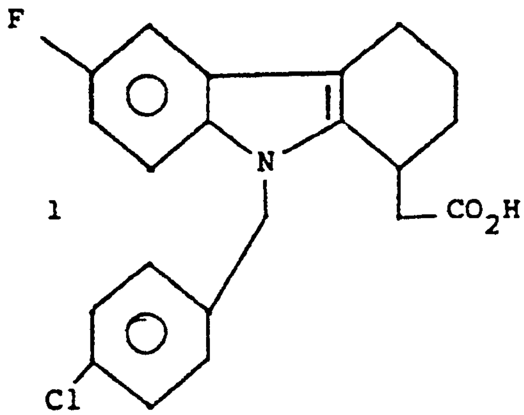 | 0.03 | 8.6 |
| 2 | 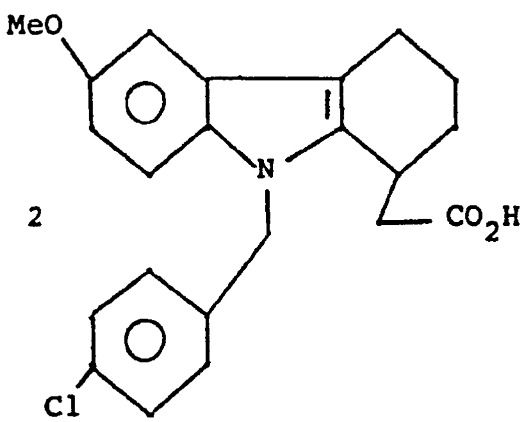 | 0.30 | 8.4 |

Table 4 (Cont'd)

| | | | |
|---|---|---|---|
| 3 | 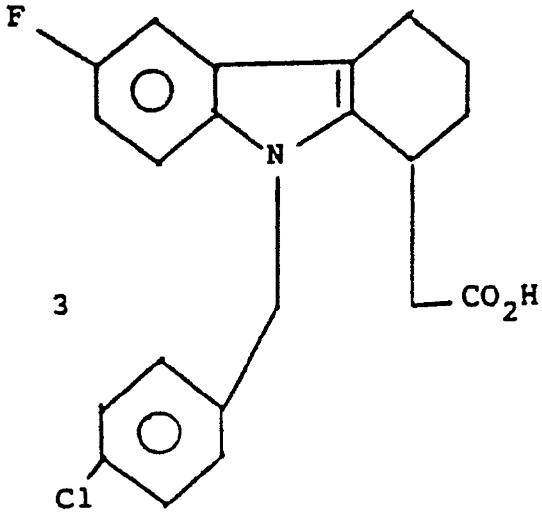 | 0.05 | 8.0 |
| 6 | 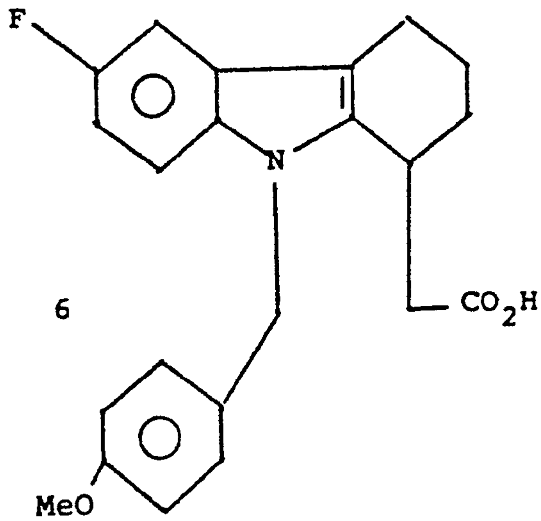 | 0.15 | 8.7 |

Table 4 (Cont'd)

| | | | |
|---|---|---|---|
| 7 | 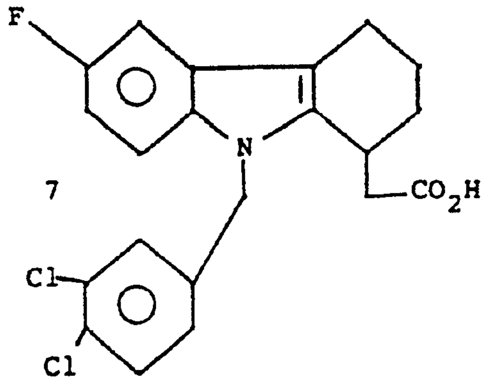 | 0.09 | 8.3 |
| 8 | 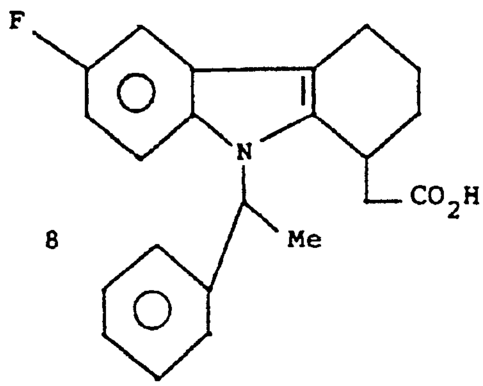 | 0.09 | < 6 |
| 9 | 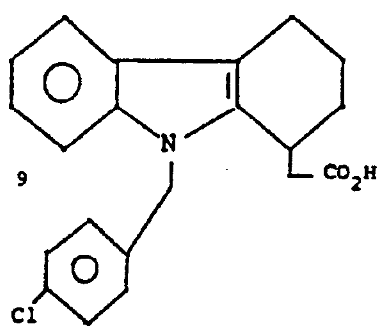 | 0.05 | 8.9 |

Table 4 (Cont'd)

| | | | |
|---|---|---|---|
| 10 | 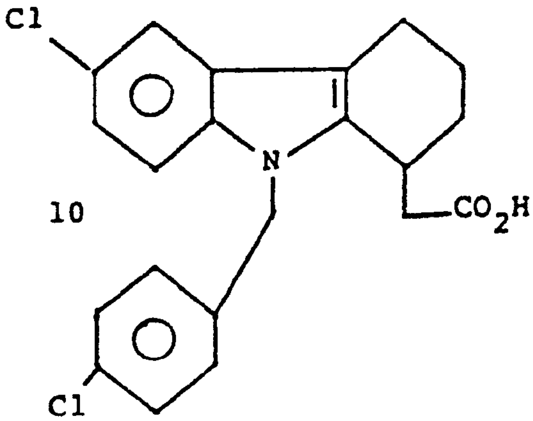 | 0.10 | 8.0 |
| 11 | 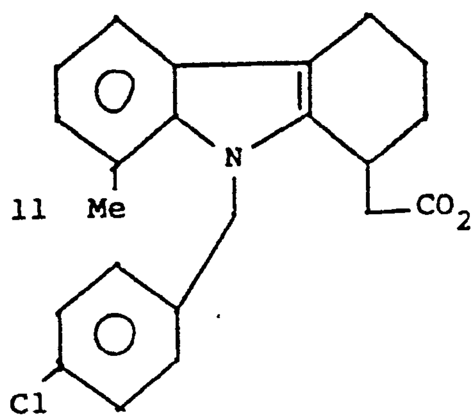 | 0.10 | 9.5 |

Table 4 (Cont'd)

| | | | |
|---|---|---|---|
| 12 | 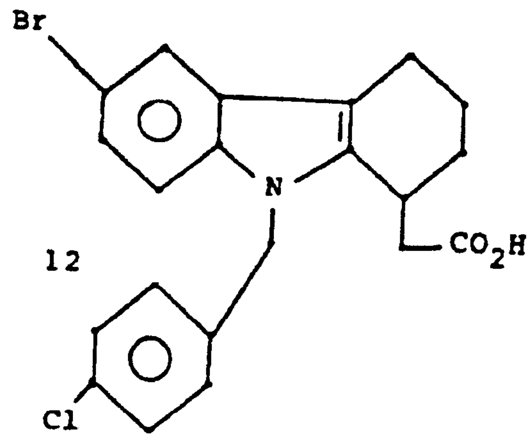 | 0.09 | 7.9 |
| 13 | 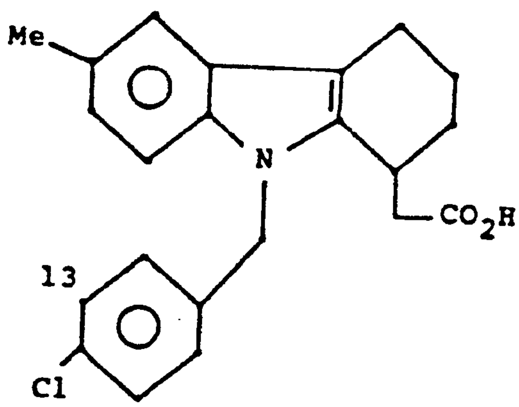 | 0.08 | 7.6 |
| 14 | 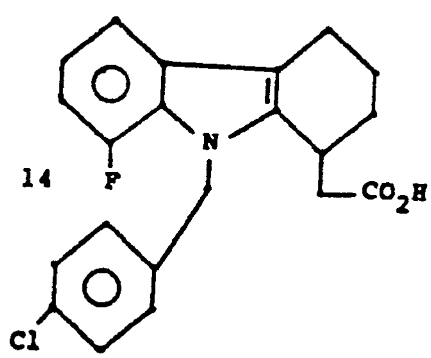 | 0.08 | 9.4 |

Table 4 (Cont'd)

| | | | |
|---|---|---|---|
| 15/16 | 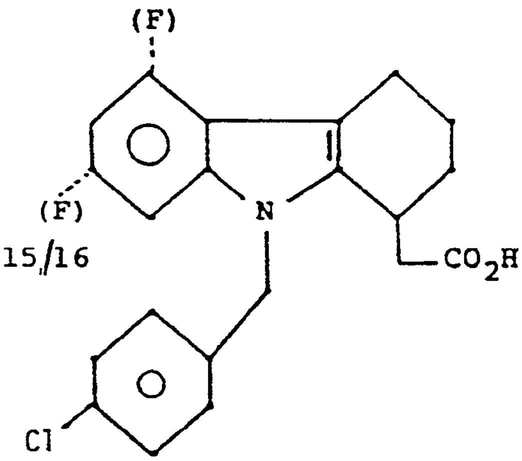 | 0.17 | 8.9 |
| 18 | 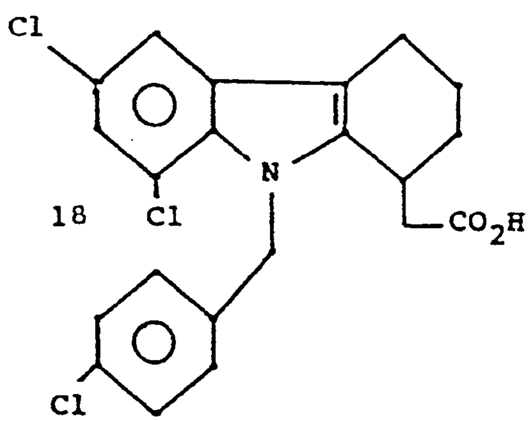 | 0.07 | 7.2 |

Table 4 (Cont'd)

| | | | |
|---|---|---|---|
| 19 | 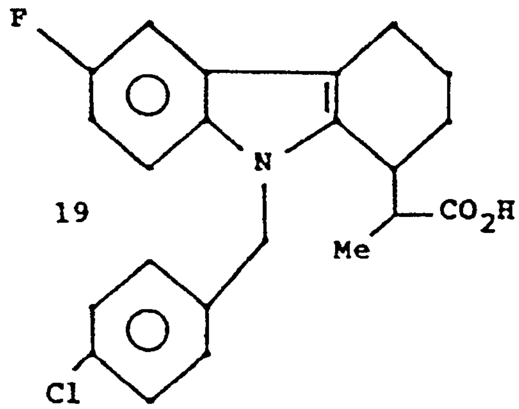 | 0.31 | 6.8 |

The magnitude of a prophylactic or therapeutic dose of a compound of Formula I will, of course, vary with the nature or the severity of the condition to be treated and with the particular compound of Formula I and its route of administration. In general, the daily dose range for anti-asthmatic, anti-allergic, or anti-thrombotic use lies within the range of from about 0.01 mg to about 100 mg per kg body weight of a mammal.

The exact amount of a compound of Formula I to be used as a cytoprotective agent will depend on, inter alia, whether it is being administered to heal damaged cells or to avoid future damage, on the nature of the damaged cells (e.g., gastro-intestinal ulcerations vs. nephrotic necrosis), and on the nature of the causative agent. An example of use of a compound of Formula I to avoid future damage is coadministration with a non-steroidal anti-inflammatory drug (for example, indomethacin).

The effective daily dosage level for compounds of Formula I inducing cytoprotection in mammals, especially humans, will generally range from about 0.002 mg/kg to about 100 mg/kg, preferably from about 0.02 mg/kg to about 30 mg/kg. The dosage may be administered in single or divided individual doses.

Any suitable route of administration may be employed for providing a mammal, especially a human with an effective dosage of a compound of Formula I. For example, oral, rectal, topical, parenteral, ocular, nasal, buccal, intravenous and the like may be employed. Dosage forms include tablets, troches, dispersions, suspensions, solutions, capsules, creams, ointments, aerosols and the like.

The pharmaceutical compositions of the present invention comprise a compound of Formula I as an active ingredient or a pharmaceutically acceptable salt thereof, and may also contain a pharmaceutically acceptable carrier and optionally other therapeutic ingredients. The term "pharmaceutically acceptable salts" refers to salts prepared from pharmaceutically acceptable non-toxic bases including inorganic bases and organic bases. The compositions include compositions suitable for oral, rectal, ophthalmic, pulmonary, nasal, dermal, topical or parenteral (including subcutaneous, intramuscular and intravenous) administration, although the most suitable route in any given case will depend on the nature and severity of the conditions being treated and on the nature of the active ingredient. They may be conveniently presented in unit dosage form and prepared by any of the methods well-known in the art of pharmacy.

For use where a composition for intravenous administration is employed, a suitable dosage range for anti-asthmatic, or anti-allergic use is from about 0.01 mg to about 20 mg (preferably from about 0.1 mg to about 10 mg) of a compound of Formula I per kg of body weight per day and for cytoprotective use from about 0.002 mg to about 100 mg (preferably from about 0.02 mg to about 30 mg and more preferably from about 0.1 mg to about 10 mg) of a compound of Formula I per kg of body weight per day. In the case where an oral composition is employed, a suitable dosage range for anti-asthmatic, or anti-allergic use is, e.g. from about 1 to about 100 mg of a compound of Formula I per kg of body weight per day, preferably from about 5 mg to about 40 mg per kg and for cytoprotective use from about 0.01 mg to about 100 mg (preferably from about 0.1 mg to about 3 mg and were preferably from about 0.1 mg to about 10 mg) of a compound of Formula I per kg of body weight per day.

For administration by inhalation, the compounds of the present invention are conveniently delivered in the form of an aerosol spray presentation from pressurized packs or a nebuliser, or a powder which may be formulated as a cartridge from which the powder composition may be inhaled with the aid of a suitable

device. The preferred delivery system for inhalation in a metered dose inhalation (MDI) aerosol, which may be formulated as a suspension or solution in fluorocarbon propellants.

Suitable topical formulations of compound I include transdermal devices, aerosols, creams, ointments, lotions, dusting powder, and the like.

In practical use, a compound of Formula I can be combined as the active ingredient in intimate admixture with a pharmaceutical carrier according to conventional pharmaceutical compounding techniques. The carrier may take a wide variety of forms depending on the form of preparation desired for administration, e.g., oral or parenteral (including intravenous). In preparing the compositions for oral dosage form, any of the usual pharmaceutical media may be employed, such as, for example, water glycols, oils, alcohols, flavoring agents, preservatives, coloring agents and the like in the case of oral liquid preparations, such as, for example, suspensions, elixirs and solutions ; or carriers such as starches, sugars, microcrystalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form, in which case solid pharmaceutical carriers are obviously employed. If desired, tablets may be sugar coated or enteric coated by standard techniques.

In addition to the common dosage forms set out above, the compounds of Formula I may also be administered by controlled release means and/or delivery devices such as those described in U.S. Patent Nos. 3,845,770 ; 3,916,899 ; 3,536,809 ; 3,598,123 ; 3,630,200 and 4,008,719, the disclosure of which is hereby incorporated herein by reference.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-agueous liguid, an oil-in-water emulsion or a water-in-oil liquid emulsion. Such compositions may be prepared by any of the methods of pharmacy but all methods include the step of bringing into association the active ingredient with the carrier which constitutes one or more necessary ingredients. In general, the compositions are prepared by uniformly and intimately admixing the active ingredient with liquid carriers or finely divided solid carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surface active or dispersing agent. Molded tablets may be made by molding in a suitable machine, a mixture of the powdered compound moistened with an inert liquid diluent. Desirably, each tablet contains from about 2.5 mg to about 500 mg of the active ingredient and each cachet or capsule contains from about 2.5 to about 500 mg of the active ingredient.

The following are examples of representative pharmaceutical dosage forms for the compounds of Formula I :

| Injectable Suspension (I.M.) | mg/ml |
|---|---|
| Compound of Formula I | 2.0 |
| Methylcellulose | 5.0 |
| Tween 80 | 0.5 |
| Benzyl alcohol | 9.0 |
| Benzalkonium chloride | 1.0 |
| Water for injection to a total volume of 1 ml | |

| Tablet | mg/tablet |
|---|---|
| Compound of Formula I | 25.0 |
| Microcrystalline Cellulose | 415.0 |
| Providone | 14.0 |
| Pregelatinized Starch | 43.5 |
| Magnesium Stearate | 2.5 |
| | 500 |

| Capsule | mg/capsule |
|---|---|
| Compound of Formula I | 25.0 |
| Lactose Powder | 573.5 |
| Magnesium Stearate | 1.5 |
| | 600 |

In addition to the compounds of Formula I, the pharmaceutical compositions of the present invention can also contain other active ingredients, such as non-steroidal anti-inflammatory drugs (NSAIDs), peripheral analgesic agents such as zomepirac, diflunisal and the like, cyclooxygenase inhibitors, leukotriene antagonists, leukotriene biosynthesis inhibitors, $H_2$-receptor antagonists, antihistaminic agents, prostaglandin antagonists, ACE inhibitors, and thromboxane synthetase inhibitors.

The weight ratio of the compound of the Formula I to the second active ingredient may be varied and will depend upon the effective dose of each ingredient. Generally, an effective dose of each will be used. Thus, for example, when a compound of the Formula I is combined with a second active ingredient the weight ratio of the compound of the Formula I to the second ingredient will generally range from about 1000: 1 to about 1 : 1000, preferably from 200 : 1 to 1 : 200. Combinations of a compound of the Formula I and other active ingredients will generally be within the aforementioned range, but in each case, an effective dose of each active ingredient should be used.

NSAIDs can be characterized into five groups :

(1) the propionic acid derivatives ;

(2) the acetic acid derivatives ;

(3) the fenamic acid derivatives ;

(4) the biphenylcarboxylic acid derivatives ; and

(5) the oxicams or a pharmaceutically acceptable salt thereof. NSAIDS which are within the scope of this invention are those disclosed in EP 140,684.

Pharmaceutical compositions comprising the Formula I compounds may also contain other inhibitors of the biosynthesis of the leukotrienes such as are disclosed in EP 138,481 (April 24, 1985), EP 115,394 (August 8, 1984), EP 136,893 (April 10, 1985), and EP 140,709 (May 8, 1985), which are hereby incorporated herein by reference.

The compounds of the Formula I may also be used in combination with leukotriene antagonists such as those disclosed in EP 106,565 (April 25, 1984) and EP 104,885 (April 4, 1984), which are hereby incorporated herein by reference and others known in the art such as those disclosed in European Patent Application Nos. 56,172 and 61,800 ; and in U.K. Patent Specification No. 2,058,785, which are hereby incorporated herein by reference.

Pharmaceutical compositions comprising the Formula I compounds may also contain as the second active ingredient, other prostaglandin antagonists such as those disclosed in European Patent Application 11,067 (May 28, 1980) or other thromboxane antagonists such as those disclosed in U.S. 4,237,160. They may also contain histidine decarboxyase inhibitors such as α-fluoromethyl-histidine, described in U.S. 4,325,961. The compounds of the Formula I may also be advantageously combined with an $H_1$ or $H_2$-receptor antagonist, such as for instance benadryl, dramamine, histadyl, phenergan, terfenadine, acetamazole, cimetidine, ranitidine, famotidine, aminothiadiazoles disclosed in EP 40,696 (December 2, 1981) and like compounds, such as those disclosed in U.S. Patent Nos. 4,283,408 ; 4,362,736 ; 4,394,508 ; and a pending application, U.S.S.N. 301,616, filed September 14, 1981. The pharmaceutical compositions may also contain a $K^+/H^+$ ATPase inhibitor such as omeprazole, disclosed in U.S. Pat. 4,255,431, and the like. Compounds of I may also be usefully combined with most cell stabilizing agents, such as 1,3-bis(2carboxychromon-5-yloxy)-2-hydroxypropane and related compounds described in British Patent Specifications 1,144,905 and 1,144,906. Another useful pharmaceutical composition comprises the Formula I compounds in combination with serotonin antagonists such as methysergide, the serotonin antagonists described in Nature, Vol. 316, pages 126-131, 1985, and the like. Each of the references referred to in this paragraph is hereby incorporated herein by reference.

When the second active ingredient in compositions of this invention is a thromboxane synthetase inhibitor, such inhibitor can be as described in UK 2,038,821 (e.g., UK 37248 and dazoxiben hydrochloride), U.S.P. 4,217,357 (e.g., UK 34787), U.S.P. 4,444,775 (e.g., CGS 13080), U.S.P. 4,226,878 (e.g., ONO 046), U.S.P. 4,495,357 (e.g., U63557A) U.S.P. 4,273,782 (e.g., UK-38485), or EP 98,690 (e.g., CV-4151).

An embodiment of the invention is a cardiovascular composition useful for treating arterial thrombosis which comprises an antithrombotic compound of the Formula I.

A further embodiment of the invention is a cardiovascular composition useful for treating arterial thrombosis which comprises : (1) the antithrombotic Formula I compound defined above ; and, (ii) an angiotensin converting enzyme (ACE) inhibitor compound which is a member of the group : carboxyalkyl dipeptide derivatives ; captopril [1-(3-mercapto-2methyl-1-oxopropyl)-L-proline] ; 2-[N-(S)-1-ethoxycarbonyl-3-phenylpropyl)-S-alanyl]-cis, endo-2-azabicyclo[3,3,0] octane-3(S)-carboxylic acid ; N-((S)-1-ethoxycarbonyl-3-phenylpropyl)-L-alanyl-N-(2-indanyl) glycine ; 1-(N-[(S)-1-ethoxy-carbonyl-3-phenylpropyl]-L-alanyl)-cis, syn-octahydro-(H-indole-2-S)-carboxylic acid ; 2-(N-[(S)-1-ethoxy-carbonyl-3-phenylpropyl]-Lalanyl)-1,2,3,4-tetrahydro-iso-isoguinoline-3(S)-carboxylic acid ; and, 1-carboxy-methyl-3(S)-(1(S)-ethoxycarbonyl-3-phenylpropylamino)-2,3,4,5-tetrahydrolH[1]-benzazepine -2-one.

In particular the class of ACE inhibitors which have been found to have a potentiating effect when used in combination with the Formula I compounds are those disclosed in U.S. Patent 4,374,829, which also discloses methods for their preparation and which patent is incorporated herein by reference. Of the carboxyalkyl dipeptides disclosed in U.S. Patent 4,374,829, those of particular interest in this invention are N-[1(S)-ethoxycarbonyl-3-phenylpropyl]-L-alanyl-L-proline, also known and referred to herein as enalapril; N-[1(S)-carboxy-3-phenylpropyl]-L-alanyl-L-proline, also know and referred to herein as enalapril diacid ; and, Nα-[1(S)-carboxy-3-phenylpropyl]-L-lysyl-L-proline, also known and referred to herein as lisinapril.

The combination composition of the invention can contain varying amounts of (i) the Formula I antithrombotic compound and (ii) ACE inhibitor antihypertensive compounds. The weight ratio of (i) : (ii) can range from about 25 to 1 ; preferably from about 10 to 1. In addition to the active ingredients of (i) alone or of (i) and (ii) in combination, the compositions of the invention can also contain other conventional pharmaceutically acceptable compounding ingredients, as necessary or desired. Such ingredients are generally referred to as carriers or diluents. Conventional procedures for preparing such compositions in appropriate dosage forms can be utilized. Whatever the dosage form, it will contain a pharmaceutically effective amount of the present composition.

The combination compositions can be administered orally or other than orally ; e.g., parenterally, by insufflation, topically, rectally, etc. ; using appropriate dosage forms ; e.g., tablets, capsules, suspensions, solutions, and the like, for oral administration ; suspension emulsions, and the like, for parenteral administration ; solutions for intravenous administration ; and ointments, transdermal patches, and the like, for topical administration. These compositions are formulated similarly to the compositions discussed above.

Treatment dosage for human beings for cardiovascular use can be varied as necessary. Generally, daily dosages of the composition of the invention can range from about 6000 to about 10 mg ; preferably, from about 3000 to about 20 mg.

The amount of active ingredient that may be combined with the carrier materials to produce a single dosage form for cardiovascular use will vary depending upon the host treated and the particular mode of administration. For example, a formulation intended for oral administration may contain from 5 mg to 5 gm of active agents compounded with an appropriate and convenient amount of carrier material which may vary from about 5 to about 95 percent of the total composition. Dosage unit forms will generally contain between from about 20 mg to about 500 mg of active ingredients.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The composition of this invention inhibits platelet accumulation at the damaged endothelial surface via the Formula I compound. This inhibitory effect is potentiated by the presence of the antihypertensive compound.

Thus, the compositions of the invention are useful in treating thrombosis and are also of value in the management of acute and chronic congestive heart failure, and limitation of myocardial infarct damage.

In vivo testing of the composition of this invention in test animals (rabbits) can be used to demonstrate that this composition is pharmaceutically effective in decreasing platelet-related arterial thrombic formation.

To demonstrate the potentiation of the antihypertensive compound on the anti-thrombotic Formula I compound comprising the combination composition of the invention, the effect of these compounds on test animals (rabbits) can be determined separately and then in combination. The effect of a different class of antihypertensive agents singly and in combination with the Formula I compound of the invention can also be determined for comparative purposes. The methods employed are described in U.S. Patent 4,558,037 which is hereby incorporated herein by reference.

The following examples illustrate the preparation of the compounds of the present invention without, however, limiting the same thereto.

All temperatures are in degrees Celsius.

Reference Compounds

6-fluoro-1,2,3,4-tetrahydrocarbazol-1-yl-acetic acid

Following the procedure of Example 1, but using 1-(4-fluorophenyl) hydrazine hydrochloride and ethyl-2-cyclohexanone acetate as starting materials, the title compound was prepared.

M.P. 124-126°C

Reference Compounds

9-[1-(2-p-chlorophenyl)ethyl]-6-fluoro-1,2,3,4-tetrahydrocarbazol-1-yl-acetic acid

Following the procedure of Example 2, but using 1-[2-(4-chlorophenyl)ethyl]-1-(4-fluorophenyl) hydrazine hydrochloride and ethyl 2-cyclohexanone acetate as starting materials, the title compound was prepared.

Empirical Formula: $C_{22}H_{21}NFClO_2$

| | C | H | N |
|---|---|---|---|
| Calculated | 68.48 | 5.49 | 3.63 |
| Found | 68.15 | 5.70 | 3.65 |

Reference Compounds

6-fluoro-9-isopropyl-1,2,3,4-tetrahydrocarbazol-1-ylacetic acid

Following the procedure of Example 1, but using 1-(2-propyl)-1-(4-fluorophenyl)hydrazine hydrochloride and ethyl 2-cyclohexanone acetate as starting materials, the title compound was prepared

Empirical Formula : $C_{17}H_{20}NFO_2$ M.P. 144-144.5°C

Example 1

9-p-chlorobenzyl-6-fluoro-1,2,3,4-tetrahydrocarbazol1-yl-acetic acid

Step I

To 3.50 g of 1-(4-chlorobenzyl)-1-(4-fluorophenyl)hydrazine hydrochloride in 70 cc of isopropanol was added 2.23 g of ethyl 2-cyclohexanone acetate. The reaction was refluxed under nitrogen for 16 hours. The resulting reaction mixture was then evaporated to dryness and the residue suspended in ether. The solid material was then filtered. The ether filtrate was washed with water, dried and evaporated. The resulting syrup was chromatographed on silica gel to give 2.8 g (42%).

Step II

To 1.59 g of ethyl ester from step I in 10 cc of methanol was added 10 cc of water and 420 mg of potassium hydroxide. The resulting solution was refluxed for 4 hours. Upon cooling the reaction mixture was then acidified with HCl (IN). The resulting precipitate was filtered and washed with water. Analytically pure material was prepared by trituration the solid with a mixture of hexane/ethyl acetate (9 : 1) followed by filtration and drying on a high vacuum pump to give 1.24 g of the title compound (89%).

Analysis calculated for $C_{21}H_{19}NClFO_2$

| | C | H | N | Cl | F |
|---|---|---|---|---|---|
| Calculated | 67.83 | 5.15 | 3.77 | 9.53 | 5.11 |
| Found | 67.88 | 5.47 | 3.63 | 9.52 | 5.12 |

Example 2

3-[9-p-chlorobenzyl-6-fluoro-1,2,3,4-tetrahydrocarbazol-1-yl]-propanoic acid

Following the procedure of Example 1, but using 1-(4-chlorobenzyl)-1-(4-fluorophenyl)hydrazine hydrochloride and methyl-2-cyclohexanone propionate as starting materials, the title compound was prepared.

Empirical formula: $C_{22}H_{21}ClFNO_2$

| | C | H | N |
|---|---|---|---|
| Calculated | 68.48 | 5.49 | 3.63 |
| Found | 68.26 | 5.57 | 3.60 |

Example 3

3-[9-p-chlorobenzyl-6-methoxy-1,2,3,4-tetrahydrocarbazol-1-yl]-propanoic acid

Following the procedure of Example 1, but using 1-(4-chlorobenzyl)-1-(4-methoxyphenyl)hydrazine hydrochloride and methyl-2-cyclohexanone propionate as starting materials, the title compound was prepared.

Empirical formula: $C_{22}H_{22}ClNO_3$

| | C | H | N | Cl |
|---|---|---|---|---|
| Calculated | 69.43 | 6.08 | 3.52 | 8.91 |
| Found | 69.24 | 5.98 | 3.60 | 8.85 |

Example 4

9-p-chlorobenzyl-6-methoxy-1,2,3,4-tetrahydrocarbazol1-yl-acetic acid

Following the procedure of Example 1, but using 1-(4-chlorobenzyl)-1-(4-methoxyphenyl)hydrazine hydrochloride and ethyl-2-cyclohexanone acetate as starting materials, the title compound was prepared.

M.P. 152-153°C

Example 5

2-[9-p-chlorobenzyl-6-fluoro-1,2,3,4-tetrahydrocarbazol-1-yl]ethanol

1.10 g of 9-p-chlorobenzyl-6-fluoro-1,2,3,4-tetrahydrocarbazo-1-yl-acetic acid ethyl ester was dissolved in 500 ml dry tetrahydrofuran and the reaction was cooled at 0°C and one equivalent of lithium aluminium hydride (LAH) was added portion wise. The reaction was allowed to warm up to room temperature and stirred for 16 hrs. The reaction mixture was quenched with $NH_4C1$ (aq.). Ethyl acetate was added (100 ml) and the organic phase separated, washed with water and brine, dried and evaporated. The product was isolated by column chromatography.

M.P. 98.0-98.5°C.

Example 6

3-[9-p-chlorobenzyl-6-methoxy-1,2,3,4-tetrahydrocarbazol-1-yl]-propanol

Following the procedure of Example 5, but using 3-[9-p-chlorobenzyl-6-methoxy-1,2,3,4-tetrahydrocarbazo-1-yl]-propanoic acid methyl ester from Example 3 as starting material, the title compound was prepared.

Empirical formula: $C_{23}H_{26}ClNO_2$

| | C | H | N |
|---|---|---|---|
| Calculated | 71.95 | 6.83 | 3.65 |
| Found | 71.86 | 6.65 | 3.81 |

Example 7

(–)9-p-chlorobenzyl-6-fluoro-1,2,3,4-tetrahydrocarbazol-1-yl-acetic acid

Step I

10.0 g of 9-p-chlorobenzyl-6-fluoro-1,2,3,4tetrahydrocarbazo-1-yl-acetic acid from Example I was dissolved in a mixture of hot (refluxing) acetonitrile (150 cc), and ethanol (25 cc) and 4.4 g of d(+) ephedrine was added. The reflux was continued for 15 min. and the hot solution was filtered and allowed to cool to room temperature. Crystals separated from the solution and were separated by filtration. After three recrystallizations from acetonitrile 3.9 g of the pure salt was obtained.

Step II

3.9 g of pure salt from step I was dissolved in 200 cc of methanol and acidified using 1 N hydrochloric acid. Water was added and the crystals were separated by filtration and dryed under vacuum. Upon trituration with hexane ethyl acetate mixture (9 : 1) the title compound was prepared.

αD = –42.5 (methanol) M.P. 151-151.5°C.

Example 8

(+)9-p-chlorobenzyl-6-fluoro-1,2,3,4-tetrahydrocarbazol-1-yl-acetic acid

Following the method of Example 7, but using 1(–) ephedrine and 9-p-chlorobenzyl-6-fluoro-1,2,3,4tetrahydrocarbazo-1-yl-acetic acid as starting material, the title compound was prepared.

αD = +43.0 (methanol) M.P. 150-150.5°C

Example 9 9-benzyl-6-fluoro-1,2,3,4-tetrahydrocarbazol-1-ylacetic acid

Following the procedure of Example 1, but using l-(benzyl)-1- (4-fluorophenyl)hydrazine hydrochloride and ethyl 2-cyclohexanone acetate as starting materials, the title compound was prepared.

Analysis calculated for $C_{21}H_{20}NFO_2$

| | C | H | N |
|---|---|---|---|
| Calculated | 74.76 | 5.98 | 4.15 |
| Found | 74.95 | 6.07 | 3.90 |

Example 10

9-p-methoxybenzyl-6-fluoro-1,2,3,4-tetrahydrocarbazol1-yl-acetic acid

Following the procedure of Example 1, but using 1-(4-methoxybenzyl)-1-(4-fluorophenyl)hydrazine hydrochloride and ethyl 2-cyclohexanone acetate as stating materials, the title compound was prepared.

Analysis calculated for $C_{22}H_{22}NFO_3$

| | C | H | N |
|---|---|---|---|
| Calculated | 71.92 | 6.04 | 3.91 |
| Found | 71.70 | 6.22 | 4.05 |

Example 11

9-(3,4-dichloro)benzyl-6-fluoro-1,2,3,4-tetrahydrorbazol-1-yl-acetic acid

Following the procedure of Example 1, but using 1-(3,4-dichlorobenzyl)-1-(4-fluorophenyl) hydrazine hydrochloride and ethyl 2-cyclohexanone acetate as starting materials, the title compound was prepared.

Analysis calculated for $C_{21}H_{18}NCl_2FO_2$.

| | C | H | N |
|---|---|---|---|
| Calculated | 62.08 | 4.47 | 3.45 |
| Found | 61.96 | 4.71 | 3.67 |

Example 12

9-[1-(1-phenyl)ethyl]-6-fluoro-1,2,3,4-tetrahydrocarbazol-1-yl-acetic acid

Following the procedure of Example 1, but using 1-[1-(1-phenyl)ethyl]-1-(4-fluorophenyl)hydrazine hydrochloride and ethyl 2-cyclohexanone acetate as starting materials, the title compound was prepared.

| | C | H | N | F |
|---|---|---|---|---|
| Calculated | 75.19 | 6.31 | 3.99 | 5.41 |
| Found | 75.18 | 6.05 | 4.11 | 5.51 |

Example 13

9-p-chlorobenzyl-1,2,3,4-tetrahydrocarbazol-1-yl-acetic acid

Following the procedure of Example 1, but using 1-(4-chlorobenzyl)-1-(phenyl)hydrazine hydrochloride and ethyl 2-cyclohexanone acetate as starting materials, the title compound was prepared.

Empirical Formula: $C_{21}H_{20}NClO_2$

| | C | H | N | Cl |
|---|---|---|---|---|
| Calculated | 71.28 | 5.70 | 3.96 | 10.02 |
| Found | 70.7n | 5.90 | 3.82 | 10.23 |

Example 14

9-p-chlorobenzyl-6-chloro-1,2,3,4-tetrahydrocarbazol1-yl-acetic acid

Following the procedure of Example 1, but using 1-(4-chlorobenzyl)-1-(4-chlorophenyl)hydrazine hydrochloride and ethyl-2-cyclohexanone acetate as starting materials, the title compound was prepared.

Empirical Formula: $C_{21}H_{19}NCl_2O_2$

| | C | H | N | Cl |
|---|---|---|---|---|
| Calculated | 69.96 | 4.93 | 3.61 | 18.26 |
| Found | 65.20 | 5.16 | 3.38 | 18.04 |

Example 15

9-p-chlorobenzyl-8-methyl-1,2,3,4-tetrahydrocarbazol1-yl-acetic acid

Following the procedure of Example 1, but using 1-(4-chlorobenzyl)-1-(2-methylphenyl)hydrazine hydrochloride and ethyl-2-cyclohexanone acetate as starting materials, the title compound was prepared.

Empirical Formula: $C_{22}H_{22}NClO_2$

| | C | H | N | Cl |
|---|---|---|---|---|
| Calculated | 71.83 | 6.03 | 3.81 | 9.64 |
| Found | 72.19 | 6.23 | 4.12 | 9.84 |

Example 16

6-bromo-9-p-chlorobenzyl-1,2,3,4-tetrahydrocarbazol-1- yl-acetic acid

Following the procedure of Example 1, but using 1-(4-chlorobenzyl)-1-(4-bromophenyl)hydrazine hydrochloride and ethyl 2-cyclohexanone acetate as starting materials, the title compound was prepared.

Empirical formula: $C_{21}H_{19}BrClNO_2$

| | C | H | N | Br | Cl |
|---|---|---|---|---|---|
| Calculated | 58.29 | 4.43 | 3.24 | 18.46 | 8.19 |
| Found | 58.49 | 4.60 | 3.44 | 18.50 | 8.27 |

Example 17

9-p-chlorobenzyl-6-methyl-1,2,3,4-tetrahydrocarbazol- 1-yl-acetic acid

Following the procedure of Example 1, but using 1-(4-chlorobenzyl)-1-(4-methylphenyl)hydrazine hydrochloride and ethyl 2-cyclohexanone acetate as starting materials, the title compound was prepared.

Empirical formula: $C_{22}H_{22}ClNO_2$

| | C | H | N | Cl |
|---|---|---|---|---|
| Calculated | 71.83 | 6.03 | 3.81 | 9.64 |
| Found | 71.72 | 6.14 | 3.77 | 9.88 |

Example 18

2-[9-p-chlorobenzyl-6-fluoro-1,2,3,4-tetrahydrocarbazol-1-yl-]propanoic acid

Following the procedure of Example 1, but using 1-(4-chlorobenzyl)-1-(4-fluorophenyl) hydrazine hydrochloride and methyl 2-(2-cyclohexanone) propionate as starting materials, the title compound was prepared.

M.P. 203-205°C.

Example 19

9-p-chlorobenzyl-8 fluoro-1,2,3,4-tetrahydrocarbazol1-yl-acetic acid

Following the procedure of Example 1, but using 1-(4-chlorobenzyl)-1-(2-fluorophenyl) hydrozine hydrochloride and ethyl 2-cyclohexanone acetate as starting materials, the title compound was prepared.

M.P. 228-229°C.

Example 20

9-p-chlorobenzyl-5-fluoro-1,2,3,4-tetrahydrocarbazol1-yl-acetic acid and
9-p-chlorobenzyl-7-fluoro-1,2,3,4-tetrahydocarbazol-1-yl-acetic acid (mixture)

Following the procedure of Example 1, but using 1-(4-chlorobenzyl)-1-(3-fluorophenyl)hydrazine hydrocloride and ethyl 2-cyclohexanone acetate as starting materials, the title compounds were prepared.

Empirical Formula : $C_{21}H_{19}NC1FO_2$

M.P. 211-219°C.

Example 21

9-p-chlorobenzyl-5,7-dichloro-1,2,3,4-tetrahydrocarbazol-1-yl-acetic acid

Following the procedure of Example 1, but using 1-(4-chlorobenzyl)-1-(3-5 dichlorophenyl)hydrozine hydrochloride and ethyl 2-cyclohexanone acetate as starting materials, the title compound was prepared.

Empirical formula : $C_{21}H_{18}NCl_3O_2$
M.P. 204-206°C.

Example 22

9-p-chlorobenzyl-6,8-dichloro-1,2,3,4-tetrahydrocarbazol-1-yl-acetic acid

Following the procedure of Example 1, but using 1-(4-chlorobenzyl)-1-(2-4-dichlorophenyl)hydrozine hydrochloride and ethyl 2-cyclohexanone acetate as starting materials, the title compound was prepared.
Empirical formula : $C_{21}H_{18}NCl_3O_2$
M.P. 203-204°C.

Example 23

9-p-Chlorobenzyl-6-trifluoromethyl-1,2,3,4-tetrahydrocarbazol-1-yl-acetic acid

Following the procedure of Example 1, but using 1-(4-chlorobenzyl)-1-(4-trifluoromethylphenyl) hydrazine hydrochloride and ethyl 2-cyclohexanone acetate as starting materials, the title compound was prepared. m.p. 167-168°C.

Example 24

9-p-Chlorobenzyl-6-methylthio-1,2,3,4-tetrahydrocarbazol-1-yl-acetic acid

Following the procedure of Example 1, but using 1-(4-chlorobenzyl)-1-(4-methylthiophenyl) hydrazine hydrochloride and ethyl 2-cyclohexanone acetate as starting materials, the title compound was prepared.

Empirical Formula: $C_{22}H_{22}ClNO_2S$

| | C | H | N | S | Cl |
|---|---|---|---|---|---|
| Calc: | 66.07 | 5.54 | 3.50 | 8.02 | 8.86 |
| Found: | 66.27 | 5.83 | 3.38 | 8.00 | 8.70 |

Example 25

9-p-Chlorobenzyl-6-methylsulfinyl-1,2,3,4-tetrahydrocarbazol-1-yl-acetic acid

Step I

To 498 mg of ethyl 9-p-chlorobenzyl-6-methylthio-1,2,3,4-tetrahydrocarbazol-1-yl-acetate from Example 24, Step I, in 10 cc of methylene chloride was added 300 mg of m-chloro perbenzoic acid. The resulting mixture was stirred for 1.5 hours at room temperature. The reaction mixture was diluted with ether and washed consecutively with a solution of sodium bicarbonate, water and brine. The crude product obtained after evaporation of the organic layer was purified on silica gel by flash chromatography eluting with 20% hexane/ethyl acetate and yielded 420 mg (82%) of the puresulfoxide derivative.

Step II

Following the procedure of Example 1, Step II, but using the ethyl ester from Step I, there was obtained the title compound. m.p. 105-107°C.

Empirical Formula: $C_{22}H_{22}ClNO_3S$

| | C | H | N | S | Cl |
|---|---|---|---|---|---|
| Calc: | 63.53 | 5.33 | 3.37 | 7.71 | 8.52 |
| Found: | 63.31 | 5.03 | 2.94 | 7.69 | 8.48 |

Example 26

9-p-Chlorobenzyl-6-methylsulfonyl-1,2,3,4-tetrahydrocarbazol-1-yl-acetic acid

Step I

To 439 mg of ethyl 9-p-chlorobenzyl-6-methylsulfinyl-1,2,3,4-tetrahydrocarbazol-1-yl-acetate from Example 25, Step I, in 10 cc of methylene chloride was added 353 mg of m-chloro perbenioic acid. The resulting mixture was stirred for 18 hours at room temperature. The reaction mixture was diluted with ether and washed consecutively with a solution of sodium bicarbonate, water and brine. The crude product obtained after evaporation of the organic layer was purified on silica gel by flash chromatography eluting with 30% hexane/ethyl acetate and yielded 200 mg (42%) of the pure sulfone derivative.

Step II

Following the procedure of Example 1, Step II, but using the ethyl ester from Step 1, there was obtained the title compound. m.p. 101-102°C.

Example 27

9-p-Chlorobenzyl-8-methylthio-1,2,3,4-tetrahydrocarbazo1-1-yl-acetic acid

Following the procedure of Example 30, but using 1-(2-methylthiophenyl) hydrazine hydrochloride and ethyl 2-cyclohexanone acetate as starting materials, the title compound was prepared. m.p. 141-142°C.

Example 28

9-p-Chlorobenzyl-8-methylsulfinyl-1, 2, 3, 4-tetrahydrocarbazol-1-yl-acetic acid

Following the procedure of Example 25, but using the ethyl ester from Example 27, Step II, there was obtained the title compound. m.p. 119-120.5°C.

Example 29

9-p-Chlorobenzyl-6-fluoro-3-methyl-1,2,3,4-tetrahydrocarbazol-1-yl-acetic acid

Following the procedure of Example 1, but using 1-(4-chlorobenzyl)-1-(4-fluorophenyl) hydrazine hydrochloride and ethyl 4-methyl-2-cyclohexanone acetate as starting materials, the title compound was prepared. m.p. 205-206°C.

Empirical Formula: $C_{22}H_{21}FClNO_2$

| | C | H | N | Cl |
|---|---|---|---|---|
| Calc: | 68.48 | 5.49 | 3.63 | 9.19 |
| Found: | 68.80 | 5.50 | 3.30 | 9.47 |

Example 30

9-p-Chlorobenzyl-6,8-difluoro-1,2,3,4-tetrahydrocarbazol-1-yl-acetic acid

Step I

To 114 g of 1-(2,4-difluorophenyl) hydrazine hydrochloride in 350 cc of 2-propanol containing 40 cc of acetyl chloride was added 138 g of ethyl 2-cyclohexanone acetate. The reaction was refluxed under nitrogen for 2 days. After cooling, 200 cc of ether was added and the precipitate filtered. The filtrate was evaporated to dryness. The resulting residue was dissolved in a (1 : 1) mixture of ether/ethyl acetate and consecutively washed with water, sodium bicarbonate solution and brine. The organic layer was dried over sodium sulfate

and evaporated to dryness. The crude product was passed through a silica gel bed eluting with 5% ethyl acetate/hexane to yield 84 g of a 1 : 2 mixture of ethyl and isopropyl esters.

Step II

84 g of esters from Step I was dissolved in 250 cc of methanol and 400 cc of sodium hydroxide (IN) was added and refluxed 4 hours. After cooling, the reaction mixture was washed with a (1 : 1) mixture of ether/hexane and the aqueous layer was acidified with HCl (IN). The resulting precipate was filtered, washed with water and air dried to afford 50 g of 6,8-difluoro-1,2,3,4-tetrahydrocarbazol-1-yl-acetic acid.

Step III

A solution of 11 : 1 g of acid from Step 11 in 100 cc of THF was added portionwise 10.3 g of potassium tert-butoxide. The resulting mixture was stirred for 45 min. at room temperature and 10.3 g p-chlorobenzyl bromide was added portionwise. The reaction mixture was stirred 18 hours at room temperature. The resulting mixture was diluted with 100 cc of water and washed with hexane. The aqueous layer was acidified with HCl (IN) and the resulting precipitate filtered washed with water and air-dried to afford 9.4 g of the title compound. m.p. 168.5-170°C.

Example 31

9-p-Chlorobenzyl-6,8-dimethyl-1,2,3,4-tetrahydrocarbazol-1-yl-acetic acid

Following the procedure of Example 30, but using 1-(2,4-dimethylphenyl) hydrazine hydrochloride and ethyl 2-cyclohexanone acetate in Step I as starting materials, the title compound was prepared. m.p. 187-188°C.

Example 32

9-p-Chlorobenzyl-6-methoxy-8-methyl-1,2,3,4-tetrahydrocarbazol-1-yl-acetic acid

Following the procedure of Example 30, but using 1-(4-methoxy-2-methylphenyl) hydrazine hydrochloride and ethyl 2-cyclohexanone acetate as starting materials, the title compound was prepared. m.p. 188-188.5°C.

Example 33

(–)-9-p-Chlorobenzyl-6,8-difluoro-1,2,3,4-tetrahydrocarbazol-1-yl-acetic acid

Following the method of Example 7, but using d(+)ephedrine and 9-p-chlorobenzyl-6,8-difluoro-1,2,3,4-tetrahydrocarbazol-1-yl-acetic acid from Example 30 as starting materials, the title compound was prepared. From Example 34 $[\alpha]_D = -64.3°$ (methanol) m.p. 130-131°C.

Example 34

(+)-9-p-Chlorobenzyl-6,8-difluoro-1,2,3,4-tetrahydrocarbazol-1-yl-acetic acid

Following the method of Example 33, but using 1(–)ephedrine, the title compound was prepared. $[\alpha]_D = +61.5°$ (methanol) m.p. 129.5-130°C.

Example 35

(–)-9-p-Chlorobenzyl-8-methyl-1,2,3,4-tetrahydrocarbazol-1-yl-acetic acid

Following the method of Example 7, but using d(+)ephedrine and 9-p-chlorobenzyl-8-methyl-1,2,3,4-tetrahydrocarbazol-1-yl-acetic acid from Example 15 as starting materials, the title compound was prepared. $[\alpha]_D = -51.6°$ (methanol) m.p. 196-198°C.

Example 36

(+)-9-p-Chlorobenzyl-8-methyl-1,2,3,4-tetrahydrocarbazol-1-yl-acetic acid

Following the method of Example 35, but using 1(–)ephedrine, the title compound was prepared. $[\alpha]_D$ = +45.9° (methanol) m.p. 195-197°C.

Example 37

(–)-9-p-Chlorobenzyl-8-fluoro-1,2,3,4-tetrahydrocarbazol-1-yl-acetic acid

Following the method of Example 7, but using d(+)ephedrine and 9-p-chlorobenzyl-8-fluoro-1,2,3,4-tetrahydrocarbazol-1-yl-acetic acid from Example 19 as starting materials, the title compound was prepared. $[\alpha]_D$ = –62.1° (methanol) m.p. 74-75°C.

Example 38

(+)-9-p-Chlorobenzyl-8-fluoro-1,2,3,4-tetrahydrocarbazol-1-yl-acetic acid

Following the method of Example 37, but using 1(–)ephedrine, the title compound was prepared. $[\alpha]_D$ = +65.2° (methanol) m.p. 94-94.5°C.

Example 39

2-(9-p-Chlorobenzyl-6,8-difluoro-1,2,3,4-tetrahydrocarbazol-1-yl)ethanol

Following the procedure of Example 5, but using a mixture of g-p-chlorobenzyl-6,8-difluoro-1,2,3,4-carbazol-1-yl-acetic acid ethyl and isopropyl esters from Example 30 as starting materials, the title compound is obtained.

Example 40

(–) or (+) 2-(g-p-Chlorobenzyl-6,8-difluoro-1,2,3,4tetrahydrocarbazol-1-yl)ethanol

Following the procedure of Example 5, but using (–) or (+) 9-p-chlorobenzyl-6,8-difluoro-1,2,3,4-tetrahydrocarbazol-1-yl-acetic acid from Example 33 or 34 as starting material, the title compounds are obtained.

## Claims

**Claims for the contracting states BE, FR, DE, GB, NL, IT, LU, SE, CH**

1. A compound of the formula :

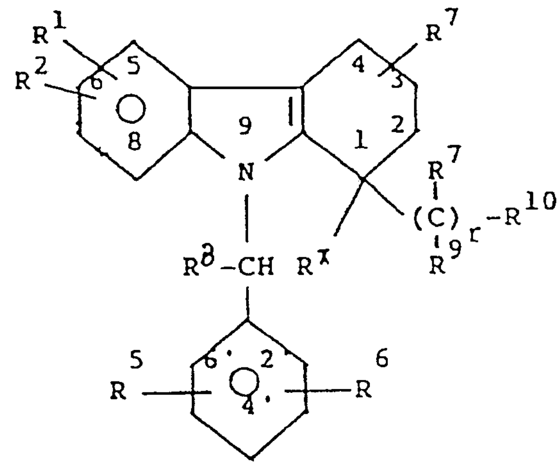

wherein :

$R^1$, $R^2$, $R^5$ and $R^6$ are each independently selected from :

(1) hydrogen ;

(2) linear alkyl having 1 to 6 carbon atoms ;

(3) M

wherein M is

a) $OR^{13}$ ;

b) halogen ;

c) $CF_3$ ;

d) $SR^{13}$ ;

e) phenyl ;

f) $COOR^{14}$ ;

g) 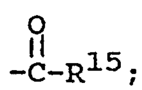

h) tetrazole ;

i) 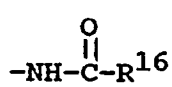 wherein $R^{16}$ is linear $C_1$ to $C_6$ alkyl;

j) $-NR^{14}R^{14}$ ;

k) $-NHSO_2R^{17}$ wherein $R^{17}$ is linear $C_1$ to $C_6$ alkyl, or $CF_3$ ;

l) $-SOR^{13}$ ;

m) $-CONR^{14}R^{14}$ ;

n) $-SO_2NR^{14}R^{14}$ ;

o) $-SO_2R^{13}$ ;

p) $NO_2$ ;

q) CN ;

r) $N_3$ ;

$R^7$ is H or linear alkyl of 1 to 6 carbons ;

$R^8$ is H or linear alkyl of 1 to 6 carbon atoms ;

each $R^9$ is independently H, OH, linear $C_1$ to $C_4$-O-alkyl or linear alkyl of 1 to 4 carbons ;

$R^{10}$ is COOH ; $CH_2OH$ ; CHO ; tetrazole ; $CONHSO_2R^{11}$ ; CN ; $CON(R^9)_2$ ; or $NHSO_2R^{11}$ wherein $R^{11}$ is OH, linear alkyl or linear alkoxy of 1 to 6 carbons, linear perhaloalkyl of 1 to 6 carbons, phenyl or phenyl substituted by linear alkyl or linear alkoxy groups of 1 to 3 carbons, halogen, hydroxy, COOH, CN, formyl

or acyl to 1 to 6 carbons ;
r is 1 to 6 ;
each $R^{13}$ independently is H ; or linear $C_1$ to $C_6$ alkyl ;
each $R^{14}$ is independently H, or linear $C_1$ to $C_6$ alkyl ; and,
each $R^{15}$ independently is H, linear $C_1$ to $C_6$ alkyl, or $CF_3$ ; or a pharmaceutically acceptable salt thereof.

2. A compound according to Claim 1 which is : 2-[9-p-chlorobenzyl-6-fluoro-1,2,3,4-tetrahydrocarbazol1-yl]-1-ethanol ;
2-(9-p-chlorobenzyl-6,8-difluoro-1,2,3,4-tetrahydrocarbazol-1-yl)ethanol ;
(–) or (+) 2-(9-p-chlorobenzyl-6,8-difluoro-1,2,3,4tetrahydrocarbazol-1-yl)ethanol ;
3-[9-p-chlorobenzyl-6-methoxy-1,2,3,4-tetrahydrocarbazol1-yl]-propanol ;
9-benzyl-6-fluoro-1,2,3,4-tetrahydrocarbazol-1-yl-acetic acid ;
9-p-chlorobenzyl-1,2,3,4-tetrahydrocarbazol-1-yl-acetic acid ;
9-p-chlorobenzyl-5-fluoro-1,2,3,4-tetrahydrocarbazol-1-yl-acetic acid ;
6-bromo-9-p-chlorobenzyl-1,2,3,4-tetrahydrocarbazol-1-yl-acetic acid ;
9-p-chlorobenzyl-6-chloro-1,2,3,4-tetrahydrocarbazol-1-yl-acetic acid ;
9-p-chlorobenzyl-6-fluoro-1,2,3,4-tetrahydrocarbazol-1-yl-acetic acid ;
(–)9-p-chlorobenzyl-6-fluoro-1,2,3,4-tetrahydrocarbazol-1-yl-acetic acid ;
(+)9-p-chlorobenzyl-6-fluoro-1,2,3,4-tetrahydrocarbazol-1-yl-acetic acid ;
9-p-chlorobenzyl-6-methoxy-1,2,3,4-tetrahydrocarbazol-1-yl-acetic acid ;
9-p-chlorobenzyl-6-methyl-1,2,3,4-tetrahydrocarbazol-1-yl-acetic acid ;
9-p-chlorobenzyl-6-methylthio-1,2,3,4-tetrahydrocarbazol-1-yl-acetic acid ;
9-p-chlorobenzyl-6-methylsulfinyl-1,2,3,4-tetrahydrocarbazol-1-yl-acetic acid ;
9-p-chlorobenzyl-6-methylsulfonyl-1,2,3,4-tetrahydrocarbazol-1-yl-acetic acid ;
9-p-chlorobenzyl-6-trifluoromethyl-1,2,3,4-tetrahydrocarbazol-1-yl-acetic acid ;
9-p-chlorobenzyl-7-fluoro-1,2,3,4-tetrahydrocarbazol-1-yl-acetic acid ;
9-p-chlorobenzyl-8-fluoro-1,2,3,4-tetrahydrocarbazol-1-yl-acetic acid ;
(–)-9-p-chlorobenzyl-8-fluoro-1,2,3,4-tetrahydrocarbazol-1-yl-acetic acid ;
(+)-9-p-chlorobenzyl-8-fluoro-1,2,3,4-tetrahydrocarbazol-1-yl-acetic acid ;
9-p-chlorobenzyl-8-methyl-1,2,3,4-tetrahydrocarbazol-1-yl-acetic acid ;
(–)-9-p-chlorobenzyl-8-methyl-1,2,3,4-tetrahydrocarbazol-1-yl-acetic acid ;
(+)-9-p-chlorobenzyl-8-methyl-1,2,3,4-tetrahydrocarbazol-1-yl-acetic acid ;
9-p-chlorobenzyl-8-methylthio-1,2,3,4-tetrahydrocarbazol-1-yl-acetic acid ;
9-p-chlorobenzyl-8-methylsulfinyl-1,2,3,4-tetrahydrocarbazol-1-yl-acetic acid ;
9-p-chlorobenzyl-5,7-dichloro-1,2,3,4-tetrahydrocarbazol-1-yl-acetic acid ;
9-p-chlorobenzyl-6,8-dichloro-1,2,3,4-tetrahydrocarbazol-1-yl-acetic acid ;
9-p-chlorobenzyl-6,8-difluoro-1,2,3,4-tetrahydrocarbazol-1-yl-acetic acid ;
(–)-9-p-chlorobenzyl-6,8-difluoro-1,2,3,4-tetrahydrocarbazol-1-yl-acetic acid ;
(+)-9-p-chlorobenzyl-6,8-difluoro-1,2,3,4-tetrahydrocarbazol-1-yl-acetic acid ;
9-p-chlorobenzyl-6,8-dimethyl-1,2,3,4-tetrahydrocarbazol-1-yl-acetic acid ;
9-p-chlorobenzyl-6-fluoro-3-methyl-1,2,3,4-tetrahydrocarbazol-1-yl-acetic acid ;
9-p-chlorobenzyl-6-methoxy-8-methyl-1,2,3,4-tetrahydrocarbazol-1-yl-acetic acid ;
9-p-methoxybenzyl-6-fluoro-1,2,3,4-tetrahydrocarbazol-1-yl-acetic acid ;
9-(3,4-dichloro)benzyl-6-fluoro-1,2,3,4-tetrahydrocarbazol-1-yl-acetic acid ;
9-[1-(1-phenyl)ethyl]-6-fluoro-1,2,3,4-tetrahydrocarbazol-1-yl-acetic acid ;
2-[9-p-chlorobenzyl-6-fluoro-1,2,3,4-tetrahydrocarbazol-1-yl]-propanoic acid ;
3-[9-p-chlorobenzyl-6-fluoro-1,2,3,4-tetrahydrocarbazol-1-yl]-propanoic acid ;
3-[9-p-chlorobenzyl-6-methoxy-1,2,3,4-tetrahydrocarbazol-1-yl]-propanoic acid ;

3. A compound according to Claim I, which is :
9-p-chlorobenzyl-6-fluoro-1,2,3,4-tetrahydrocarbazol-1-yl-acetic acid ;
9-p-chlorobenzyl-8-methyl-1,2,3,4-tetrahydrocarbazol-1-yl-acetic acid ;
9-p-chlorobenzyl-8 fluoro-1,2,3,4-tetrahydrocarbazol-1-yl-acetic acid ;
9-p-chlorobenzyl-6,8-difluoro-1,2,3,4-tetrahydrocarbazol-1-yl-acetic acid.

4. A compound according to Claim 1, which is :
(–)9-p-chlorobenzyl-6-fluoro-1,2,3,4-tetrahydrocarbazol-1-yl-acetic acid ;
(–)-9-p-Chlorobenzyl-6,8-difluoro-1,2,3,4-tetrahydrocarbazol-1-yl-acetic acid ;
(–)-9-p-Chlorobenzyl-8-methyl-1,2,3,4-tetrahydrocarbazol-1-yl-acetic acid ;
(–)-9-p-Chlorobenzyl-8-fluoro-1,2,3,4-tetrahydrocarbazol-1-yl-acetic acid.

5. A compound of the formula

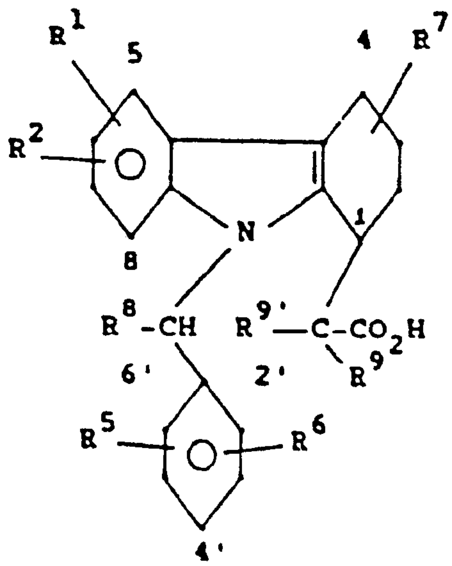

wherein:

| $R^1$ | $R^2$ | $R^5$ | $R^6$ | $R^9, R^{9'}$ | $R^7$ | $R^8$ |
|---|---|---|---|---|---|---|
| 6-F | H | H | H | H, H | H | H |
| 6-F | H | H | H | H, H | H | Me |
| 6-F | 8-F | 4'-n-$C_3H_7$ | H | H, H | H | H |
| 6-F | H | 4'-F | H | H, H | H | H |
| H | H | 4'-Cl | H | H, H | H | H |
| 5-F | H | 4'-Cl | H | H, H | H | H |
| 6-Me | H | 4'-Cl | H | H, H | H | H |
| 6-F | H | 4'-Cl | H | H, H | H | H |
| 6-F (-) isomer | H | 4'-Cl | H | H, H | H | H |
| 6-F (+) isomer | H | 4'-Cl | H | H, H | H | H |
| 6-Cl | H | 4'-Cl | H | H, H | H | H |
| 6-Br | H | 4'-Cl | H | H, H | H | H |

| $R^1$ | $R^2$ | $R^5$ | $R^6$ | $R^9,R^{9'}$ | $R^7$ | $R^8$ |
|---|---|---|---|---|---|---|
| 6-$CF_3$ | H | 4'-Cl | H | H, H | H | H |
| 6-OMe | H | 4'-Cl | H | H, H | H | H |
| 6-SMe | H | 4'-Cl | H | H, H | H | H |
| 6-S(O)Me | H | 4'-Cl | H | H, H | H | H |
| 6-$S(O)_2Me$ | H | 4'-Cl | H | H, H | H | H |
| 6-$N_3$ | H | 4'-Cl | H | H, H | H | H |
| 7-F | H | 4'-Cl | H | H, H | H | H |
| 8-Me | H | 4'-Cl | H | H, H | H | H |
| 8-Me(-)isomer | H | 4'-Cl | H | H, H | H | H |
| 8-Me(+)isomer | H | 4'-Cl | H | H, H | H | H |
| 8-F | H | 4'-Cl | H | H, H | H | H |
| 8-F(-)isomer | H | 4'-Cl | H | H, H | H | H |
| 8-F(+)isomer | H | 4'-Cl | H | H, H | H | H |
| 8-Br | H | 4'-Cl | H | H, H | H | H |
| 8-SMe | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| 8-S(O)Me | H | 4'-Cl | H | H, H | H | H |
| 6-Me | 8-Me | 4'-Cl | H | H, H | H | H |
| 6-F | 8-F | 4'-Cl | H | H, H | H | H |
| 6-F(-)isomer | 8-F | 4'-Cl | H | H, H | H | H |
| 6-F(+)isomer | 8-F | 4'-Cl | H | H, H | H | H |
| 5-Cl | 7-Cl | 4'-Cl | H | H, H | H | H |
| 6-Cl | 8-Cl | 4'-Cl | H | H, H | H | H |
| 6-F | 8-Me | 4'-Cl | H | H, H | H | H |
| 6-OMe | 8-Me | 4'-Cl | H | H, H | H | H |
| 6-F | H | 4'-Cl | H | H, H | 1-Me | H |
| 6-F | H | 4'-Cl | H | H, H | 3-Me | H |
| 6-F | H | 4'-Cl | H | Me, H | H | H |
| 6-F | H | 4'-Cl | H | Me, Me | H | H |
| 6-F | H | 4'-Br | H | H, H | H | H |
| 6-F | 8-F | 4'-I | H | H, H | H | H |

| Compound | $R^1$ | $R^2$ | $R^5$ | $R^6$ | $R^9, R^{9'}$ | $R^7$ | $R^8$ |
|---|---|---|---|---|---|---|---|
| 45 | 6-F | 8-F | 4'-OMe | H | H,H | H | H |
| 46 | 6-F | 8-F | 4'-OH | H | H,H | H | H |
| 47 | 6-F | 8-F | 4'-SMe | H | H,H | H | H |
| 48 | 6-F | H | 4'-S(O)Me | H | H,H | H | H |
| 49 | 6-F | 8-F | 4'-NHCOMe | H | H,H | H | H |
| 50 | 6-F | H | 4'-$S(O)_2$Me | H | H,H | H | H |
| 51 | 6-F | H | 4'-F | H | H,H | H | H |
| 52 | 6-F | H | 4'-Br | H | H,H | H | H |
| 53 | 6-F | 8-Me | 4'-Cl | H | H,H | H | H |
| 54 | 6-F | H | 4'-$CO_2$H | H | H,H | H | H |
| 55 | 6-F | H | 4'-$CO_2$Me | H | H,H | H | H |
| 56 | 6-F | 8-F | 4'-n-$C_3H_7$ | H | H,H | H | H |
| 57 | 6-F | 8-F | 3'-I | 4'-OH | H,H | H | H |
| 58 | 6-F | 8-F | 4'-I | H | H,H | H | H |
| 59 | 6-$N_3$ | H | 4'-Cl | H | H,H | H | H |
| 60 | 6-F | H | 4'-$N_3$ | H | H,H | H | H |

6. The use of a compound as claimed in claim 1 for the manufacture of a medicament for inhibiting leukotriene synthesis in a mammal.

7. The use of a compound as claimed in claim 1 for the manufacture of a medicament for antagonizing prostaglandins in mammals.

8. A pharmaceutical composition which comprises a compound of Claim 1 and a pharmaceutically

acceptable carrier.

9. A pharmaceutical composition which comprises a compound of Claim 1 and an effective amount of a second active ingredient selected from the group consisting of non-steroidal anti-inflammatory drugs ; peripheral analgesic agents ; cyclooxygenase inhibitors ; leukotriene antagonists ; leukotriene biosynthesis inhibitors ; $H_1$-receptor antagonists ; $H_2$ receptor antagonists ; prostaglandin antagonists ; ACE inhibitors, or thromboxane synthetase inhibitors.

10. A pharmaceutical composition according to Claim 9, wherein the second active ingredient is indomethacin.

## Claims for the contracting states AT, GR, ES

1. A process for the preparation of a compound of the formula :

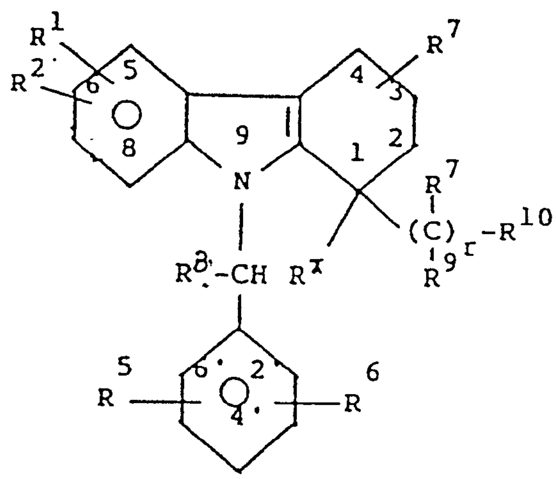

wherein :

$R^1$, $R^2$, $R^5$ and $R^6$ are each independently selected from :

(1) hydrogen ;

(2) linear alkyl having 1 to 6 carbon atoms ;

(3) M

wherein M is

a) $OR^{13}$ ;

b) halogen ;

c) $CF_3$ ;

d) $SR^{13}$ ;

e) phenyl ;

f) $COOR^{14}$ ;

g) 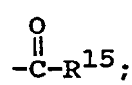

h) tetrazole ;

i) 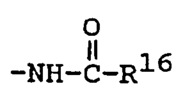 wherein $R^{16}$ is linear $C_1$ to $C_6$ alkyl;

j) $-NR^{14}R^{14}$ ;

k) $-NHSO_2R^{17}$ wherein $R^{17}$ is linear $C_1$ to $C_6$ alkyl, or CF3 ;

l) $-SOR^{13}$ ;
m) $-CONR^{14}R^{14}$ ;
n) $-SO_2NR^{14}R^{14}$ ;
o) $-SO_2R^{13}$ ;
p) $NO_2$ ;
q) CN ;
r) $N_3$ ;
$R^7$ is H or linear alkyl of 1 to 6 carbons ;
$R^8$ is H or linear alkyl of 1 to 6 carbon atoms ;
each $R^9$ is independently H, OH, linear $C_1$ to $C_4$-O-alkyl or linear alkyl of 1 to 4 carbons ;
$R^{10}$ is COOH ; $CH_2OH$ ; CHO ; tetrazole ; $CONHSO_2R^{11}$ ; CN ; $CON(R^9)_2$ ; or $NHSO_2R^{11}$ wherein $R^{11}$ is OH, linear alkyl or linear alkoxy of 1 to 6 carbons, linear perhaloalkyl of 1 to 6 carbons, phenyl or phenyl substituted by linear alkyl or linear alkoxy groups of 1 to 3 carbons, halogen, hydroxy, COOH, CN, formyl or acyl to 1 to 6 carbons ;
r is 1 to 6 ;
each $R^{13}$ independently is H ; or linear $C_1$ to $C_6$ alkyl ;
each $R^{14}$ is independently H, or linear $C_1$ to $C_6$ alkyl ; and
each $R^{15}$ independently is H, linear $C_1$ to $C_6$ alkyl, or $CF_3$ ;
or a pharmaceutically acceptable salt thereof ; which process comprises
a) reacting a compound of formula II with a compound of formula III :

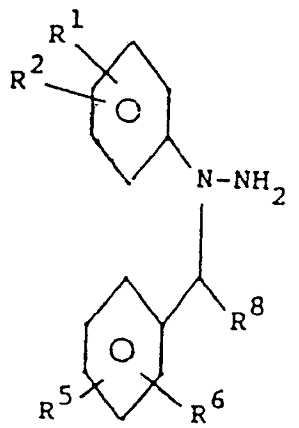

II

+

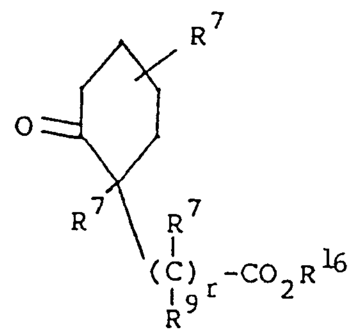

III

wherein $R^{16}$ represents an ester radical ; to produce a compound of formula I wherein $R^{10}$ represents COOH or an ester thereof ; or
b) reacting a compound of formula

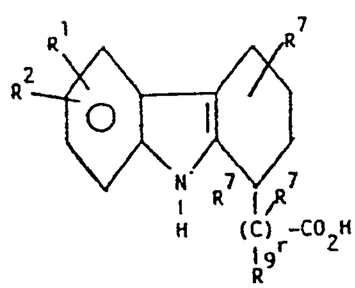

with a compound of formula (V) :

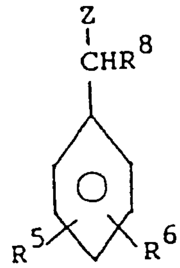

(V)

where Z is a leaving group ; to produce a compound of formula I wherein $R^{10}$ represents COOH or an ester thereof ; and

c) after step (a) or step (b), optionally converting one group $R^{10}$ to a different group $R^{10}$.

2. A process as claimed in claim 1 for the preparation of a compound which is :
2-[9-p-chlorobenzyl-6-fluoro-1,2,3,4-tetrahydrocarbazol-1-yl]-1-ethanol ;
2-(9-p-chlorobenzyl-6,8-difluoro-1,2,3,4-tetrahydrocarbazol-1-yl)ethanol ;
(–) or (+) 2-(9-p-chlorobenzyl-6,8-difluoro-1,2,3,4tetrahydrocarbazol-1-yl)ethanol ;
3-[9-p-chlorobenzyl-6-methoxy-1,2,3,4-tetrahydrocarbazol-1-yl]-propanol ;
9-benzyl-6-fluoro-1,2,3,4-tetrahydrocarbazol-1-ylacetic acid ;
9-p-chlorobenzyl-1,2,3,4-tetrahydrocarbazol-1-ylacetic acid ;
9-p-chlorobenzyl-5-fluoro-1,2,3,4-tetrahydrocarbazol-1-yl-acetic acid ;
6-bromo-9-p-chlorobenzyl-1,2,3,4-tetrahydrocarbazol-1-yl-acetic acid ;
9-p-chlorobenzyl-6-chloro-1,2,3,4-tetrahydrocarbazol-1-yl-acetic acid ;
9-p-chlorobenzyl-6-fluoro-1,2,3,4-tetrahydrocarbazol-1-yl-acetic acid ;
(–)9-p-chlorobenzyl-6-fluoro-1,2,3,4-tetrahydrocarbazol-1-yl-acetic acid ;
(+)9-p-chlorobenzyl-6-fluoro-1,2,3,4-tetrahydrocarbazol-1-yl-acetic acid ;
9-p-chlorobenzyl-6-methoxy-1,2,3,4-tetrahydrocarbazol-1-yl-acetic acid ;
9-p-chlorobenzyl-6-methyl-1,2,3,4-tetrahydrocarbazol-1-yl-acetic acid ;
9-p-chlorobenzyl-6-methylthio-1,2,3,4-tetrahydrocarbazol-1-yl-acetic acid ;
9-p-chlorobenzyl-6-methylsulfinyl-1,2,3,4-tetrahydrocarbazol-1-yl-acetic acid ;
9-p-chlorobenzyl-6-methylsulfonyl-1,2,3,4-tetrahydrocarbazol-1-yl-acetic acid ;
9-p-chlorobenzyl-6-trifluoromethyl-1,2,3,4-tetrahydrocarbazol-1-yl-acetic acid ;
9-p-chlorobenzyl-7-fluoro-1,2,3,4-tetrahydrocarbazol-1-yl-acetic acid ;
9-p-chlorobenzyl-8-fluoro-1,2,3,4-tetrahydrocarbazol-1-yl-acetic acid ;
(–)-9-p-chlorobenzyl-8-fluoro-1,2,3,4-tetrahydrocarbazol-1-yl-acetic acid ;
(+)-9-p-chlorobenzyl-8-fluoro-1,2,3,4-tetrahydrocarbazol-1-yl-acetic acid ;
9-p-chlorobenzyl-8-methyl-1,2,3,4-tetrahydrocarbazol-1-yl-acetic acid ;
(–)-9-p-chlorobenzyl-8-methyl-1,2,3,4-tetrahydrocarbazol-1-yl-acetic acid ;
(+)-9-p-chlorobenzyl-8-methyl-1,2,3,4-tetrahydrocarbazol-1-yl-acetic acid ;
9-p-chlorobenzyl-8-methylthio-1,2,3,4-tetrahydrocarbazol-1-yl-acetic acid ;
9-p-chlorobenzyl-8-methylsulfinyl-1,2,3,4-tetrahydrocarbazol-1-yl-acetic acid ;
9-p-chlorobenzyl-5,7-dichloro-1,2,3,4-tetrahydrocarbazol-1-yl-acetic acid ;
9-p-chlorobenzyl-6,8-dichloro-1,2,3,4-tetrahydrocarbazol-1-yl-acetic acid ;
9-p-chlorobenzyl-6,8-difluoro-1,2,3,4-tetrahydrocarbazol-1-yl-acetic acid ;
(–)-9-p-chlorobenzyl-6,8-difluoro-1,2,3,4-tetrahydrocarbazol-1-yl-acetic acid ;
(+)-9-p-chlorobenzyl-6,8-difluoro-1,2,3,4-tetrahydrocarbazol-1-yl-acetic acid ;
9-p-chlorobenzyl-6,8-dimethyl-1,2,3,4-tetrahydrocarbazol-1-yl-acetic acid ;
9-p-chlorobenzyl-6-fluoro-3-methyl-1,2,3,4-tetrahydrocarbazol-1-yl-acetic acid ;
9-p-chlorobenzyl-6-methoxy-8-methyl-1,2,3,4-tetrahydrocarbazol-1-yl-acetic acid ;
9-p-methoxybenzyl-6-fluoro-1,2,3,4-tetrahydrocarbazol-1-yl-acetic acid ;
9-(3,4-dichloro)benzyl-6-fluoro-1,2,3,4-tetrahydrocarbazol-1-yl-acetic acid ;
9-[1-(1-phenyl)ethyl]-6-fluoro-1,2,3,4-tetrahydrocarbazol-1-yl-acetic acid ;
2-[9-p-chlorobenzyl-6-fluoro-1,2,3,4-tetrahydrocarbazol-1-yl]propanoic acid ;
3-[9-p-chlorobenzyl-6-fluoro-1,2,3,4-tetrahydrocarbazol-1-yl]-propanoic acid ;

3-[9-p-chlorobenzyl-6-methoxy-1,2,3,4-tetrahydrocarbazol-1-yl]-propanoic acid ;
3. A process as claimed in claim 1 for the preparation of a compound which is :
9-p-chlorobenzyl-6-fluoro-1,2,3,4-tetrahydrocarbazol-1-yl-acetic acid ;
9-p-chlorobenzyl-8-methyl-1,2,3,4-tetrahydrocarbazol-1-yl-acetic acid ;
9-p-chlorobenzyl-8 fluoro-1,2,3,4-tetrahydrocarbazol-1-yl-acetic acid ;
9-p-chlorobenzyl-6,8-difluoro-1,2,3,4-tetrahydrocarbazol-1-yl-acetic acid.
4. A process as claimed in claim 1 for the preparation of a compound which is :
(–)9-p-chlorobenzyl-6-fluoro-1,2,3,4-tetrahydrocarbazol-1-yl-acetic acid ;
(–)-9-p-Chlorobenzyl-6,8-difluoro-1,2,3,4-tetrahydrocarbazol-1-yl-acetic acid ;
(–)-9-p-Chlorobenzyl-8-methyl-1,2,3,4-tetrahydrocarbazol-1-yl-acetic acid ;
(–)-9-p-Chlorobenzyl-8-fluoro-1,2,3,4-tetrahydrocarbazol-1-yl-acetic acid.
5. A process as claimed in claim 1 for the preparation of a compound of the formula :

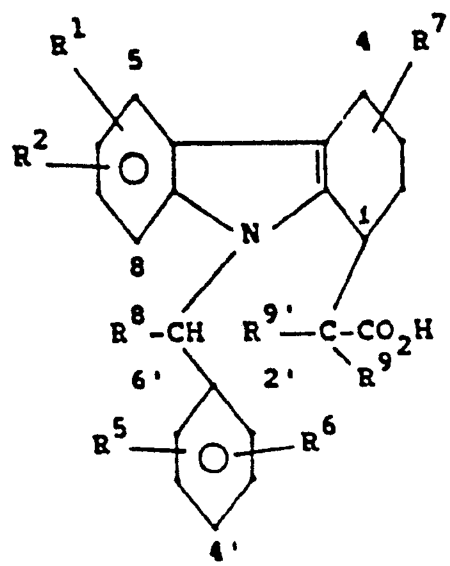

wherein:

| $R^1$ | $R^2$ | $R^5$ | $R^6$ | $R^9$,$R^{9'}$ | $R^7$ | $R^8$ |
|---|---|---|---|---|---|---|
| 6-F | H | H | H | H, H | H | H |
| 6-F | H | H | H | H, H | H | Me |
| 6-F | 8-F | 4'-n-$C_3H_7$ | H | H, H | H | H |
| 6-F | H | 4'-F | H | H, H | H | H |
| H | H | 4'-Cl | H | H, H | H | H |
| 5-F | H | 4'-Cl | H | H, H | H | H |
| 6-Me | H | 4'-Cl | H | H, H | H | H |
| 6-F | H | 4'-Cl | H | H, H | H | H |
| 6-F (-) isomer | H | 4'-Cl | H | H, H | H | H |
| 6-F (+) isomer | H | 4'-Cl | H | H, H | H | H |
| 6-Cl | H | 4'-Cl | H | H, H | H | H |
| 6-Br | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| 6-$CF_3$ | H | 4'-Cl | H | H, H | H | H |
| 6-OMe | H | 4'-Cl | H | H, H | H | H |
| 6-SMe | H | 4'-Cl | H | H, H | H | H |
| 6-S(O)Me | H | 4'-Cl | H | H, H | H | H |
| 6-$S(O)_2Me$ | H | 4'-Cl | H | H, H | H | H |
| 6-$N_3$ | H | 4'-Cl | H | H, H | H | H |
| 7-F | H | 4'-Cl | H | H, H | H | H |
| 8-Me | H | 4'-Cl | H | H, H | H | H |
| 8-Me(-)isomer | H | 4'-Cl | H | H, H | H | H |
| 8-Me(+)isomer | H | 4'-Cl | H | H, H | H | H |
| 8-F | H | 4'-Cl | H | H, H | H | H |
| 8-F(-)isomer | H | 4'-Cl | H | H, H | H | H |
| 8-F(+)isomer | H | 4'-Cl | H | H, H | H | H |
| 8-Br | H | 4'-Cl | H | H, H | H | H |
| 8-SMe | H | 4'-Cl | H | H, H | H | H |

| $R^1$ | $R^2$ | $R^5$ | $R^6$ | $R^9$, $R^{9'}$ | $R^7$ | $R^8$ |
|---|---|---|---|---|---|---|
| 8-S(O)Me | H | 4'-Cl | H | H, H | H | H |
| 6-Me | 8-Me | 4'-Cl | H | H, H | H | H |
| 6-F | 8-F | 4'-Cl | H | H, H | H | H |
| 6-F(-)isomer | 8-F | 4'-Cl | H | H, H | H | H |
| 6-F(+)isomer | 8-F | 4'-Cl | H | H, H | H | H |
| 5-Cl | 7-Cl | 4'-Cl | H | H, H | H | H |
| 6-Cl | 8-Cl | 4'-Cl | H | H, H | H | H |
| 6-F | 8-Me | 4'-Cl | H | H, H | H | H |
| 6-OMe | 8-Me | 4'-Cl | H | H, H | H | H |
| 6-F | H | 4'-Cl | H | H, H | 1-Me | H |
| 6-F | H | 4'-Cl | H | H, H | 3-Me | H |
| 6-F | H | 4'-Cl | H | Me, H | H | H |
| 6-F | H | 4'-Cl | H | Me, Me | H | H |
| 6-F | H | 4'-Br | H | H, H | H | H |
| 6-F | 8-F | 4'-I | H | H, H | H | H |

| $R^1$ | $R^2$ | $R^5$ | $R^6$ | $R^9, R^{9'}$ | $R^7$ | $R^8$ |
|---|---|---|---|---|---|---|
| 6-F | H | 4'-$N_3$ | H | H, H | H | H |
| 6-F | 8-F | 4'-OH | H | H, H | H | H |
| 6-F | H | 4'-OMe | H | H, H | H | H |
| 6-F | 8-F | 4'-OMe | H | H, H | H | H |
| 6-F | 8-F | 4'-SMe | H | H, H | H | H |
| 6-F | H | 4'-S(O)Me | H | H, H | H | H |
| 6-F | H | 4'-$S(O)_2Me$ | H | H, H | H | H |
| 6-F | 8-F | 4'-NHCOMe | H | H, H | H | H |
| 6-F | H | 4'-$CO_2H$ | H | H, H | H | H |
| 6-F | H | 4'-$CO_2Me$ | H | H, H | H | H |
| 6-F | 8-F | 2'-Cl | 4'-Cl | H, H | H | H |
| 6-F | H | 3'-Cl | 4'-Cl | H, H | H | H |
| 6-F | 8-F | 3'-Cl | 4'-Cl | H, H | H | H |
| 6-F | 8-F | 3'-I | 4'-OH | H, H | H | H |

6. A process for preparing a pharmaceutical composition which comprises mixing a compound of Formula I with a pharmaceutically acceptable carrier.

7. A process for preparing a pharmaceutical composition which comprises mixing a compound of Formula I with an effective amount of a second active ingredient selected from the group consisting of non-steroidal anti-inflammatory drugs ; peripheral analgesic agents ; cyclooxygenase inhibitors ; leukotriene antagonists ; leukotriene biosynthesis inhibitors ; $H_1$-receptor antagonists ; $H_2$ receptor antagonists ; prostaglandin antagonists ; ACE inhibitors, or thromboxane synthetase inhibitors.

8. A process as claimed in claim 7 wherein the second active ingredient is a non-steroidal anti-inflammatory drug.

9. A process as claimed in claim 8 wherein the non-steroidal anti-inflammatory drug is indomethacin.

10. A process as claimed in claim 7 wherein the weight ratio of said compound of Formula I to said second active ingredient ranges from about 200 : 1 to 1 : 200.

## Ansprüche

**Patentansprüche für die Vertragsstaaten : BE, CH, FR, DE, GB, NL, IT, LI, LU, SE**

1. Verbindung der Formel

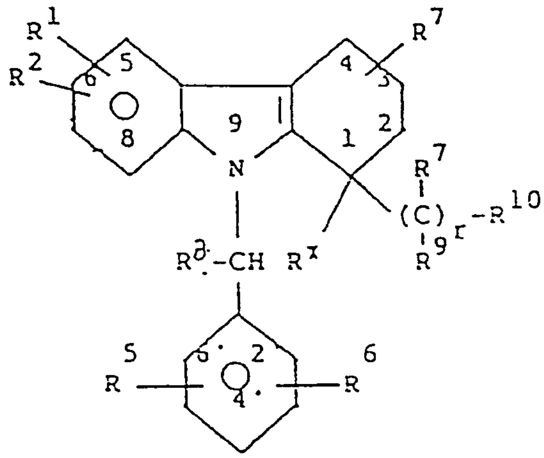

worin

$R^1$, $R^2$, $R^5$ und $R^6$ jeweils unabhängig ausgewählt sind aus

(1) Wasserstoff ;

(2) linearem Alkyl mit 1 bis 6 Kohlenstoffatomen ;

(3) M, worin M

a) $OR^{13}$ ;

b) Halogen ;

c) $CF_3$ ;

d) $SR^{13}$ ;

e) Phenyl ;

f) $COOR^{14}$ ;

g) 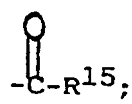 ;

h) Tetrazol ;

i) 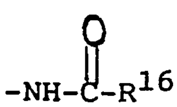, worin $R^{16}$ lineares $C_1$-$C_6$-Alkyl ist;

j) $-NR^{14}R^{14}$,

k) $-NHSO_2R^{17}$, worin $R^{17}$ lineares $C_1$-$C_6$-Alkyl oder $CF_3$ ist ;

l) $-SOR^{13}$ ;

m) $-CONR^{14}R^{14}$ ;

n) $-SO_2NR^{14}R^{14}$ ;

o) $-SO_2R^{13}$ ;

p) $NO_2$ ;

q) CN ;

r) $N_3$ ist ;

$R^7$ H oder lineares Alkyl mit 1 bis 6 Kohlenstoffen ist ;

$R^8$ H oder lineares Alkyl mit 1 bis 6 Kohlenstoffatomen ist ;

jedes $R^9$ unabhängig H, OH, lineares $C_1$-bis$C_4$-O-Alkyl oder lineares Alkyl mit 1 bis 4 Kohlenstoffen ist; $R^{10}$ COOH ; $CH_2OH$ ; CHO ; Tetrazol, $CONHSO_2R^{11}$ ; CN ;

$CON(R^9)_2$ oder $NHSO_2R^{11}$ ist, worin $R^{11}$ OH, lineares Alkyl oder lineares Alkoxy mit 1 bis 6 Kohlenstoffen, lineares Perhalogenalkyl mit 1 bis 6 Kohlenstoffen, Phenyl oder durch lineare Alkyl- oder lineare Alkoxygruppen mit 1 bis 3 Kohlenstoffen, Halogen, Hydroxy, COOH, CN, Formyl oder Acyl mit 1 bis 6 Kohlenstoffatomen substituiertes Phenyl ist ;

r 1 bis 6 ist ;

jedes $R^{13}$ unabhängig H oder lineares $C_1$-$C_6$-Alkyl ist ;

jedes $R^{14}$ unabhängig H oder lineares $C_1$-$C_6$-Alkyl ist ;

und jedes $R^{15}$ unabhängig H, lineares $C_1$-$C_6$-Alkyl oder $CF_3$ ist ;

oder ein pharmazeutisch annehmbares Salz davon.

2. Verbindung nach Anspruch 1, welche 2-[9-p-Chlorbenzyl-6-fluor-1,2,3,4-tetrahydrocarbazol1-yl]-1-ethanol ;

2-(9-p-Chlorbenzyl-6,8-difluor-1,2,3,4-tetrahydrocarbazol-1-yl)ethanol ;

(–)- oder (+)-2-(9-p-Chlorbenzyl-6,8-difluor-1,2,3,4-tetrahydrocarbazol-1-yl)ethanol ;

3-[9-p-Chlorbenzyl-6-methoxy-1,2,3,4-tetrahydrocarbazol-1-yl]propanol ;

9-Benzyl-6-fluor-1,2,3,4-tetrahydrocarbazol-1-yl-essigsäure ;

9-p-Chlorbenzyl-1,2,3,4-tetrahydrocarbazol-1-yl-essigsäure ;

9-p-Chlorbenzyl-5-fluor-1,2,3,4-tetrahydrocarbazol-1-yl-essigsäure ;

6-Brom-9-p-chlorbenzyl-1,2,3,4-tetrahydrocarbazol-1-yl-essigsäure ;

9-p-Chlorbenzyl-6-chlor-1,2,3,4-tetrahydrocarbazol-1-yl-essigsäure ;

9-p-Chlorbenzyl-6-fluor-1,2,3,4-tetrahydrocarbazol-1-yl-essigsäure ;

(–)-9-p-Chlorbenzyl-6-fluor-1,2,3,4-tetrahydrocarbazol-1-yl-essigsäure ;

(+)-9-p-Chlorbenzyl-6-fluor-1,2,3,4-tetrahydrocarbazol-1-yl-essigsäure ;

9-p-Chlorbenzyl-6-methoxy-1,2,3,4-tetrahydrocarbazol-1-yl-essigsäure ;

9-p-Chlorbenzyl-6-methyl-1,2,3,4-tetrahydrocarbazol-1-yl-essigsäure ;

9-p-Chlorbenzyl-6-methylthio-1,2,3,4-tetrahydrocarbazol-1-yl-essigsäure ;

9-p-Chlorbenzyl-6-methylsulfinyl-1,2,3,4-tetrahydrocarbazol-1-yl-essigsäure ;

9-p-Chlorbenzyl-6-methylsulfonyl-1,2,3,4-tetrahydrocarbazol-1-yl-essigsäure ;

9-p-Chlorbenzyl-6-trifluormethyl-1,2,3,4-tetrahydrocarbazol-1-yl-essigsäure ;

9-p-Chlorbenzyl-7-fluor-1,2,3,4-tetrahydrocarbozol-1-yl-essigsäure ;

9-p-Chlorbenzyl-8-fluor-1,2,3,4-tetrahydrocarbazol-1-yl-essigsäure ;

(–)-9-p-Chlorbenzyl-8-fluor-1,2,3,4-tetrahydrocarbazol-1-yl-essigsäure ;

(+)-9-p-Chlorbenzyl-8-fluor-1,2,3,4-tetrahydrocarbazol-1-yl-essigsäure ;

9-p-Chlorbenzyl-8-methyl-1,2,3,4-tetrahydrocarbazol-1-yl-essigsäure ;

(–)-9-p-Chlorbenzyl-8-methyl-1,2,3,4-tetrahydrocarbazol-1-yl-essigsäure ;

(+)-9-p-Chlorbenzyl-8-methyl-1,2,3,4-tetrahydrocarbazol-1-yl-essigsäure ;

9-p-Chlorbenzyl-8-methylthio-1,2,3,4-tetrahydrocarbazol-1-yl-essigsäure ;

9-p-Chlorbenzyl-8-methylsulfinyl-1,2,3,4-tetrahydrocarbazol-1-yl-essigsäure ;

9-p-Chlorbenzyl-5,7-dichlor-1,2,3,4-tetrahydrocarbazol-1-yl-essigsäure ;

9-p-Chlorbenzyl-6,8-dichlor-1,2,3,4-tetrahydrocarbazol-1-yl-essigsäure ;

9-p-Chlorbenzyl-6,8-difluor-1,2,3,4-tetrahydrocarbazol-1-yl-essigsäure ;

(–)-9-p-Chlorbenzyl-6,8-difluor-1,2,3,4-tetrahydrocarbazol-1-yl-essigsäure ;

(+)-9-p-Chlorbenzyl-6,8-difluor-1,2,3,4-tetrahydrocarbazol-1-yl-essigsäure ;

9-p-Chlorbenzyl-6,8-dimethyl-1,2,3,4-tetrahydrocarbazol-1-yl-essigsäure ;

9-p-Chlorbenzyl-6-fluor-3-methyl-1,2,3,4-tetrahydrocarbazol-1-yl-essigsäure ;

9-p-Chlorbenzyl-6-methoxy-8-methyl-1,2,3,4-tetrahydrocarbazol-1-yl-essigsäure ;

9-p-Methoxybenzyl-6-fluor-1,2,3,4-tetrahydrocarbazol-1-yl-essigsäure ;

9-(3,4-Dichlor)benzyl-6-fluor-1,2,3,4-tetrahydrocarbazol-1-yl-essigsäure ;

9-[1-(1-Phenyl)ethyl]-6-fluor-1,2,3,4-tetrahydrocarbazol-1-yl-essigsäure ;

2-[9-p-Chlorbenzyl-6-fluor-1,2,3,4-tetrahydrocarbazol1-yl]-propionsäure ;

3-[9-p-Chlorbenzyl-6-fluor-1,2,3,4-tetrahydrocarbazol-1-yl]-propionsäure ;

3-[9-p-Chlorbenzyl-6-methoxy-1,2,3,4-tetrahydrocarbazol-1-yl]-propionsäure ist.

3. Verbindung nach Anspruch 1, welche 9-p-Chlorbenzyl-6-fluor-1,2,3,4-tetrahydrocarbazol-1-yl-essigsäure ;

9-p-Chlorbenzyl-8-methyl-1,2,3,4-tetrahydrocarbazol-1-yl-essigsäure ;

9-p-Chlorbenzyl-8-fluor-1,2,3,4-tetrahydrocarbazol-1-yl-essigsäure ;

9-p-Chlorbenzyl-6,8-difluor-1,2,3,4-tetrahydrocarbazol-1-yl-essigsäure ist.

4. Verbindung nach Anspruch 1, welche
(–)-9-p-Chlorbenzyl-6-fluor-1,2,3,4-tetrahydrocarbazol-1-yl-essigsäure ;
(–)-9-p-Chlorbenzyl-6,8-difluor-1,2,3,4-tetrahydrocarbazol-1-yl-essigsäure ;
(–)-9-p-Chlorbenzyl-8-methyl-1,2,3,4-tetrahydrocarbazol-1-yl-essigsäure ;
(–)-9-p-Chlorbenzyl-8-fluor-1,2,3,4-tetrahydrocarbazol-1-yl-essigsäure ist.

5. Verbindung der Formel

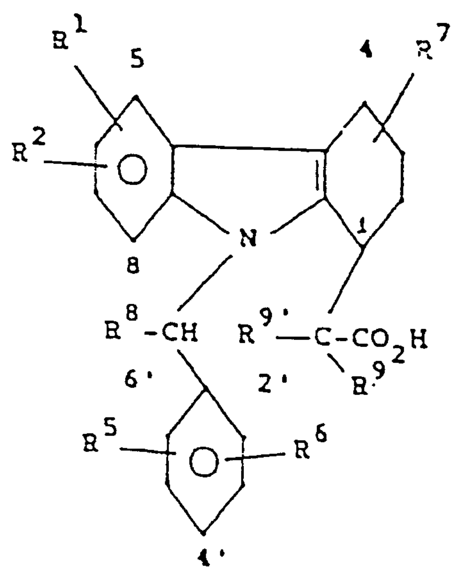

worin

| $R^1$ | $R^2$ | $R^5$ | $R^6$ | $R^9$, $R^{9'}$ | $R^7$ | $R^8$ |
|---|---|---|---|---|---|---|
| 6-F | H | H | H | H, H | H | H |
| 6-F | H | H | H | H, H | H | Me |
| 6-F | 8-F | 4'-n-$C_3H_7$ | H | H, H | H | H |
| 6-F | H | 4'-F | H | H, H | H | H |
| H | H | 4'-Cl | H | H, H | H | H |
| 5-F | H | 4'-Cl | H | H, H | H | H |
| 6-Me | H | 4'-Cl | H | H, H | H | H |
| 6-F | H | 4'-Cl | H | H, H | H | H |
| 6-F (-)-Isomer | H | 4'-Cl | H | H, H | H | H |
| 6-F (+)-Isomer | H | 4'-Cl | H | H, H | H | H |
| 6-Cl | H | 4'-Cl | H | H, H | H | H |
| 6-Br | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| 6-$CF_3$ | H | 4'-Cl | H | H, H | H | H |
| 6-OMe | H | 4'-Cl | H | H, H | H | H |
| 6-SMe | H | 4'-Cl | H | H, H | H | H |
| 6-S(O)Me | H | 4'-Cl | H | H, H | H | H |
| 6-$S(O)_2Me$ | H | 4'-Cl | H | H, H | H | H |
| 6-$N_3$ | H | 4'-Cl | H | H, H | H | H |
| 7-F | H | 4'-Cl | H | H, H | H | H |
| 8-Me | H | 4'-Cl | H | H, H | H | H |
| 8-Me(-) isomer | H | 4'-Cl | H | H, H | H | H |
| 8-Me(+) isomer | H | 4'-Cl | H | H, H | H | H |
| 8-F | H | 4'-Cl | H | H, H | H | H |
| 8-F(-) isomer | H | 4'-Cl | H | H, H | H | H |
| 8-F(+) isomer | H | 4'-Cl | H | H, H | H | H |
| 8-Br | H | 4'-Cl | H | H, H | H | H |
| 8-SMe | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| 8-S(O)Me | H | 4'-Cl | H | H, H | H | H |
| 6-Me | 8-Me | 4'-Cl | H | H, H | H | H |
| 6-F | 8-F | 4'-Cl | H | H, H | H | H |
| 6-F(-)isomer | 8-F | 4'-Cl | H | H, H | H | H |
| 6-F(+)isomer | 8-F | 4'-Cl | H | H, H | H | H |
| 5-Cl | 7-Cl | 4'-Cl | H | H, H | H | H |
| 6-Cl | 8-Cl | 4'-Cl | H | H, H | H | H |
| 6-F | 8-Me | 4'-Cl | H | H, H | H | H |
| 6-OMe | 8-Me | 4'-Cl | H | H, H | H | H |
| 6-F | H | 4'-Cl | H | H, H | 1-Me | H |
| 6-F | H | 4'-Cl | H | H, H | 3-Me | H |
| 6-F | H | 4'-Cl | H | Me, H | H | H |
| 6-F | H | 4'-Cl | H | Me, Me | H | H |
| 6-F | H | 4'-Br | H | H, H | H | H |
| 6-F | 8-F | 4'-I | H | H, H | H | H |

| | $R^1$ | $R^2$ | $R^5$ | $R^6$ | $R^9$,$R^{9'}$ | $R^7$ | $R^8$ |
|---|---|---|---|---|---|---|---|
| 45 | 6-F | 8-F | 4'-OMe | H | H,H | H | H |
| 46 | 6-F | 8-F | 4'-OH | H | H,H | H | H |
| 47 | 6-F | 8-F | 4'-SMe | H | H,H | H | H |
| 48 | 6-F | H | 4'-S(O)Me | H | H,H | H | H |
| 49 | 6-F | 8-F | 4'-NHCOMe | H | H,H | H | H |
| 50 | 6-F | H | 4'-$S(O)_2$Me | H | H,H | H | H |
| 51 | 6-F | H | 4'-F | H | H,H | H | H |
| 52 | 6-F | H | 4'-Br | H | H,H | H | H |
| 53 | 6-F | 8-Me | 4'-Cl | H | H,H | H | H |
| 54 | 6-F | H | 4'-$CO_2H$ | H | H,H | H | H |
| 55 | 6-F | H | 4'-$CO_2$Me | H | H,H | H | H |
| 56 | 6-F | 8-F | 4'-n-$C_3H_7$ | H | H,H | H | H |
| 57 | 6-F | 8-F | 3'-I | 4'-OH | H,H | H | H |
| 58 | 6-F | 8-F | 4'-I | H | H,H | H | H |
| 59 | 6-$N_3$ | H | 4'-Cl | H | H,H | H | H |
| 60 | 6-F | H | 4'-$N_3$ | H | H,H | H | H |

6. Verwendung einer Verbindung nach Anspruch 1 zur Herstellung eines Medikaments zur Inhibierung der Leucotriensynthese in einem Säugetier.

7. Verwendung einer Verbindung nach Anspruch 1 zur Herstellung eines Medikaments zur Antagonisierung von Prostaglandinen in Säugetieren.

8. Pharmazeutische Zusammensetzung, die eine Verbindung nach Anspruch 1 und einen pharmazeutisch annehmbaren Träger umfaßt.

9. Pharmazeutische Zusammensetzung, die eine Verbindung nach Anspruch 1 und eine wirksame Menge eines zweiten wirksamen Bestandteils, ausgewählt aus der aus nicht steroiden entzündungshemmenden Mitteln, peripheren Analgetika, Cyclooxygenaseinhibitoren, Leukotrienantagonisten, Leukotrien-Biosynthese-Inhibitoren, $H_1$-Receptor-Antagonisten, $H_2$-Receptor-Antagonisten, Prostaglandinantagonisten, ACE-Inhibitoren oder Thromboxan-Synthetase-Inhibitoren bestehenden Gruppe, umfaßt.

10. Pharmazeutische Zusammensetzung nach Anspruch 9, worin der zweite wirksame Bestandteil Indomethacin ist.

**Patentansprüche für die Vertragsstaaten : AT, ES, GR**

1. Verfahren zur Herstellung einer Verbindung der Formel

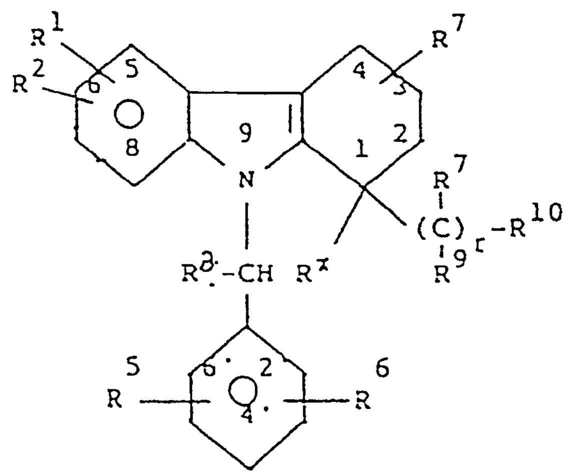

worin

$R^1$, $R^2$, $R^5$ und $R^6$ jeweils unabhängig ausgewählt sind aus

(1) Wasserstoff ;

(2) linearem Alkyl mit 1 bis 6 Kohlenstoffatomen ;

(3) M, worin M

a) $OR^{13}$ ;

b) Halogen ;

c) $CF_3$ ;

d) $SR^{13}$ ;

e) Phenyl ;

f) $COOR^{14}$ ;

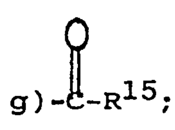

h) Tetrazol ;

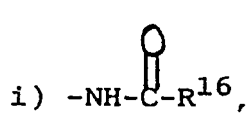

worin $R^{16}$ lineares $C_1$-$C_6$-Alkyl ist ;

j) $-NR^{14}R^{14}$ ;

k) $-NHSO_2R^{17}$, worin $R^{17}$ lineares $C_1$-$C_6$-Alkyl oder $CF_3$ ist ;

l) $-SOR^{13}$ ;
m) $-CONR^{14}R^{14}$ ;
n) $-SO_2NR^{14}R^{14}$ ;
o) $-SO_2R^{13}$ ;
p) $NO_2$ ;
q) CN ;
r) $N_3$ ist ;
$R^7$ H oder lineares Alkyl mit 1 bis 6 Kohlenstoffen ist ;
$R^8$ H oder lineares Alkyl mit 1 bis 6 Kohlenstoffatomen ist ;
jedes $R^9$ unabhängig H, OH, lineares $C_1$-bis$C_4$-O-Alkyl oder lineares Alkyl mit 1 bis 4 Kohlenstoffen ist;

$R^{10}$ COOH ; $CH_2OH$ ; CHO ; Tetrazol, $CONHSO_2R^{11}$ ; CN ; $CON(R^9)2$ oder $NHSO_2R^{11}$ ist, worin $R^{11}$ OH, lineares Alkyl oder lineares Alkoxy mit 1 bis 6 Kohlenstoffen, lineares Perhalogenalkyl mit 1 bis 6 Kohlenstoffen, Phenyl oder durch lineare Alkyl- oder lineare Alkoxygruppen mit 1 bis 3 Kohlenstoffen, Halogen, Hydroxy, COOH, CN, Formyl oder Acyl mit 1 bis 6 Kohlenstoffatomen substituiertes Phenyl ist ;

r 1 bis 6 ist ;
jedes $R^{13}$ unabhängig H oder lineares $C_1$-$C_6$-Alkyl ist ;
jedes $R^{14}$ unabhängig H oder lineares $C_1$-$C_6$-Alkyl ist ;
und jedes $R^{15}$ unabhängig H, lineares $C_1$-$C_6$-Alkyl oder $CF_3$ ist ;
oder eines pharmazeutisch annehmbaren Salzes davon, welches Verfahren

a) die Umsetzung einer Verbindung der Formel II mit einer Verbindung der Formel III

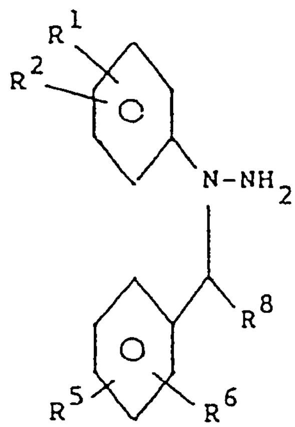

II

+

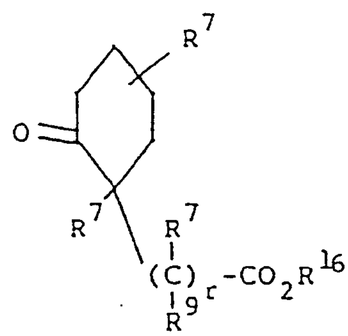

III

worin $R^{16}$ einen Esterrest darstellt, zur Erzeugung einer Verbindung der Formel I, worin $R^{10}$ COOH oder einen Ester davon darstellt ; oder

b) die Umsetzung einer Verbindung der Formel

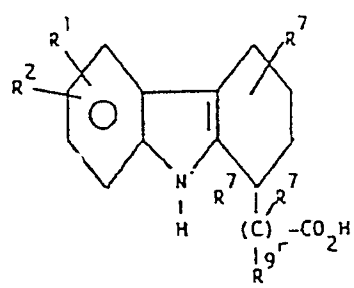

mit einer Verbindung der Formel (V)

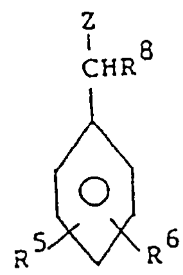

(V)

worin Z eine austretende Gruppe ist ; unter Erzeugung einer Verbindung der Formel I, worin $R^{10}$ COOH oder einen Ester davon darstellt ; und

c) nach Schritt (a) oder Schritt (b) gegebenenfallsdie Umwandlung einer $R^{10}$-Gruppe in eine andere $R^{10}$-Gruppe umfaßt.

2. Verfahren nach Anspruch 1 zur Herstelung einer Verbindung, welche

2-[9-p-Chlorbenzyl-6-fluor-1,2,3,4-tetrahydrocarbazol-1-yl]-1-ethanol ;
2-(9-p-Chlorbenzyl-6,8-difluor-1,2,3,4-tetrahydrocarbazol-1-yl)ethanol ;
(–)- oder (+)-2-(9-p-Chlorbenzyl-6,8-difluor-1,2,3,4-tetrahydrocarbazol-1-yl)ethanol ;
3-[9-p-Chlorbenzyl-6-methoxy-1,2,3,4-tetrahydrocarbazol-1-yl]propanol ;
9-Benzyl-6-fluor-1,2,3,4-tetrahydrocarbazol-1-yl-essigsäure ;
9-p-Chlorbenzyl-1,2,3,4-tetrahydrocarbazol-1-yl-essigsäure ;
9-p-Chlorbenzyl-5-fluor-1,2,3,4-tetrahydrocarbazol-1-yl-essigsäure ;
6-Brom-9-p-chlorbenzyl-1,2,3,4-tetrahydrocarbazol-1-yl-essigsäure ;
9-p-Chlorbenzyl-6-chlor-1,2,3,4-tetrahydrocarbazol-1-yl-essigsäure ;
9-p-Chlorbenzyl-6-fluor-1,2,3,4-tetrahydrocarbazol-1-yl-essigsäure ;
(–)-9-p-Chlorbenzyl-6-fluor-1,2,3,4-tetrahydrocarbazol-1-yl-essigsäure ;
(+)-9-p-Chlorbenzyl-6-fluor-1,2,3,4-tetrahydrocarbazol-1-yl-essigsäure ;
9-p-Chlorbenzyl-6-methoxy-1,2,3,4-tetrahydrocarbazol-1-yl-essigsäure ;
9-p-Chlorbenzyl-6-methyl-1,2,3,4-tetrahydrocarbazol-1-yl-essigsäure ;
9-p-Chlorbenzyl-6-methylthio-1,2,3,4-tetrahydrocarbazol-1-yl-essigsäure ;
9-p-Chlorbenzyl-6-methylsulfinyl-1,2,3,4-tetrahydrocarbazol-1-yl-essigsäure ;
9-p-Chlorbenzyl-6-methylsulfonyl-1,2,3,4-tetrahydrocarbazol-1-yl-essigsäure ; '
9-p-Chlorbenzyl-6-trifluormethyl-1,2,3,4-tetrahydrocarbazol-1-yl-essigsäure ;
9-p-Chlorbenzyl-7-fluor-1,2,3,4-tetrahydrocarbozol-1-yl-essigsäure ;
9-p-Chlorbenzyl-8-fluor-1,2,3,4-tetrahydrocarbazol-1-yl-essigsäure ;
(–)-9-p-Chlorbenzyl-8-fluor-1,2,3,4-tetrahydrocarbazol-1-yl-essigsäure ;
(+)-9-p-Chlorbenzyl-8-fluor-1,2,3,4-tetrahydrocarbazol-1-yl-essigsäure ;
9-p-Chlorbenzyl-8-methyl-1,2,3,4-tetrahydrocarbazol-1-yl-essigsäure ;
(–)-9-p-Chlorbenzyl-8-methyl-1,2,3,4-tetrahydrocarbazol-1-yl-essigsäure ;
(+)-9-p-Chlorbenzyl-8-methyl-1,2,3,4-tetrahydrocarbazol-1-yl-essigsäure ;
9-p-Chlorbenzyl-8-methylthio-1,2,3,4-tetrahydrocarbazol-1-yl-essigsäure ;
9-p-Chlorbenzyl-8-methylsulfinyl-1,2,3,4-tetrahydrocarbazol-1-yl-essigsäure ;
9-p-Chlorbenzyl-5,7-dichlor-1,2,3,4-tetrahydrocarbazol-1-yl-essigsäure ;
9-p-Chlorbenzyl-6,8-dichlor-1,2,3,4-tetrahydrocarbazol-1-yl-essigsäure ;
9-p-Chlorbenzyl-6,8-difluor-1,2,3,4-tetrahydrocarbazol-1-yl-essigsäure ;
(–)-9-p-Chlorbenzyl-6,8-difluor-1,2,3,4-tetrahydrocarbazol-1-yl-essigsäure ;
(+)-9-p-Chlorbenzyl-6,8-difluor-1,2,3,4-tetrahydrocarbazol-1-yl-essigsäure ;
9-p-Chlorbenzyl-6,8-dimethyl-1,2,3,4-tetrahydrocarbazol-1-yl-essigsäure ;
9-p-Chlorbenzyl-6-fluor-3-methyl-1,2,3,4-tetrahydrocarbazol-1-yl-essigsäure ;
9-p-Chlorbenzyl-6-methoxy-8-methyl-1,2,3,4-tetrahydrocarbazol-1-yl-essigsäure ;
9-p-Methoxybenzyl-6-fluor-1,2,3,4-tetrahydrocarbazol-1-yl-essigsäure ;
9-(3,4-Dichlor)benzyl-6-fluor-1,2,3,4-tetrahydrocarbazol-1-yl-essigsäure ;
9-[1-(1-Phenyl)ethyl]-6-fluor-1,2,3,4-tetrahydrocarbazol-1-yl-essigsäure ;
2-[9-p-Chlorbenzyl-6-fluor-1,2,3,4-tetrahydrocarbazol-1-yl]-propionsäure ;

3-[9-p-Chlorbenzyl-6-fluor-1,2,3,4-tetrahydrocarbazol-1-yl]-propionsäure ;
3-[9-p-Chlorbenzyl-6-methoxy-1,2,3,4-tetrahydrocarbazol-1-yl]-propionsäure ist.

3. Verfahren nach Anspruch 1 zur Herstellung einer Verbindung, welche
9-p-Chlorbenzyl-6-fluor-1,2,3,4-tetrahydrocarbazol-1-yl-essigsäure ;
9-p-Chlorbenzyl-8-methyl-1,2,3,4-tetrahydrocarbazol-1-yl-essigsäure ;
9-p-Chlorbenzyl-8-fluor-1,2,3,4-tetrahydrocarbazol-1-yl-essigsäure ;
9-p-Chlorbenzyl-6,8-difluor-1,2,3,4-tetrahydrocarbazol-1-yl-essigsäure ist.

4. Verfahren nach Anspruch 1 zur Herstellung einer Verbindung, welche
(–)-9-p-Chlorbenzyl-6-fluor-1,2,3,4-tetrahydrocarbazol-1-yl-essigsäure ;
(–)-9-p-Chlorbenzyl-6,8-difluor-1,2,3,4-tetrahydrocarbazol-1-yl-essigsäure ;
(–)-9-p-Chlorbenzyl-8-methyl-1,2,3,4-tetrahydrocarbazol-1-yl-essigsäure ;
(–)-9-p-Chlorbenzyl-8-fluor-1,2,3,4-tetrahydrocarbazol-1-yl-essigsäure ist.

5. Verfahren nach Anspruch 1 zur Herstellung einer Verbindung der Formel

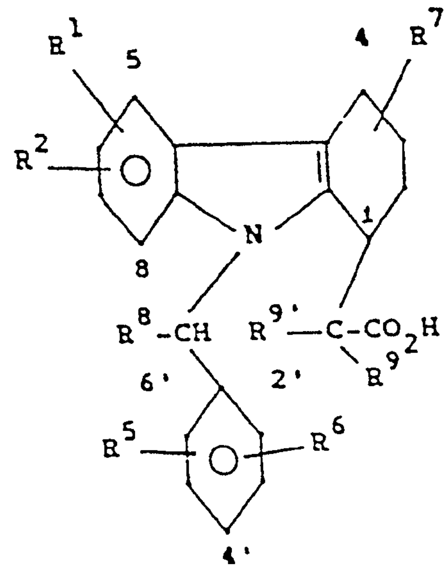

worin

| $R^1$ | $R^2$ | $R^5$ | $R^6$ | $R^9, R^{9'}$ | $R^7$ | $R^8$ |
|---|---|---|---|---|---|---|
| 6-F | H | H | H | H, H | H | H |
| 6-F | H | H | H | H, H | H | Me |
| 6-F | 8-F | 4'-n-$C_3H_7$ | H | H, H | H | H |
| 6-F | H | 4'-F | H | H, H | H | H |
| H | H | 4'-Cl | H | H, H | H | H |
| 5-F | H | 4'-Cl | H | H, H | H | H |
| 6-Me | H | 4'-Cl | H | H, H | H | H |
| 6-F | H | 4'-Cl | H | H, H | H | H |
| 6-F (-) isomer | H | 4'-Cl | H | H, H | H | H |
| 6-F (+) isomer | H | 4'-Cl | H | H, H | H | H |
| 6-Cl | H | 4'-Cl | H | H, H | H | H |
| 6-Br | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| 6-$CF_3$ | H | 4'-Cl | H | H, H | H | H |
| 6-OMe | H | 4'-Cl | H | H, H | H | H |
| 6-SMe | H | 4'-Cl | H | H, H | H | H |
| 6-S(O)Me | H | 4'-Cl | H | H, H | H | H |
| 6-$S(O)_2Me$ | H | 4'-Cl | H | H, H | H | H |
| 6-$N_3$ | H | 4'-Cl | H | H, H | H | H |
| 7-F | H | 4'-Cl | H | H, H | H | H |
| 8-Me | H | 4'-Cl | H | H, H | H | H |
| 8-Me(-)isomer | H | 4'-Cl | H | H, H | H | H |
| 8-Me(+)isomer | H | 4'-Cl | H | H, H | H | H |
| 8-F | H | 4'-Cl | H | H, H | H | H |
| 8-F(-)isomer | H | 4'-Cl | H | H, H | H | H |
| 8-F(+)isomer | H | 4'-Cl | H | H, H | H | H |
| 8-Br | H | 4'-Cl | H | H, H | H | H |
| 8-SMe | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| 8-S(O)Me | H | 4'-Cl | H | H, H | H | H |
| 6-Me | 8-Me | 4'-Cl | H | H, H | H | H |
| 6-F | 8-F | 4'-Cl | H | H, H | H | H |
| 6-F(-) isomer | 8-F | 4'-Cl | H | H, H | H | H |
| 6-F(+) isomer | 8-F | 4'-Cl | H | H, H | H | H |
| 5-Cl | 7-Cl | 4'-Cl | H | H, H | H | H |
| 6-Cl | 8-Cl | 4'-Cl | H | H, H | H | H |
| 6-F | 8-Me | 4'-Cl | H | H, H | H | H |
| 6-OMe | 8-Me | 4'-Cl | H | H, H | H | H |
| 6-F | H | 4'-Cl | H | H, H | 1-Me | H |
| 6-F | H | 4'-Cl | H | H, H | 3-Me | H |
| 6-F | H | 4'-Cl | H | Me, H | H | H |
| 6-F | H | 4'-Cl | H | Me, Me | H | H |
| 6-F | H | 4'-Br | H | H, H | H | H |
| 6-F | 8-F | 4'-I | H | H, H | H | H |

| $R^1$ | $R^2$ | $R^5$ | $R^6$ | $R^9, R^{9'}$ | $R^7$ | $R^8$ |
|---|---|---|---|---|---|---|
| 6-F | H | 4'-$N_3$ | H | H, H | H | H |
| 6-F | 8-F | 4'-OH | H | H, H | H | H |
| 6-F | H | 4'-OMe | H | H, H | H | H |
| 6-F | 8-F | 4'-OMe | H | H, H | H | H |
| 6-F | 8-F | 4'-SMe | H | H, H | H | H |
| 6-F | H | 4'-S(O)Me | H | H, H | H | H |
| 6-F | H | 4'-$S(O)_2Me$ | H | H, H | H | H |
| 6-F | 8-F | 4'-NHCOMe | H | H, H | H | H |
| 6-F | H | 4'-$CO_2H$ | H | H, H | H | H |
| 6-F | H | 4'-$CO_2Me$ | H | H, H | H | H |
| 6-F | 8-F | 2'-Cl | 4'-Cl | H, H | H | H |
| 6-F | H | 3'-Cl | 4'-Cl | H, H | H | H |
| 6-F | 8-F | 3'-Cl | 4'-Cl | H, H | H | H |
| 6-F | 8-F | 3'-I | 4'-OH | H, H | H | H |

30

6. Verfahren zur Herstellung einer pharmazeutischen Zusammensetzung durch Mischen einer Verbindung der Formel I mit einem pharmazeutisch annehmbaren Träger.

7. Verfahren zur Herstellung einer pharmazeutischen Zusammensetzung durch Mischen einer Verbindung der Formel I mit einer wirksamen Menge eines zweiten aktiven Bestandteils, ausgewählt aus der aus nicht steroiden entzündungshemmenden Mitteln, peripheren Analgetika, Cyklooxygenaseinhibitoren, Leukotrienantagonisten, Leukotrien-Biosynthese-Inhibitoren, $H_1$-Receptorantagonisten, $H_2$-Receptor-Antagonisten, Prostaglandin-Antagonisten, ACE-Inhibitoren oder Thromboxan-Synthetase-Inhibitoren bestehenden Gruppe, umfaßt.

8. Verfahren nach Anspruch 7, worin der zweite wirksame Bestandteil ein nicht steroides entzündungshemmendes Mittel ist.

9. Verfahren nach Anspruch 8, worin das nicht-steroide entzündungshemmende Mittel Indomethacin ist.

10. Verfahren nach Anspruch 7, worin das Gewichtsverhältnis der Verbindung der Formel I zu dem zweiten wirksamen Bestandteil im Bereich von etwa 200 : 1 bis 1 : 200 liegt.

## Revendications

**Revendications pour les Etats contractants : BE, CH, FR, DE, GB, NL, IT, LI LU, SE**

1. Composé de formule

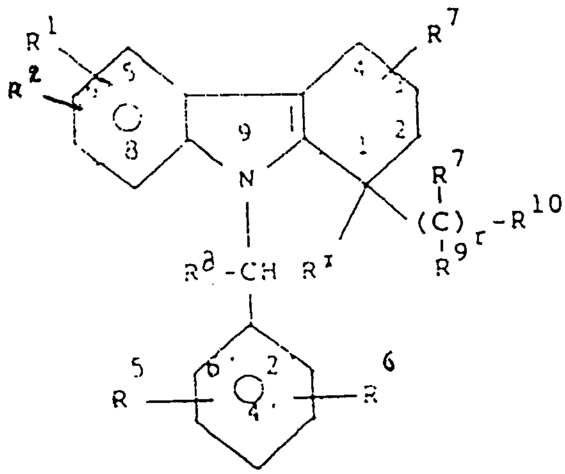

où

$R^1$, $R^2$, $R^5$ et $R^6$ sont choisis chacun indépendamment parmi :
(a) hydrogène ;
(2) alcoyle linéaire en $C_{1-6}$ ;
(3) M
où M représente
a) $OR^{13}$ ;
b) halogène ;
c) $CF_3$ ;
d) $SR^{13}$ ;
e) phényle ;
f) $COOR^{14}$ ;

g) 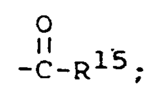

h) tétrazole ;

i) 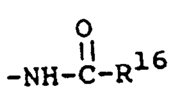

où $R^{16}$ est un alcoyle linéaire en $C_{1-8}$ ;
j) $NR^{14}R^{14}$ ;
k) $-NHSO_2R^{17}$ où $R^{17}$ est un alcoyle linéaire en $C_{1-6}$, ou $CF_3$ ;
l) $-SOR^{13}$

m) $CONR^{14}R^{14}$ ;
n) $-SO_2NR^{14}R^{14}$ ;
o) $-SO_2R^{13}$ ;
p) $NO_2$ ;
q) CN ;
r) $N_3$ ;
$R^7$ représente H ou un alcoyle linéaire en $C_{1-6}$ ;
$R^8$ représente H ou un alcoyle linéaire en $C_{1-6}$ ;
chaque $R^9$ représente indépendamment H, OH, un O-alcoyle linéaire en $C_{1-4}$ ou un alcoyle linéaire en $C_{1-4}$ ;
$R^{10}$ représente COOH ; $CH_2OH$ ; CHO ; tétrazole, $CONHSO_2R^{11}$ ; CN ; $CON(R^9)_2$ ; ou $NHSO_2R^{11}$ où $R^{11}$ représente OH un alcoyle linéaire ou un alcoxy linéaire en $C_{1-6}$, un perhaloalcoyle en $C_{1-6}$, un phényle ou un phényle substitué par un alcoyle linéaire ou des groupes alcoxy linéaires en $C_{1-3}$, halogène, hydroxy, COOH, CN, formyle ou acyle en $C_{1-6}$ ;
r vaut de 1 à 6 ;
chaque $R^{13}$ représente indépendamment H ; ou un alcoyle linéaire en $C_{1-6}$ ;
chaque $R^{14}$ représente indépendamment H, ou un alcoyle linéaire en $C_{1-6}$ ; et
chaque $R^{15}$ représente indépendamment H, un alcoyle linéaire en $C_{1-6}$ ou $CF_3$ ;
ou un de leurs sels pharmaceutiquement acceptables.

2. Composé selon la revendication 1, qui est
2-[9-p-chlorobenzyl-6-fluoro-1,2,3,4-tétrahydrocarbazol-1-yl]-1-éthanol ;
2-(9-chlorobenzyl-6,8-difluoro-1,2,3,4-tétrahydrocarbazol-1-yl)éthanol ;
(–) ou (+) 2-(9-p-chlorobenzyl)-6,8-difluoro-1,2,3,4-tétrahydrocarbazol-1-yl)éthanol ;
3-[9-p-chlorobenzyl-6-méthoxy-1,2,3,4-tétrahydrocarbazol-1-yl]-propanol ;
Acide 9-benzyl-6-fluoro-1,2,3,4-tétrahydrocarbazol-1-yl-acétique ;
Acide 9-p-chlorobenzyl-1,2,3,4-tétrahydrocarbazol-1-yl-acétique ;
Acide 9-p-chlorobenzyl-5-fluoro-1,2,3,4-tétrahydrocarbazol-1-yl-acétique ;
Acide 6-bromo-9-p-chlorobenzyl-1,2,3,4-tétrahydrocarbazol-1-yl-acétique ;
Acide 9-p-chlorobenzyl-6-chloro-1,2,3,4-tétrahydrocarbazol-1-yl-acétique ;
Acide 9-p-chlorobenzyl-6-fluoro-1,2,3,4-tétrahydrocarbazol-1-yl-acétique ;
Acide (–)9-p-chlorobenzyl-6-fluoro-1,2,3,4-tétrahydrocarbazol-1-yl-acétique ;
Acide (+)9-p-chlorobenzyl-6-fluoro-1,2,3,4-tétrahydrocarbazol-1-yl-acétique ;
Acide 9-p-chlorobenzyl-6-méthoxy-1,2,3,4-tétrahydrocarbazol-1-yl-acétique ;
Acide 9-p-chlorobenzyl-6-méthyl-1,2,3,4-tétrahydrocarbazol-1-yl-acétique ;
Acide 9-p-chlorobenzyl-6-méthylthio-1,2,3,4-tétrahydrocarbazol-1-yl-acétique ;
Acide 9-p-chlorobenzyl-6-méthylsulfinyl-1,2,3,4-tétrahydrocarbazol-1-yl-acétique ;
Acide 9-p-chlorobenzyl-6-méthylsulfonyl-1,2,3,4-tétrahydrocarbazol-1-yl-acétique ;
Acide 9-p-chlorobenzyl-6-trifluorométhyl-1,2,3,4-tétrahydrocarbazol-1-yl-acétique ;
Acide 9-p-chlorobenzyl-7-fluoro-1,2,3,4-tétrahydrocarbazol-1-yl-acétique ;
Acide 9-p-chlorobenzyl-8-fluoro-1,2,3,4-tétrahydrocarbazol-1-yl-acétique ;
Acide (–)-9-p-chlorobenzyl-8-fluoro-1,2,3,4-tétrahydrocarbazol-1-yl-acétique ;
Acide (+)-9-p-chlorobenzyl-8-fluoro-1,2,3,4-tétrahydrocarbazol-1-yl-acétique ;
Acide 9-p-chlorobenzyl-8-méthyl-1,2,3,4-tétrahydrocarbazol-1-yl-acétique ;
Acide (–)-9-p-chlorobenzyl-8-méthyl-1,2,3,4-tétrahydrocarbazol-1-yl-acétique ;
Acide (+)-9-p-chlorobenzyl-8-méthyl-1,2,3,4-tétrahydrocarbazol-1-yl-acétique ;
Acide 9-p-chlorobenzyl-8-méthylthio-1,2,3,4-tétrahydrocarbazol-1-yl-acétique ;
Acide 9-p-chlorobenzyl-8-méthylsulfinyl-1,2,3,4-tétrahydrocarbazol-1-yl-acétique ;
Acide 9-p-chlorobenzyl-5,7-dichloro-1,2,3,4-tétrahydrocarbazol-1-yl-acétique ;
Acide 9-p-chlorobenzyl-6,8-dichloro-1,2,3,4-tétrahydrocarbazol-1-yl-acétique ;
Acide 9-p-chlorobenzyl-6,8-difluoro-1,2,3,4-tétrahydrocarbazol-1-yl-acétique ;
Acide (–)-9-p-chlorobenzyl-6,8-difluoro-1,2,3,4-tétrahydrocarbazol-1-yl-acétique ;
Acide (+)-9-p-chlorobenzyl-6,8-difluoro-1,2,3,4-tétrahydrocarbazol-1-yl-acétique ;
Acide 9-p-chlorobenzyl-6,8-diméthyl-1,2,3,4-tétrahydrocarbazol-1-yl-acétique ;
Acide 9-p-chlorobenzyl-6-fluoro-3-méthyl-1,2,3,4-tétrahydrocarbazol-1-yl-acétique ;
Acide 9-p-chlorobenzyl-6-méthoxy-8-méthyl-1,2,3,4-tétrahydro-carbazol-1-yl-acétique ;
Acide 9-p-méthoxybenzyl-6-fluoro-1,2,3,4-tétrahydrocarbazol-1-yl-acétique ;
Acide 9-(3,4-dichloro)benzyl-6-fluoro-1,2,3,4-tétrahydrocarbazol-1-yl-acétique ;
Acide 9-[1-(1-phényl)éthyl]-6-fluoro-1,2,3,4-tétrahydrocarbazol-1-yl-acétique ;

Acide 2-[9-p-chlorobenzyl-6-fluoro-1,2,3,4-tétrahydrocarbazol-1-yl]-propanoïque ;
Acide 3-[9-p-chlorobenzyl-6-fluoro-1,2,3,4-tétrahydrocarbazol-1-yl]-propanoïque ;
Acide 3-[9-p-chlorobenzyl-6-méthoxy-1,2,3,4-tétrahydrocarbazol-1-yl]-propanoïque.

3. Composé selon la revendication 1, qui est
Acide 9-p-chlorobenzyl-6-fluoro-1,2,3,4-tétrahydrocarbazol-1-yl-acétique ;
Acide 9-p-chlorobenzyl-8-méthyl-1,2,3,4-tétrahydrocarbazol-1-yl-acétique ;
Acide 9-p-chlorobenzyl-8-fluoro-1,2,3,4-tétrahydrocarbazol-1-yl-acétique ;
Acide 9-p-chlorobenzyl-6,8-difluoro-1,2,3,4-tétrahydrocarbazol-1-yl-acétique.

4. Composé selon la revendication 1, qui est :
Acide (–)9-p-chlorobenzyl-6-fluoro-1,2,3,4-tétrahydrocarbazol-1-yl-acétique ;
Acide (–)-9-p-chlorobenzyl-6,8-difluoro-1,2,3,4-tétrahydrocarbazol-1-yl-acétique ;
Acide (–)-9-p-chlorobenzyl-8-méthyl-1,2,3,4-tétrahydrocarbazol-1-yl-acétique ;
Acide (–)-9-p-chlorobenzyl-8-fluoro-1,2,3,4-tétrahydrocarbazol-1-yl-acétique.

5. Composé de formule :

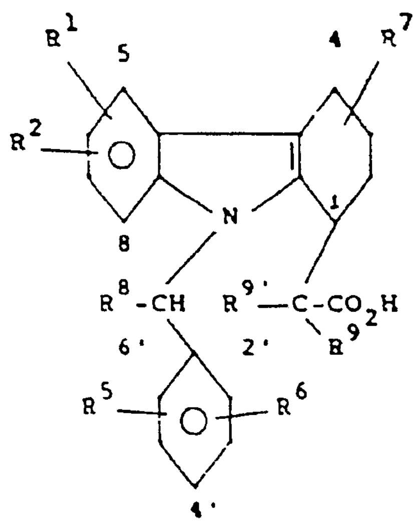

où :

| $R^1$ | $R^2$ | $R^5$ | $R^6$ | $R^9, R^{9'}$ | $R^7$ | $R^8$ |
|---|---|---|---|---|---|---|
| 6-F | H | H | H | H, H | H | H |
| 6-F | H | H | H | H, H | H | Me |
| 6-F | 8-F | 4'-n-$C_3H_7$ | H | H, H | H | H |
| 6-F | H | 4'-F | H | H, H | H | H |
| H | H | 4'-Cl | H | H, H | H | H |
| 5-F | H | 4'-Cl | H | H, H | H | H |
| 6-Me | H | 4'-Cl | H | H, H | H | H |
| 6-F | H | 4'-Cl | H | H, H | H | H |
| 6-F isomère (-) | H | 4'-Cl | H | H, H | H | H |
| 6-F isomère (+' | H | 4'-Cl | H | H, H | H | H |
| 6-Cl | H | 4'-Cl | H | H, H | H | H |
| 6-Br | H | 4'-Cl | H | H, H | H | H |

| $R^1$ | $R^2$ | $R^5$ | $R^6$ | $R^9$, $R^{9'}$ | $R^7$ | $R^8$ |
|---|---|---|---|---|---|---|
| 6-$CF_3$ | H | 4'-Cl | H | H, H | H | H |
| 6-OMe | H | 4'-Cl | H | H, H | H | H |
| 6-SMe | H | 4'-Cl | H | H, H | H | H |
| 6-S(O)Me | H | 4'-Cl | H | H, H | H | H |
| 6-$S(O)_2Me$ | H | 4'-Cl | H | H, H | H | H |
| 6-$N_3$ | H | 4'-Cl | H | H, H | H | H |
| 7-F | H | 4'-Cl | H | H, H | H | H |
| 8-Me | H | 4'-Cl | H | H, H | H | H |
| Isomère 8-Me(-) | H | 4'-Cl | H | H, H | H | H |
| Isomère 8-Me(+) | H | 4'-Cl | H | H, H | H | H |
| 8-F | H | 4'-Cl | H | H, H | H | H |
| Isomère I-F(-) | H | 4'-Cl | H | H, H | H | H |
| Isomère 8-F(+) | H | 4'-Cl | H | H, H | H | H |
| 8-Br | H | 4'-Cl | H | H, H | H | H |
| 8-SMe | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| 8-S(O)Me | H | 4'-Cl | H | H, H | H | H |
| 6-Me | 8-Me | 4'-Cl | H | H, H | H | H |
| 6-F | 8-F | 4'-Cl | H | H, H | H | H |
| Isomère 6-F(-) | 8-F | 4'-Cl | H | H, H | H | H |
| Isomère 6-F(+) | 8-F | 4'-Cl | H | H, H | H | H |
| 5-Cl | 7-Cl | 4'-Cl | H | H, H | H | H |
| 6-Cl | 8-Cl | 4'-Cl | H | H, H | H | H |
| 6-F | 8-Me | 4'-Cl | H | H, H | H | H |
| 6-OMe | 8-Me | 4'-Cl | H | H, H | H | H |
| 6-F | H | 4'-Cl | H | H, H | 1-Me | H |
| 6-F | H | 4'-Cl | H | H, H | 3-Me | H |
| 6-F | H | 4'-Cl | H | Me, H | H | H |
| 6-F | H | 4'-Cl | H | Me, Me | H | H |
| 6-F | H | 4'-Br | H | H, H | H | H |
| 6-F | 8-F | 4'-I | H | H, H | H | H |

| Composé | $R^1$ | $R^2$ | $R^5$ | $R^6$ | $R^9$,$R^{9'}$ | $R^7$ | $R^8$ |
|---|---|---|---|---|---|---|---|
| 45 | 6-F | 8-F | 4'-OMe | H | H,H | H | H |
| 46 | 6-F | 8-F | 4'-OH | H | H,H | H | H |
| 47 | 6-F | 8-F | 4'-SMe | H | H,H | H | H |
| 48 | 6-F | H | 4'-S(O)Me | H | H,H | H | H |
| 49 | 6-F | 8-F | 4'-NHCOMe | H | H,H | H | H |
| 50 | 6-F | H | 4'-$S(O)_2Me$ | H | H,H | H | H |
| 51 | 6-F | H | 4'-F | H | H,H | H | H |
| 52 | 6-F | H | 4'-Br | H | H,H | H | H |
| 53 | 6-F | 8-Me | 4'-Cl | H | H,H | H | H |
| 54 | 6-F | H | 4'-$CO_2H$ | H | H,H | H | H |
| 55 | 6-F | H | 4'-$CO_2Me$ | H | H,H | H | H |
| 56 | 6-F | 8-F | 4'-n-$C_3H_7$ | H | H,H | H | H |
| 57 | 6-F | 8-F | 3'-I | 4'-OH | H,H | H | H |
| 58 | 6-F | 8-F | 4'-I | H | H,H | H | H |
| 59 | 6-$N_3$ | H | 4'-Cl | H | H,H | H | H |
| 60 | 6-F | H | 4'-$N_3$ | H | H,H | H | H |

6. Application d'un composé selon la revendication 1 à la préparation d'un composé selon la revendication 1 à la préparation d'un médicament pour inhiber la synthèse des leucotriènes chez les mammifères.

7. Application d'un composé selon la revendication 1 à la préparation d'un médicament pour jouer un rôle d'antagoniste des prostaglandines chez les mammifères.

8. Composition pharmaceutique qui comprend un composé de la revendication 1 et un support pharmaceutiquement acceptable.

9. Composition pharmaceutique qui comprend un composé de la revendication 1 et une quantité efficace d'un second ingrédient actif choisi dans le groupe constitué par les médicaments antiinflammatoires non-stéroïdiques, les agents analgésiques périphériques ; les inhibiteurs de cyclooxygénase ; les antagonistes des leucotriènes ; les inhibiteurs de la biosynthèse des leucotriènes ; les antagonistes des récepteurs $H_1$ ; les antagonistes des récepteurs $H_2$ ; les antagonistes de prostaglandine ; les inhibiteurs d'ACE ou les inhibiteurs de la thromboxane synthétase.

10. Composition pharmaceutique selon la revendication 9, où le second ingrédient actif est l'indométhacine.

**Revendications pour les Etats contractants AT, ES, GR**

1. Procédé de préparation d'un composé de formule :

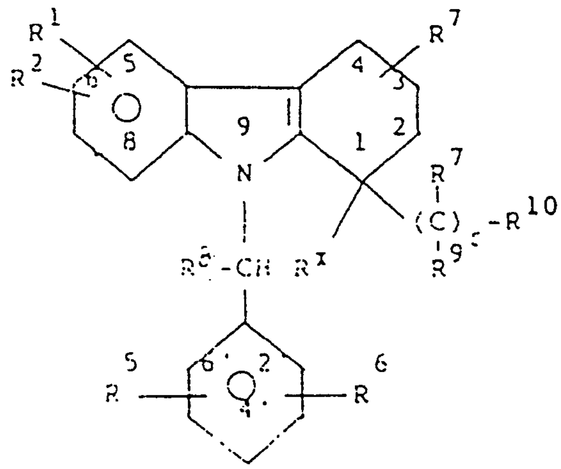

où

$R^1$, $R^2$, $R^5$ et $R^6$ sont choisis chacun indépendamment parmi :

(a) hydrogène ;

(2) alcoyle linéaire en $C_{1-6}$ ;

(3) M

où M représente

a) $OR^{13}$ ;

b) halogène ;

c) $CF_3$ ;

d) $SR^{13}$ ;

e) phényle ;

f) $COOR^{14}$ ;

g) 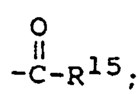

h) tétrazole ;

i) 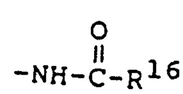

où $R^{16}$ est un alcoyle linéaire en $C_{1-6}$ ;

j) $NR^{14}R^{14}$ ;

k) $-NHSO_2R^{17}$ où $R^{17}$ est un alcoyle linéaire en $C_{1-6}$, ou

l) $-SOR^{13}$

m) $-CONR^{14}R^{14}$ ;

n) $-SO_2NR^{14}R^{14}$ ;

o) $-SO_2R^{13}$ ;

p) $NO_2$ ;

q) CN ;

r) $N_3$ ;

$R^7$ représente H ou un alcoyle linéaire en $C_{1-6}$ ;

$R^8$ représente H ou un alcoyle linéaire en $C_{1-6}$ ;

chaque $R^9$ représente indépendamment H, OH, un O-alcoyle linéaire en $C_{1-4}$ ou un alcoyle linéaire en $C_{1-4}$ ;

$R^{10}$ représente COOH ; $CH_2OH$ ; CHO ; tétrazole, $CONHSO_2R^{11}$ ; CN ; $CON(R^9)_2$ ; ou $NHSO_2R^{11}$ où $R^{11}$ représente OH, un alcoyle linéaire ou un alcoxy linéaire en $C_{1-6}$, un perhaloalcoyle en $C_{1-6}$, un phényle ou un phényle substitué par un alcoyle

linéaire ou des groupes alcoxy linéaires en $C_{1-3}$, halogène ; hydroxy, COOH, CN, formyle ou acyle en $C_{1-6}$ ;

r vaut de 1 à 6

chaque $R^{13}$ représente indépendamment H ; ou un alcoyle linéaire en $C_{1-6}$ ;

chaque $R^{14}$ représente indépendamment H, ou un alcoyle linéaire en $C_{1-6}$ ; et

chaque $R^{15}$ représente indépendamment H, un alcoyle linéaire en $C_{1-6}$ ou $CF_3$ ;

ou d'un de ses sels pharmaceutiquement acceptables ; procédé dans lequel

a) on fait réagir un composé de formule III

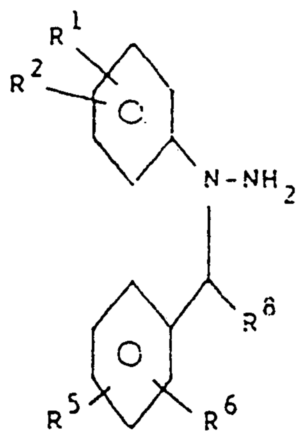

II

+

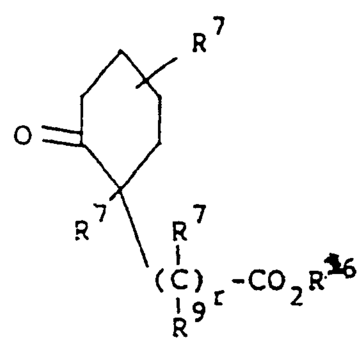

III

où $R^{16}$ représente un radical ester ; pour produire un composé de formule I où $R^{10}$ représente COOH ou un de ses esters ; ou b) on fait réagir un composé de formule

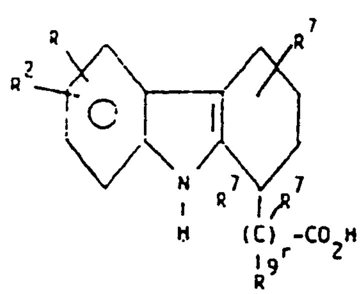

avec un composé de formule (V)

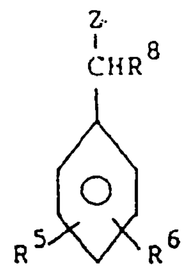

(V)

où Z est un groupe sortant ; pour produire un composé de formule I où $R^{10}$ représente COOH ou un de ses esters ; et

c) après étape a) ou étape b), le cas échéant, on transforme le groupe $R^{10}$ en un groupe $R^{10}$ différent.

2. Procédé selon la revendication 1, de préparation d'un composé qui est :
2-[9-p-chlorobenzyl-6-fluoro-1,2,3,4-tétrahydrocarbazol-1-yl]-1-éthanol ;
2-(9-chlorobenzyl-6,8-difluoro-1,2,3,4-tétrahydrocarbazol-1-yl)éthanol ;
(–) ou (+) 2-(9-p-chlorobenzyl)-6,8-difluoro-1,2,3,4-tétrahydrocarbazol-1-yl)éthanol
3-[9-p-chlorobenzyl-6-méthoxy-1,2,3,4-tétrahydrocarbazol-1-yl]-propanol ;
Acide 9-benzyl-6-fluoro-1,2,3,4-tétrahydrocarbazol-1-yl-acétique ;
Acide 9-p-chlorobenzyl-1,2,3,4-tétrahydrocarbazol-1-yl-acétique ;
Acide 9-p-chlorobenzyl-5-fluoro-1,2,3,4-tétrahydiocarbazol-1-yl-acétique ;
Acide 6-bromo-9-p-chlorobenzyl-1,2,3,4-tétrahydrocarbazol-1-yl-acétique ;
Acide 9-p-chlorobenzyl-6-chloro-1,2,3,4-tétrahydrocarbazol-1-yl-acétique ;
Acide 9-p-chlorobenzyl-6-fluoro-1,2,3,4-tétrahydrocarbazol-1-yl-acétique ;
Acide (–)9-p-chlorobenzyl-6-fluoro-1,2,3,4-tétrahydrocarbazol-1-yl-acétique ;
Acide (+)9-p-chlorobenzyl-6-fluoro-1,2,3,4-tétrahydrocarbazol-1-yl-acétique ;
Acide 9-p-chlorobenzyl-6-méthoxy-1,2,3,4-tétrahydrocarbazol-1-yl-acétique ;
Acide 9-p-chlorobenzyl-6-méthyl-1,2,3,4-tétrahydrocarbazol-1-yl-acétique ;
Acide 9-p-chlorobenzyl-6-méthylthio-1,2,3,4-tétrahydrocarbazol-1-yl-acétique ;
Acide 9-p-chlorobenzyl-6-méthylsulfinyl-1,2,3,4-tétra- hydrocarbazol-1-yl-acétique ;
Acide 9-p-chlorobenzyl-6-méthylsulfonyl-1,2,3,4-tétrahydrocarbazol-1-yl-acétique ;
Acide 9-p-chlorobenzyl-6-trifluorométhyl-1,2,3,4-tétrahydrocarbazol-1-yl-acétique ;
Acide 9-p-chlorobenzyl-7-fluoro-1,2,3,4-tétrahydrocarbazol-1-yl-acétique ;
Acide 9-p-chlorobenzyl-8-fluoro-1,2,3,4-tétrahydrocarbazol-1-yl-acétique ;
Acide (–)-9-p-chlorobenzyl-8-fluoro-1,2,3,4-tétrahydrocarbazol-1-yl-acétique ;
Acide (+)-9-p-chlorobenzyl-8-fluoro-1,2,3,4-tétrahydrocarbazol-1-yl-acétique ;
Acide 9-p-chlorobenzyl-8-méthyl-1,2,3,4-tétrahydrocarbazol-1-yl-acétique ;
Acide (–)-9-p-chlorobenzyl-8-méthyl-1,2,3,4tétrahydrocarbazol-1-yl-acétique ;
Acide (+)-9-p-chlorobenzyl-8-méthyl-1,2,3,4-tétrahydrocarbazol-1-yl-acétique ;
Acide 9-p-chlorobenzyl-8-méthylthio-1,2,3,4-tétrahydrocarbazol-1-yl-acétique ;
Acide 9-p-chlorobenzyl-8-méthylsulfinyl-1,2,3,4-tétrahydrocarbazol-1-yl-acétique ;
Acide 9-p-chlorobenzyl-5,7-dichloro-1,2,3,4-tétrahydrocarbazol-1-yl-acétique ;
Acide 9-p-chlorobenzyl-6,8-dichloro-1,2,3,4-tétrahydrocarbazol-1-yl-acétique ;
Acide 9-p-chlorobenzyl-6,8-difluoro-1,2,3,4-tétrahydrocarbazol-1-yl-acétique ;
Acide (–)-9-p-chlorobenzyl-6,8-difluoro-1,2,3,4-tétrahydrocarbazol-1-yl-acétique ;
Acide (+)-9-p-chlorobenzyl-6,8-difluoro-1,2,3,4-tétrahydro-carbazol-1-yl-acétique ;
Acide 9-p-chlorobenzyl-6,8-diméthyl-1,2,3,4-tétrahydrocarbazol-1-yl-acétique ;
Acide 9-p-chlorobenzyl-6-fluoro-3-méthyl-1,2,3,4-tétrahydrocarbazol-1-yl-acétique ;
Acide 9-p-chlorobenzyl-6-méthoxy-8-méthyl-1,2,3,4-tétrahydro-carbazol-1-yl-acétique ;
Acide 9-p-méthoxybenzyl-6-fluoro-1,2,3,4-tétrahydrocarbazol-1-yl-acétique ;
Acide 9-(3,4-dichloro)benzyl-6-fluoro-1,2,3,4-tétrahydrocarbazol-1-yl-acétique ;

Acide 9-[1-(1-phényl)éthyl]-6-fluoro-1,2,3,4-tétrahydrocarbazol-1-yl-acétique ;
Acide 2-[9-p-chlorobenzyl-6-fluoro-1,2,3,4-tétrahydrocarbazol-1-yl]propanoïque ;
Acide 3-[9-p-chlorobenzyl-6-fluoro-1,2,3,4-tétrahydrocarbazol-1-yl]-propanoïque ;

3. Procédé de préparation selon la revendication 1 d'un composé qui est :
Acide 9-p-chlorobenzyl-6-fluoro-1,2,3,4-tétrahydrocarbazol-1-yl-acétique ;
Acide 9-p-chloronenzyl-8-méthyl-1,2,3,4-tétrahydrocarbazol-1-yl-acétique ;
Acide 9-p-chlorobenzyl-8-fluoro-1,2,3,4-tétrahydrocarbazol-1-yl-acétique ;
Acide 9-p-chlorobenzyl-6,8-difluoro-1,2,3,4-tétrahydrocarbazol-1-yl-acétique.

4. Procédé selon la revendication 1 de préparation d'un composé qui est :
Acide (−)9-p-chlorobenzyl-6-fluoro-1,2,3,4-tétrahydrocarbazol-1-yl-acétique ;
Acide (−)-9-p-chlorobenzyl-6,8-difluoro-1,2,3,4-tétrahydrocarbazol-1-yl-acétique ;
Acide (−)-9-p-chlorobenzyl-8-méthyl-1,2,3,4-tétrahydrocarbazol-1-yl-acétique ;
Acide (−)-9-p-chlorobenzyl-8-fluoro-1,2,3,4-tétrahydrocarbazol-1-yl-acétique.

5. Procédé selon la revendication 1 de préparation d'un composé de formule :

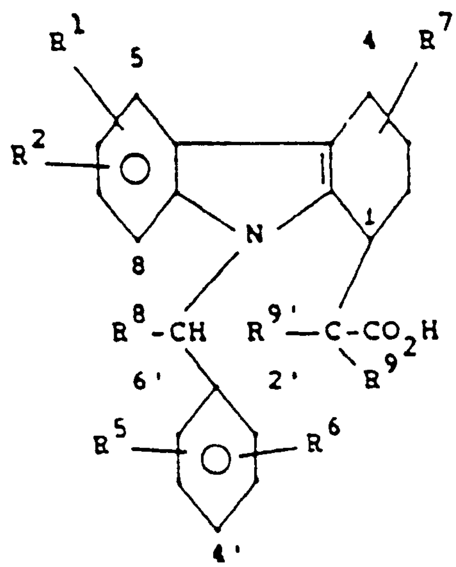

dans laquelle :

| $R^1$ | $R^2$ | $R^5$ | $R^6$ | $R^9$, $R^{9'}$ | $R^7$ | $R^8$ |
|---|---|---|---|---|---|---|
| 6-F | H | H | H | H, H | H | H |
| 6-F | H | H | H | H, H | H | Me |
| 6-F | 8-F | 4'-n-$C_3H_7$ | H | H, H | H | H |
| 6-F | H | 4'-F | H | H, H | H | H |
| H | H | 4'-Cl | H | H, H | H | H |
| 5-F | H | 4'-Cl | H | H, H | H | H |
| 6-Me | H | 4'-Cl | H | H, H | H | H |
| 6-F | H | 4'-Cl | H | H, H | H | H |
| 6-F Isomère (-) | H | 4'-Cl | H | H, H | H | H |
| 6-F Isomère (+) | H | 4'-Cl | H | H, H | H | H |
| 6-Cl | H | 4'-Cl | H | H, H | H | H |
| 6-Br | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| $6-CF_3$ | H | 4'-Cl | H | H, H | H | H |
| 6-OMe | H | 4'-Cl | H | H, H | H | H |
| 6-SMe | H | 4'-Cl | H | H, H | H | H |
| 6-S(O)Me | H | 4'-Cl | H | H, H | H | H |
| $6-S(O)_2Me$ | H | 4'-Cl | H | H, H | H | H |
| $6-N_3$ | H | 4'-Cl | H | H, H | H | H |
| 7-F | H | 4'-Cl | H | H, H | H | H |
| 8-Me | H | 4'-Cl | H | H, H | H | H |
| Isomère 8-Me(-) | H | 4'-Cl | H | H, H | H | H |
| Isomère 8-Me(+) | H | 4'-Cl | H | H, H | H | H |
| 8-F | H | 4'-Cl | H | H, H | H | H |
| Isomère I-F(-) | H | 4'-Cl | H | H, H | H | H |
| Isomère 8-F(+) | H | 4'-Cl | H | H, H | H | H |
| 8-Br | H | 4'-Cl | H | H, H | H | H |
| 8-SMe | H | 4'-Cl | H | H, H | H | H |

| $R^1$ | $R^2$ | $R^5$ | $R^6$ | $R^9, R^{9'}$ | $R^7$ | $R^8$ |
|---|---|---|---|---|---|---|
| 8-S(O)Me | H | 4'-Cl | H | H, H | H | H |
| 6-Me | 8-Me | 4'-Cl | H | H, H | H | H |
| 6-F | 8-F | 4'-Cl | H | H, H | H | H |
| Isomère 6-F(-) | 8-F | 4'-Cl | H | H, H | H | H |
| Isomère 6-F(+) | 8-F | 4'-Cl | H | H, H | H | H |
| 5-Cl | 7-Cl | 4'-Cl | H | H, H | H | H |
| 6-Cl | 8-Cl | 4'-Cl | H | H, H | H | H |
| 6-F | 8-Me | 4'-Cl | H | H, H | H | H |
| 6-OMe | 8-Me | 4'-Cl | H | H, H | H | H |
| 6-F | H | 4'-Cl | H | H, H | 1-Me | H |
| 6-F | H | 4'-Cl | H | H, H | 3-Me | H |
| 6-F | H | 4'-Cl | H | Me, H | H | H |
| 6-F | H | 4'-Cl | H | Me, Me | H | H |
| 6-F | H | 4'-Br | H | H, H | H | H |
| 6-F | 8-F | 4'-I | H | H, H | H | H |

| Composé | $R^1$ | $R'$ | $R^5$ | $R^6$ | $R^9,R^{9'}$ | $R^7$ | $R^8$ |
|---|---|---|---|---|---|---|---|
| 45 | 6-F | 8-F | 4'-OMe | H | H,H | H | H |
| 46 | 6-F | 8-F | 4'-OH | H | H,H | H | H |
| 47 | 6-F | 8-F | 4'-SMe | H | H,H | H | H |
| 48 | 6-F | H | 4'-S(O)Me | H | H,H | H | H |
| 49 | 6-F | 8-F | 4'-NHCOMe | H | H,H | H | H |
| 50 | 6-F | H | $4'-S(O)_2Me$ | H | H,H | H | H |
| 51 | 6-F | H | 4'-F | H | H,H | H | H |
| 52 | 6-F | H | 4'-Br | H | H,H | H | H |
| 53 | 6-F | 8-Me | 4'-Cl | H | H,H | H | H |
| 54 | 6-F | H | $4'-CO_2H$ | H | H,H | H | H |
| 55 | 6-F | H | $4'-CO_2Me$ | H | H,H | H | H |
| 56 | 6-F | 8-F | $4'-n-C_3H_7$ | H | H,H | H | H |
| 57 | 6-F | 8-F | 3'-I | 4'-OH | H,H | H | H |
| 58 | 6-F | 8-F | 4'-I | H | H,H | H | H |
| 59 | $6-N_3$ | H | 4'-Cl | H | H,H | H | H |
| 60 | 6-F | H | $4'-N_3$ | H | H,H | H | H |

6. Procédé de préparation d'une composition pharmaceutique, dans lequel on mélange un composé de formule I avec un support pharmaceutiquement acceptable.

7. Procédé de préparation d'une composition pharmaceutique, dans lequel on mélange un composé de formule I avec une quantité efficace d'un second ingrédient actif choisi dans le groupe constitué par les

médicaments anti-inflammatoires non-stéroïdiques, les agents analgésiques périphériques ; les inhibiteurs de cyclo-oxygénnase ; les antagonistes des leucotriènes ; les inhibiteurs de la biosynthèse des leucotriènes ; les antagonistes des récepteurs $H_1$ ; les antagonistes des récepteurs $H_2$ ; les antagonistes de prostaglandine ; les inhibiteurs d' ACE ou les inhibiteurs de la thromboxane synthétase.

8. Procédé selon la revendication 7, dans lequel le second ingrédient actif est un médicament anti-inflammatoire non-stéroïdique.

9. Procédé selon la revendication 8, dans lequel le médicament anti-inflammatoire non-stéroïdique est l'indométhacine.

10. Procédé selon la revendication 7, dans lequel le rapport pondéral dudit composé de formule I et dudit second ingrédient actif s'établit entre environ 200 : 1 et 1 : 200.